# EUROPEAN PATENT APPLICATION

(11) **EP 4 847 507 A1**
(43) Date of publication of application: **02.12.2026**
(21) Application number: 26177222.2
(22) Date of filing: 18.09.2020
(51) Int. Cl.: C07D 417/04, A61P 35/00, C07D 417/14

(54) **4-SUBSTITUTED INDOLE AND INDAZOLE SULFONAMIDO DERIVATIVES AS PARG INHIBITORS**

(30) Priority: 20.09.2019 US 201962903438 P
(62) Document number(s) of the earlier application(s) in accordance with Art. 76 EPC: 20786137.8 / 4 031 249
(71) Applicant: Ideaya Biosciences, Inc., South San Francisco, CA 94080 (US)
(72) Inventor: SUTTON, JR, James Clifford, South San Francisco, 94080 (US); DILLON, Michael Patrick, South San Francisco, 94080 (US)
(74) Representative: J A Kemp LLP

(57) **Abstract**

Provided herein are compounds having the formula: 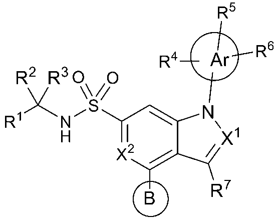 or a pharmaceutically acceptable salt thereof, wherein R¹, R², R³, R⁴, R⁵, R⁶, R⁷, Ar, X¹, X², and ring B have the meanings as provided herein. The provided compounds are useful Poly ADP-ribose glycohydrolase (PARG) inhibitors.

## Description

### CROSS-REFERENCES TO RELATED APPLICATIONS

This application claims the benefit under 35 U.S.C. 119(e) of U.S. Provisional Application No. 62/903,438, filed on September 20, 2019, which is hereby incorporated herein by reference in its entirety for all purposes.

### STATEMENT AS TO RIGHTS TO INVENTIONS MADE UNDER FEDERALLY SPONSORED RESEARCH AND DEVELOPMENT

NOT APPLICABLE

### REFERENCE TO A "SEQUENCE LISTING," A TABLE, OR A COMPUTER PROGRAM LISTING APPENDIX SUBMITTED ON A COMPACT DISK

NOT APPLICABLE

### BACKGROUND OF THE INVENTION

Cancer is caused by uncontrolled and unregulated cellular proliferation. The consequence of this often-rapid proliferation is a high level of oxidative stress within the tumor which damages DNA and leads to a much-increased mutation rate. Tumor cells therefore engage and rely heavily upon DNA damage repair mechanisms.

Single-strand breaks (SSBs) are the most common type of lesion arising in cells and PARG (Poly ADP-ribose glycohydrolase) together with PARP is involved along with a number of other proteins in single strand break repair (SSBR) and another repair mechanism called base excision repair (BER).

One of the earliest events during single strand DNA repair is the binding of PARP (poly ADP-ribose polymerase) to the break and the rapid synthesis of poly ADP-ribose (PAR) on PARP itself. This molecular structure serves as a signal to recruit other DNA repair proteins, initially XRCC1, which will then repair the break (Mortusewicz, Fouquerel et al. 2011). The signal initiated by these PAR chains is short-lived as they are rapidly degraded by the enzyme PAR glycohydrolase (PARG). When PARP is bound to PAR, its catalytic activity is reduced and therefore PARG activity helps to restore PARP to its catalytically active form (Curtin and Szabo 2013).

PARG exists as a single gene with isoforms that reside in the nucleus, mitochondria and cytosol. The only other known protein with glycohydrolase activity is ARH3 which is localized to the mitochondria (Mashimo, Kato et al. 2014). Although, known primarily for its direct role in DNA repair, PARG impacts PAR signaling in splicing, transcriptional and epigenetic pathways (Ji and Tulin 2009) (Le May, Iltis et al. 2012) (Dahl, Maturi et al. 2014) (Guastafierro, Catizone et al. 2013) (Caiafa, Guastafierro et al. 2009).

Cancer cells may become addicted to a specific DNA repair pathway when other mechanisms of DNA repair are non-functional. Tumors carrying mutations in proteins involved in double strand break repair are often more sensitive to PARP inhibitors of SSBR. There is already some evidence that PARG depletion inhibits SSBR and reduces survival of BRCA2-deficient cells (Fathers, Drayton et al. 2012). However, other tumor mutations may give rise to deficiencies in double strand DNA repair mechanisms (so-called "BRCA-ness") thereby sensitizing tumour cells to PARG inhibition.

PARG depletion has been studied in a number of murine and human model systems. Murine cells that are null or depleted for PARG display an increased sensitivity to experimental and clinical DNA damaging agents. However, as deficiency in PARG doesn't sensitize to all agents (e.g. gemcitabine, camptothecin) this suggests a specificity for PARG function with certain pathways of DNA damage repair and chemo- and radiotherapies (Fujihara, Ogino et al. 2009) (Shirai, Fujimori et al. 2013) (Zhou, Feng et al. 2010) (Zhou, Feng et al. 2011).

In humans PARG depletion sensitizes lung, cervical and pancreatic cancer cells to γ-irradiation or experimental DNA damaging agents (e.g. hydrogen peroxide, Methylmethanesulfonate) (Ame, Fouquerel et al. 2009) (Nakadate, Kodera et al. 2013) (Shirai, Poetsch et al. 2013).

PARP inhibitors are currently undergoing a raft of clinical trials where the concept of synthetic lethality or chemo-sensitization is being explored. Clinical resistance to PARP inhibitors has already been described (Drost and Jonkers 2014) (Barber, Sandhu et al. 2013) and therefore there is a requirement that alternative inhibitors targeting the DNA damage repair machinery are found. As PARG depletion leads to reduced rates of SSBR to the same extent as depletion of PARP1, PARG inhibition may provide a therapeutic advantage in PARP inhibitor resistant cells **(**Fisher, Hochegger et al. 2007**).** Furthermore, depletion of PARG has been reported to lead to a markedly different gene expression pattern to that of PARP depletion in breast cancer cells **(**Frizzell, Gamble et al. 2009).

Although current models show that PARG depletion leads to PARP-dependent effects on DNA repair, recent research has shown a mechanistic differentiation from PARP inhibition. Following a genotoxic stimulus depletion of PARG, in contrast to PARP depletion, leads to a drop in NAD levels. This leads to lung cancer cell death that may be as a result of energy failure **(**Erdelyi, Bai et al. 2009).

Cell permeable PARG inhibitors have been limited to compounds such as Tannic acid or Gallotannin which have questionable specificity for PARG and limited bioavailability **(**Sun, Zhang et al. 2012**) (**Fathers, Drayton et al. 2012**) (**Blenn, Wyrsch et al. 2011**).**

An object of this invention is to provide cell permeable inhibitors of PARG.

### SUMMARY

In one aspect, provided herein are compounds of Formula (I): 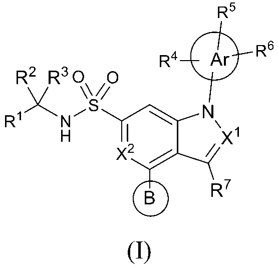 wherein:
X¹ is selected from the group consisting of N or CR⁸ where R⁸ is hydrogen, halo, C₁₋₂ alkyl, and C₁₋₂ haloalkyl;
X² is selected from the group consisting of N, CH, and CF;
R¹ is selected from the group consisting of hydrogen, cyano, formyl, -CONH₂, -CH₂OH, -CH₂OC₁₋₂ alkyl, C₁₋₂ alkyl, and C₁₋₂ haloalkyl;
R² and R³ are C₁₋₂ alkyl; or
R² and R³ together with the carbon atom to which they are attached form cyclopropyl;
Ar is a 5-membered heteroaryl;
R⁴ is selected from the group consisting of C₁₋₃ alkyl, C₁₋₃ haloalkyl, hydroxyC₁₋₃alkyl, -C(O)H and cyano;
R⁵ and R⁶ are each independently absent or selected from the group consisting of hydrogen, C₁₋₆ alkyl, hydroxy, C₁₋₆ alkoxy, halo, C₁₋₆ haloalkyl, and C₁₋₆ haloalkoxy;
R⁷ is selected from the group consisting of hydrogen, deuterium, halo, C₁₋₆ alkyl, and C₁₋₆ haloalkyl; and
ring B is C₃₋₆ cycloalkyl, phenyl, heteroaryl, heterocyclyl, fused heterocyclyl, spiro heterocyclyl, or bridged heterocyclyl wherein phenyl, heteroaryl, heterocyclyl, fused heterocyclyl, spiro heterocyclyl, and bridged heterocyclyl of ring B are substituted with R^{a}, R^{b}, and/or R^{c} wherein R^{a} is hydrogen, C₁₋₆ alkyl, hydroxy, C₁₋₆ alkoxy, halo, C₁₋₆ haloalkyl, C₁₋₆ haloalkoxy, hydroxyC₁₋₆ alkyl, heteroaryl, heterocyclyl, -C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is hydrogen or C₁₋₆ alkyl), -C(O)NR^{f}R^{g} (where R^{f} and R^{g} are independently selected from hydrogen, C₁₋₆ alkyl, C₁₋₆ haloalkyl, aminoC₁₋₆ alkyl, and hydroxyC₁₋₆ alkyl; or R^{f} and R^{g} together with the nitrogen atom to which they are attached form heterocyclyl), or -S(O)₂NR^{h}Rⁱ (where R^{h} and Rⁱ are independently selected from hydrogen, C₁₋₆ alkyl, C₁₋₆ haloalkyl, aminoC₁₋₆ alkyl, or hydroxyC₁₋₆ alkyl; or R^{h} and Rⁱ together with the nitrogen atom to which they are attached form heterocyclyl), and R^{b} and R^{c} are independently selected from hydrogen, C₁₋₆ alkyl, hydroxy, C₁₋₆ alkoxy, halo, C₁₋₆ haloalkyl, and C₁₋₆ haloalkoxy; or
ring B is a five to six membered heterocyclyl substituted with R^{a}, R^{b}, and/or R^{c}, wherein R^{b} and R^{c} are on adjacent ring vertices and are combined to form a four to six membered heterocyclyl having 0 to 2 additional heteroatom ring vertices selected from N, O, and S, and R^{a} is selected from hydrogen, C₁₋₆ alkyl, hydroxy, C₁₋₆ alkoxy, halo, C₁₋₆ haloalkyl, C₁₋₆ haloalkoxy, hydroxyC₁₋₆ alkyl, heteroaryl, heterocyclyl, -C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is hydrogen or C₁₋₆ alkyl), -C(O)NR^{f}R^{g} (where R^{f} and R^{g} are independently selected from hydrogen, C₁₋₆ alkyl, C₁₋₆ haloalkyl, aminoC₁₋₆ alkyl, and hydroxyC₁₋₆ alkyl; or R^{f} and R^{g} together with the nitrogen atom to which they are attached form heterocyclyl), and -S(O)₂NR^{h}Rⁱ (where R^{h} and Rⁱ are independently selected from hydrogen, C₁₋₆ alkyl, C₁₋₆ haloalkyl, aminoC₁₋₆ alkyl, or hydroxyC₁₋₆ alkyl; or R^{h} and Rⁱ together with the nitrogen atom to which they are attached form heterocyclyl); and
further wherein heteroaryl and heterocyclyl of R^{a}; phenyl, heteroaryl, and heterocyclyl of R^{d} and heterocyclyl formed by R^{f} and R^{g} and R^{h} and Rⁱ are unsubstituted or substituted with one, two, or three substituents independently selected from C₁₋₆ alkyl, hydroxy, C₁₋₆ alkoxy, halo, C₁₋₆ haloalkyl, and C₁₋₆ haloalkoxy; or
a pharmaceutically acceptable salt thereof.

In another aspect, provided herein is a pharmaceutical composition comprising a compound of Formula (I) (or any embodiments thereof), or a pharmaceutically acceptable salt thereof, and one or more pharmaceutically acceptable excipients.

In another aspect, provided herein is a compound of Formula (I) (or any embodiments thereof), or a pharmaceutically acceptable salt thereof, or a pharmaceutical composition as defined herein, for use in therapy.

In another aspect, provided herein is a compound of Formula (I) (or any embodiments thereof), or a pharmaceutically acceptable salt thereof, or a pharmaceutical composition as defined herein, for use in the treatment of cancer. In one embodiment, the cancer is a human cancer.

In another aspect, provided herein is a compound of Formula (I) (or any embodiments thereof), or a pharmaceutically acceptable salt thereof, or a pharmaceutical composition as defined herein, for use in the production of a PARG inhibitory effect.

In another aspect, provided herein is the use of a compound of Formula (I) (or any embodiments thereof), or a pharmaceutically acceptable salt thereof, in the manufacture of a medicament for use in the treatment of cancer. Suitably, the medicament is for use in the treatment of human cancers.

In another aspect, provided herein is the use of a compound of Formula (I) (or any embodiments thereof), or a pharmaceutically acceptable salt thereof, in the manufacture of a medicament for use in the production of a PARG inhibitory effect.

In another aspect, provided herein is a method of inhibiting PARG *in vitro* or *in vivo*, said method comprising contacting a cell with an effective amount of a compound of Formula (I) (or any embodiments thereof), or a pharmaceutically acceptable salt thereof.

In another aspect, provided herein is a method of inhibiting cell proliferation *in vitro* or *in vivo*, said method comprising contacting a cell with an effective amount of a compound of Formula (I) (or any embodiments thereof), or a pharmaceutically acceptable salt thereof.

In another aspect, provided herein is a method of treating cancer in a patient in need of such treatment, said method comprising administering to said patient a therapeutically effective amount of a compound of Formula (I) (or any embodiments thereof), or a pharmaceutically acceptable salt thereof, or a pharmaceutical composition as defined herein.

In another aspect, provided herein is a method of identifying PARG activity in a test compound of PARG inhibitory activity, said method comprising (i) contacting the test compound with isolated PARG enzyme, a biotinylated-PARylated PARP substrate to form a PARG reaction pre-mixture; (ii) contacting the PARG reaction pre-mixture with a detection antibody and streptavidin-europium to form a PARG reaction mixture; and (iii) measuring fluorescence intensity of the PARG reaction mixture, wherein said method further comprises performing steps (i)-(iii) with a positive control sample represented by Formula (I) (or any embodiments thereof).

In another aspect, provided are methods of synthesizing a compound of Formula (I) (or any embodiments thereof), or a pharmaceutically acceptable salt thereof, as defined herein.

In another aspect, provided herein is a compound as defined herein, or a pharmaceutically acceptable salt, obtainable by, or obtained by, or directly obtained by a method of synthesis as defined herein.

In another aspect, provided herein are novel intermediates as defined herein which are suitable for use in any one of the synthetic methods as set out herein.

Preferred, suitable, and optional features of any one particular aspect of the present invention are also preferred, suitable, and optional features of any other aspect.

### BRIEF DESCRIPTION OF THE DRAWINGS

NOT APPLICABLE

### DETAILED DESCRIPTION OF THE INVENTION

Before the present invention is further described, it is to be understood that the invention is not limited to the particular embodiments set forth herein, and it is also to be understood that the terminology used herein is for the purpose of describing particular embodiments only, and is not intended to be limiting.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention. Unless defined otherwise, all technical and scientific terms used herein have the same meaning as commonly understood by one of ordinary skill in the art to which this invention belongs.

As used herein, the singular forms "a," "an," and "the" include plural referents unless the context clearly dictates otherwise. It is further noted that the claims may be drafted to exclude any optional element. As such, this statement is intended to serve as antecedent basis for use of such exclusive terminology such as "solely," "only" and the like in connection with the recitation of claim elements, or use of a "negative" limitation.

The publications discussed herein are provided solely for their disclosure prior to the filing date of the present application. Further, the dates of publication provided may be different from the actual publication dates, which may need to be independently confirmed.

### General

Provided herein, for example, are compounds and compositions for inhibition of PARG, and pharmaceutical compositions comprising the same. Also provided herein are, for example, methods of treating or preventing a disease, disorder or condition, or a symptom thereof, mediated by inhibition of PARG.

### Definitions

Unless otherwise indicated, the following terms are intended to have the meaning set forth below. Other terms are defined elsewhere throughout the specification.

The term "alkyl", by itself or as part of another substituent, means, unless otherwise stated, a saturated straight or branched chain hydrocarbon radical, having the number of carbon atoms designated (*i.e.* C₁-₈ means one to eight carbons). Alkyl can include any number of carbons, such as C₁₋₂, C₁₋₃, C₁₋₄, C₁₋₅, C₁₋₆, C₁₋₇, C₁₋₈, C₁₋₉, C₁₋₁₀, C₂₋₃, C₂₋₄, C₂₋₅, C₂₋₆, C₃₋₄, C₃₋₅, C₃₋₆, C₄₋₅, C₄₋₆ and C₅₋₆. Examples of alkyl groups include methyl, ethyl, n-propyl, isopropyl, n-butyl, t-butyl, isobutyl, sec-butyl, n-pentyl, n-hexyl, n-heptyl, n-octyl, and the like.

The term "alkylene" refers to a straight or branched, saturated hydrocarbon radical having the number of carbon atoms indicated, and linking at least two other groups, *i.e*., a divalent hydrocarbon radical. The two moieties linked to the alkylene can be linked to the same atom or different atoms of the alkylene group. Representative alkylene groups include, but are not limited to, methylene, ethylene, propylene, isopropylene, butylene, isobutylene, sec-butylene, pentylene and hexylene.

"Bridged heterocyclyl" means a saturated 5 to 7 membered monocyclic heterocycle having two non-adjacent ring atoms linked by a (X)ₙ group where n is 1 to 3, each X is CRR', NR, S(O)ₙ₁, or O wherein no more than one X is NR, S(O)ₙ₁ or O, and R and R' are independently H or methyl (also may be referred to herein as "bridging" group). The 5 to 7 membered heterocycle further having from one to three heteroatoms independently selected from N, O, and S(O)ₙ₁, the remaining ring atoms being carbon, where n1 is an integer from 0 to 2. Examples include, but are not limited to, 2-azabicyclo[2.2.2]octane, quinuclidine, 7-oxabicyclo[2.2.1]heptane, and the like. Additional examples include 3,8-diazabicyclo[3.2.1]octane, and the like.

The term "cycloalkyl" refers to a saturated hydrocarbon ring having the indicated number of ring atoms (*e.g.*, C₃₋₆ cycloalkyl). Cycloalkyl is optionally substituted with one, two, or three substituents independently selected from C₁₋₆ alkyl, halo, hydroxy, C₁₋₆ haloalkyl, C₁₋₆ haloalkoxy, or cyano, unless stated otherwise. Representative examples include, but are not limited to, cyclopropyl, cyclobutyl, cyclopentyl, and the like.

The term "fused heterocyclyl" as used herein, means a saturated monocyclic ring of 4 to 7 ring atoms having from one to three heteroatoms independently selected from N, N(oxide), O, S, SO and SO₂ and the remaining ring atoms being carbon, and further wherein the heterocyclyl ring is fused to two adjacent ring members of a phenyl, a five or six membered heteroaryl, or C₃₋₆ cycloalkyl, each as defined herein, unless stated otherwise. The fused heterocyclyl can be attached to the remainder of the molecule through any ring atom. For sake of clarity, the number of ring atoms in the saturated monocyclic ring includes the two common ring vertices shared with the fused group (e.g., the phenyl, five or six membered heteroaryl, or C₃₋₆ cycloalkyl). Additionally, a cycloalkyl moiety in a fused heterocyclyl group is substituted as defined in the claims. Non limiting examples of the fused heterocyclyl include 2,3-dihydrobenzo[b][1,4]-dioxinyl, 2-oxabicyclo[3.1.0]hexanyl, and the like.

The term "halo" or "halogen," by itself or as part of another substituent, mean, unless otherwise stated, a fluorine, chlorine, bromine, or iodine atom.

The term "haloalkyl," means alkyl, as defined above, that is substituted with one to five halo atoms and includes monohaloalkyl and polyhaloalkyl. For example, the term "C₁-₄ haloalkyl" includes trifluoromethyl, 2,2,2-trifluoroethyl, 4-chlorobutyl, 3-bromopropyl, and the like.

The terms "alkoxy," and "haloalkoxy" refer to alkyl and haloalkyl groups respectively, each as defined herein, that is attached to the remainder of the molecule via an oxygen atom.

The term "aminoalkyl," means alkyl, as defined above, that is substituted with one NRR' where R and R' are independently hydrogen, C₁₋₆ alkyl, hydroxyC₁₋₆ alkyl, C₁₋₆ alkoxyC₁₋₆ alkyl, or -COC₁₋₆ alkyl. For example, the term "aminoC₁-₆ alkyl" is meant to include NH₂methyl, methylaminoethyl, diethylaminoethyl, dimethylaminoethyl, acetylaminoethyl, and the like.

The term "aryl" means, unless otherwise stated, an aromatic, hydrocarbon group which can be a single ring or multiple rings (up to three rings) which are fused together or linked covalently. Non-limiting examples of aryl groups include phenyl, naphthyl and biphenyl.

The term "heteroaryl" refers to a 5- to 10-membered aromatic ring that contains from one to five heteroatoms selected from N, O, and S, wherein the nitrogen and sulfur atoms are optionally oxidized, and the nitrogen atom(s) are optionally quaternized. A heteroaryl group can be attached to the remainder of the molecule through a heteroatom. Non-limiting examples of heteroaryl groups include pyridyl, pyridazinyl, pyrazinyl, pyrimindinyl, triazinyl, quinolinyl, quinoxalinyl, quinazolinyl, cinnolinyl, phthalazinyl, benzotriazinyl, purinyl, benzimidazolyl, benzopyrazolyl, benzotriazolyl, benzisoxazolyl, isobenzofuryl, isoindolyl, indolizinyl, benzotriazinyl, thienopyridinyl, thienopyrimidinyl, pyrazolopyrimidinyl, imidazopyridines, benzothiaxolyl, benzofuranyl, benzothienyl, indolyl, quinolyl, isoquinolyl, isothiazolyl, pyrazolyl, indazolyl, pteridinyl, imidazolyl, triazolyl, tetrazolyl, oxazolyl, isoxazolyl, thiadiazolyl, pyrrolyl, thiazolyl, furyl, thienyl and the like.

The term "heterocycloalkyl" or "heterocyclyl" refers to a saturated or partially unsaturated 4 to 10 membered monocyclic or bicyclic ring having from one to four heteroatoms independently selected from N, O, and S and the remaining ring atom being carbon. The nitrogen and sulfur atoms are optionally oxidized, and the nitrogen atom(s) are optionally quaternized and one or two ring carbon atoms of the heterocyclic ring may be replaced by - C=(O) group. Non limiting examples of heterocycloalkyl groups include pyrrolidine, imidazolidine, pyrazolidine, butyrolactam, valerolactam, imidazolidinone, hydantoin, dioxolane, piperidine, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrahydrothiophene, , and the like. A heterocycloalkyl group can be attached to the remainder of the molecule through a ring carbon or a heteroatom. Non limiting examples of heterocycloalkyl groups include pyridine-2(H)-one.

The term "spiro heterocyclyl" as used herein, means a saturated or partially unsaturated bicyclic ring of 5 to 12 ring atoms wherein one to three ring atoms are heteroatoms independently selected from N, N(oxide), O, S, SO and SO₂ and the remaining ring atoms being carbon and further wherein the 2 rings are linked together by one common atom. Non limiting examples of the spiro heterocyclyl include 6-azaspiro[3.4]octane, 2-oxa-6-azaspiro[3.4]octan-6-yl, 4-oxaspiro[2.4]heptanyl, spiro[3.5]non-6-ene, and 2,7-diazaspiro[4.4]nonanyl.

The term "hydroxyalkyl," means alkyl, as defined above, that is substituted with one or two hydroxy. For example, the term " hydroxyC₁-₄ alkyl" is mean to include hydroxymethyl, 1-, or 2-hydroxyethyl, 1,2-dihydroxyethyl, hydroxypropyl, and the like.

As used herein, a wavy line, " ", that intersects a single, double or triple bond in any chemical structure depicted herein, represent the point attachment of the single, double, or triple bond to the remainder of the molecule. Additionally, a bond extending to the center of a ring (e.g., a phenyl ring) is meant to indicate attachment at any of the available ring vertices. One of skill in the art will understand that multiple substituents shown as being attached to a ring will occupy ring vertices that provide stable compounds and are otherwise sterically compatible.

As used herein, the term "heteroatom" is meant to include oxygen (O), nitrogen (N), sulfur (S) and silicon (Si).

The term "pharmaceutically acceptable salts" is meant to include salts of the compounds of Formula (I) which are prepared with relatively nontoxic acids or bases, depending on the particular substituents found on the compounds described herein. When compounds of Formula (I) contain relatively acidic functionalities, base addition salts can be obtained by contacting the neutral form of such compounds with a sufficient amount of the desired base, either neat or in a suitable inert solvent. Examples of salts derived from pharmaceutically acceptable inorganic bases include aluminum, ammonium, calcium, copper, ferric, ferrous, lithium, magnesium, manganic, manganous, potassium, sodium, zinc and the like. Salts derived from pharmaceutically-acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like. When compounds of Formula (I) contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge, S.M., et al, "Pharmaceutical Salts", Journal of Pharmaceutical Science, 1977, 66, 1-19). Certain specific compounds of the present invention contain both basic and acidic functionalities that allow the compounds to be converted into either base or acid addition salts.

The neutral forms of the compounds of Formula (I) may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention. In addition to salt forms, provided herein are compounds of Formula (I) which are in a prodrug form. Prodrugs of the compounds of Formula (I) are those compounds that readily undergo chemical changes under physiological conditions to provide the compounds of Formula (I). Additionally, prodrugs can be converted to the compounds of Formula (I) by chemical or biochemical methods in an *ex vivo* environment. For example, prodrugs can be slowly converted to the compounds of Formula (I) when placed in a transdermal patch reservoir with a suitable enzyme or chemical reagent. Prodrugs are described in more detail elsewhere herein.

Certain compounds of Formula (I) can exist in unsolvated forms as well as solvated forms, including hydrated forms. In general, the solvated forms are equivalent to unsolvated forms and are intended to be encompassed within the scope of the present invention. Certain compounds of Formula (I) may exist in multiple crystalline or amorphous forms. In general, all physical forms are equivalent for the uses contemplated by the present invention and are intended to be within the scope of the present invention.

Certain compounds of Formula (I) possess asymmetric carbon atoms (optical centers) or double bonds; the racemates, diastereomers, geometric isomers, regioisomers and individual isomers (e.g., separate enantiomers) are all intended to be encompassed within the scope of the present invention. When a stereochemical depiction is shown, it is meant to refer the compound in which one of the isomers is present and substantially free of the other isomer. 'Substantially free of' another isomer indicates at least an 80/20 ratio of the two isomers, more preferably 90/10, or 95/5 or more. In some embodiments, one of the isomers will be present in an amount of at least 99%.

The compounds of Formula (I) may also contain unnatural proportions of atomic isotopes at one or more of the atoms that constitute such compounds. Unnatural proportions of an isotope may be defined as ranging from the amount found in nature to an amount consisting of 100% of the atom in question. For example, the compounds may incorporate radioactive isotopes, such as for example tritium (³H), iodine-125 (¹²⁵I) or carbon-14 (¹⁴C), or nonradioactive isotopes, such as deuterium (²H) or carbon-13 (¹³C). Such isotopic variations can provide additional utilities to those described elsewhere within this application. For instance, isotopic variants of the compounds of the invention may find additional utility, including but not limited to, as diagnostic and/or imaging reagents, or as cytotoxic/radiotoxic therapeutic agents. Additionally, isotopic variants of the compounds of Formula (I) can have altered pharmacokinetic and pharmacodynamic characteristics which can contribute to enhanced safety, tolerability or efficacy during treatment. All isotopic variations of the compounds of Formula (I), whether radioactive or not, are intended to be encompassed within the scope of the present invention.

The terms "patient" or "subject" are used interchangeably to refer to a human or a non-human animal (e.g., a mammal). In one embodiment, the patient or subject is a human.

The terms "administration", "administer" and the like, as they apply to, for example, a subject, cell, tissue, organ, or biological fluid, refer to contact of, for example, an inhibitor of PARG, a pharmaceutical composition comprising same, or a diagnostic agent to the subject, cell, tissue, organ, or biological fluid. In the context of a cell, administration includes contact (e.g., in vitro or ex vivo) of a reagent to the cell, as well as contact of a reagent to a fluid, where the fluid is in contact with the cell.

The terms "treat", "treating", treatment" and the like refer to a course of action (such as administering an inhibitor of PARG or a pharmaceutical composition comprising same) initiated after a disease, disorder or condition, or a symptom thereof, has been diagnosed, observed, and the like so as to eliminate, reduce, suppress, mitigate, or ameliorate, either temporarily or permanently, at least one of the underlying causes of a disease, disorder, or condition afflicting a subject, or at least one of the symptoms associated with a disease, disorder, condition afflicting a subject. Thus, treatment includes inhibiting (e.g., arresting the development or further development of the disease, disorder or condition or clinical symptoms association therewith) an active disease.

The term "in need of treatment" as used herein refers to a judgment made by a physician or other caregiver that a subject requires or will benefit from treatment. This judgment is made based on a variety of factors that are in the realm of the physician's or caregiver's expertise.

The terms "prevent", "preventing", "prevention" and the like refer to a course of action (such as administering a PARG inhibitor or a pharmaceutical composition comprising same) initiated in a manner (e.g., prior to the onset of a disease, disorder, condition or symptom thereof) so as to prevent, suppress, inhibit or reduce, either temporarily or permanently, a subject's risk of developing a disease, disorder, condition or the like (as determined by, for example, the absence of clinical symptoms) or delaying the onset thereof, generally in the context of a subject predisposed to having a particular disease, disorder or condition. In certain instances, the terms also refer to slowing the progression of the disease, disorder or condition or inhibiting progression thereof to a harmful or otherwise undesired state.

The term "in need of prevention" as used herein refers to a judgment made by a physician or other caregiver that a subject requires or will benefit from preventative care. This judgment is made based on a variety of factors that are in the realm of a physician's or caregiver's expertise.

The terms "inhibiting" and "reducing," or any variation of these terms in relation of PARG, includes any measurable decrease or complete inhibition to achieve a desired result. For example, there may be a decrease of about 5%, 10%, 15%, 20%, 25%, 30%, 35%, 40%, 45%, 50%, 55%, 60%, 65%, 70%, 75%, 80%, 85%, 90%, 95%, 99%, or more, reduction of PARG activity compared to normal. About as used herein means within ± 10%, preferably ± 5% of a given value.

The phrase "therapeutically effective amount" refers to the administration of an agent to a subject, either alone or as part of a pharmaceutical composition and either in a single dose or as part of a series of doses, in an amount capable of having any detectable, positive effect on any symptom, aspect, or characteristic of a disease, disorder or condition when administered to the subject. The therapeutically effective amount can be ascertained by measuring relevant physiological effects, and it can be adjusted in connection with the dosing regimen and diagnostic analysis of the subject's condition, and the like. By way of example, measurement of the serum level of a PARG inhibitor (or, e.g., a metabolite thereof) at a particular time post-administration may be indicative of whether a therapeutically effective amount has been used.

The term "substantially pure" indicates that a component makes up greater than about 50% of the total content of the composition, and typically greater than about 60% of the total content. More typically, "substantially pure" refers to compositions in which at least 75%, at least 85%, at least 90% or more of the total composition is the component of interest.

### Embodiments

1. In embodiment 1, the compound of Formula (I) or a pharmaceutically acceptable salt thereof, is as described in the Summary.
2. In embodiment 2, the compound of Formula (I) of embodiment 1 or a pharmaceutically acceptable salt thereof, is wherein X¹ is N.
3. In embodiment 3, the compound of Formula (I) of embodiment 1, or a pharmaceutically acceptable salt thereof is wherein X¹ is CR⁸. In a first subembodiment of embodiment 3, the compound of Formula (I) or a pharmaceutically acceptable salt thereof is wherein R⁸ is hydrogen, fluoro, methyl, ethyl, difluoromethyl, or trifluoromethyl. In a second subembodiment of embodiment 3, the compound of Formula (I) or a pharmaceutically acceptable salt thereof is wherein R⁸ is hydrogen, fluoro, or methyl. In a third subembodiment of embodiment 3, the compound of Formula (I) or a pharmaceutically acceptable salt thereof is wherein R⁸ is hydrogen. In a fourth subembodiment of embodiment 3, the compound of Formula (I) or a pharmaceutically acceptable salt thereof is wherein R⁸ is halo, C₁₋₂ alkyl, or C₁₋₂ haloalkyl.
4. In embodiment 4, the compound of Formula (I) of any one of embodiments 1 to 3 and subembodiments contained therein, or a pharmaceutically acceptable salt thereof is wherein X² is CH or CF. In a first subembodiment of embodiment 4, the compound of Formula (I) or a pharmaceutically acceptable salt thereof is wherein X² is CH.
5. In embodiment 5, the compound of Formula (I) of any one of embodiments 1 to 3 and subembodiments contained therein, or a pharmaceutically acceptable salt thereof is wherein X² is N.
6. In embodiment 6, the compound of Formula (I) of any one of embodiments 1 to 5 and subembodiments contained therein, or a pharmaceutically acceptable salt thereof is wherein R¹ is hydrogen, cyano, methyl, or ethyl. In a first subembodiment of embodiment 6, the compound of Formula (I) or a pharmaceutically acceptable salt thereof is wherein R¹ is hydrogen. In a second subembodiment of embodiment 6, the compound of Formula (I) or a pharmaceutically acceptable salt thereof is wherein R¹ is cyano. In a third subembodiment of embodiment 6, the compound of Formula (I) or a pharmaceutically acceptable salt thereof is wherein R¹ is methyl. In a fourth subembodiment of embodiment 6, the compound of Formula (I) or a pharmaceutically acceptable salt thereof is wherein R¹ is C₁₋₂ alkyl. In a fifth subembodiment of embodiment 6, the compound of Formula (I) or a pharmaceutically acceptable salt thereof is wherein R¹ is -CONH₂. In a sixth subembodiment of embodiment 6, the compound of Formula (I) or a pharmaceutically acceptable salt thereof is wherein R¹ is cyano or C₁₋₂ alkyl. In a sixth subembodiment of embodiment 6, the compound of Formula (I) or a pharmaceutically acceptable salt thereof is wherein R¹ is cyano, C₁₋₂ alkyl, or -CONH₂. In a seventh subembodiment of embodiment 6, the compound of Formula (I) or a pharmaceutically acceptable salt thereof is wherein R¹ is cyano or -CONH₂.
7. In embodiment 7, the compound of Formula (I) of any one of embodiments 1 to 6 and subembodiments contained therein, or a pharmaceutically acceptable salt thereof is wherein R⁷ is hydrogen, deuterium, fluoro, chloro, methyl, difluoromethyl, or trifluorommethyl. In a first subembodiment of embodiment 7, the compound of Formula (I) or a pharmaceutically acceptable salt thereof is wherein R⁷ is hydrogen, chloro, or fluoro. In a second subembodiment of embodiment 7, the compound of Formula (I) or a pharmaceutically acceptable salt thereof is wherein R⁷ is hydrogen. In a second subembodiment of embodiment 7, the compound of Formula (I) or a pharmaceutically acceptable salt thereof is wherein R⁷ is halo. In a third subembodiment of embodiment 7, the compound of Formula (I) or a pharmaceutically acceptable salt thereof is wherein R⁷ is C₁₋₆ alkyl, and C₁₋₆ haloalkyl.
8. In embodiment 8, the compound of Formula (I) of any one of embodiments 1 to 7 and subembodiments contained therein, or a pharmaceutically acceptable salt thereof is wherein R² and R³ are C₁₋₂ alkyl. In a first subembodiment of embodiment 8, the compound of Formula (I) or a pharmaceutically acceptable salt thereof is wherein R² and R³ are independently methyl or ethyl, preferably methyl.
9. In embodiment 9, the compound of Formula (I) of any one of embodiments 1 to 7 and subembodiments contained therein, or a pharmaceutically acceptable salt thereof is wherein R² and R³ together with the carbon atom to which they are attached form cyclopropyl.
10. In embodiment 10, the compound of Formula (I) of any one of embodiments 1 to 9 and subembodiments contained therein, or a pharmaceutically acceptable salt thereof is wherein Ar is imidazolyl, isoxazolyl, pyrazolyl, 1,2,3-triazolyl, 1,2,4-triazolyl, thiazolyl, 1,2,4-oxadiazolyl, 1,3,4-oxadiazolyl, 1,2,4-thiadiazolyl, or 1,3,4-thiadiazolyl. In a first subembodiment of embodiment 10, the compound of Formula (I) or a pharmaceutically acceptable salt thereof is wherein Ar is 1,3,4-thiadiazol-2-yl. In a second subembodiment of embodiment 10, the compound of Formula (I) or a pharmaceutically acceptable salt thereof is wherein Ar is imidazolyl, isoxazolyl, pyrazolyl, 1,2,3-triazolyl, 1,2,4-triazolyl, thiazolyl, 1,2,4-thiadiazolyl, or 1,3,4-thiadiazolyl. In a third subembodiment of embodiment 10, the compound of Formula (I) or a pharmaceutically acceptable salt thereof is wherein Ar is 1,2,4-thiadiazolyl, or 1,3,4-thiadiazolyl.
11. In embodiment 11, the compound of Formula (I) of any one of embodiments 1 to 10 and subembodiments contained therein, or a pharmaceutically acceptable salt thereof is wherein R⁴ is attached to the carbon atom of Ar that is meta to the atom of Ar that is attached to the nitrogen atom of the remainder of the molecule. In a first subembodiment of embodiment 11, the compound of Formula (I) or a pharmaceutically acceptable salt thereof is wherein R⁴ is methyl, ethyl, difluoromethyl, trifluoromethyl, cyano, or C(O)H_{.} In a second subembodiment of embodiment 11, the compound of Formula (I) or a pharmaceutically acceptable salt thereof is wherein R⁴ is difluoromethyl, cyano, or C(O)H_{.} In a third subembodiment of embodiment 11, the compound of Formula (I) or a pharmaceutically acceptable salt thereof is wherein R⁴ is difluoromethyl_{.} In a fourth subembodiment of embodiment 11, the compound of Formula (I) or a pharmaceutically acceptable salt thereof is wherein R⁴ is cyano_{.} In a fifth subembodiment of embodiment 11, the compound of Formula (I) or a pharmaceutically acceptable salt thereof is wherein R⁴ is C₁₋₃ haloalkyl_{.} In a sixth subembodiment of embodiment 11, the compound of Formula (I) or a pharmaceutically acceptable salt thereof is wherein R⁴ is C₁₋₃ alkyl, hydroxyC₁₋₃alkyl, -C(O)H or cyano. In a sixth subembodiment of embodiment 11, the compound of Formula (I) or a pharmaceutically acceptable salt thereof is wherein R⁴ is C₁₋₃ alkyl or hydroxyC₁₋₃alkyl.
12. In embodiment 12, the compound of Formula (I) of any one of embodiments 1 to 11 and subembodiments contained therein, or a pharmaceutically acceptable salt thereof is wherein R⁵ and R⁶ are are each independently hydrogen or absent. In a first subembodiment of embodiment 12, the compound of Formula (I) of any one of embodiments 1 to 11 and subembodiments contained therein, or a pharmaceutically acceptable salt thereof is wherein R⁵ and R⁶ are absent. In a second subembodiment of embodiment 12, the compound of Formula (I) of any one of embodiments 1 to 11 and subembodiments contained therein, or a pharmaceutically acceptable salt thereof is wherein R⁵ and R⁶ are hydrogen.
13. In embodiment 13, the compound of Formula (I) of any one of embodiments 1 to 12 and subembodiments contained therein, or a pharmaceutically acceptable salt thereof is wherein ring B is C₃₋₆ cycloalkyl. In a first subembodiment of embodiment 13, the compound of Formula (I) or a pharmaceutically acceptable salt thereof is wherein ring B is cyclopropyl, cyclobutyl, cyclopentyl or cyclohexyl.
14. In embodiment 14, the compound of Formula (I) of any one of embodiments 1 to 12 and subembodiments contained therein, or a pharmaceutically acceptable salt thereof is wherein ring B is phenyl substituted with R^{a}, R^{b}, and/or R^{c} as defined in the Summary. In a first subembodiment of embodiment 14, the compound of Formula (I) or a pharmaceutically acceptable salt thereof is wherein ring B is phenyl substituted with R^{a}, R^{b}, and/or R^{c} where R^{a} is hydrogen, C₁₋₆ alkyl, hydroxy, C₁₋₆ alkoxy, halo, C₁₋₆ haloalkyl, hydroxyC₁₋₆ alkyl, heteroaryl, - C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is hydrogen or C₁₋₆ alkyl), -C(O)NR^{f}R^{g} (where R^{f} and R^{g} are independently selected from hydrogen, C₁₋₆ alkyl, C₁₋₆ haloalkyl, aminoC₁₋₆ alkyl, and hydroxyC₁₋₆ alkyl), and R^{b} and R^{c} are independently selected from hydrogen, C₁₋₆ alkyl, hydroxy, C₁₋₆ alkoxy, halo, C₁₋₆ haloalkyl, and C₁₋₆ haloalkoxy; and further wherein heteroaryl of R^{a}; phenyl, heteroaryl, and heterocyclyl of R^{d} are unsubstituted or substituted with one, two, or three substituents independently selected from C₁₋₆ alkyl, and C₁₋₆ haloalkyl. In a second subembodiment of embodiment 14, the compound of Formula (I) or a pharmaceutically acceptable salt thereof is wherein ring B is phenyl substituted with R^{a} where R^{a} is -C(O)NR^{f}R^{g} (where R^{f} and R^{g} are independently selected from hydrogen, C₁₋₆ alkyl, C₁₋₆ haloalkyl, aminoC₁₋₆ alkyl, and hydroxyC₁₋₆ alkyl).
15. In embodiment 15, the compound of Formula (I) of any one of embodiments 1 to 12 and subembodiments contained therein, or a pharmaceutically acceptable salt thereof is wherein ring B is heteroaryl substituted with R^{a}, R^{b}, and/or R^{c} as defined in the Summary. In a first subembodiment of embodiment 15, the compound of Formula (I) or a pharmaceutically acceptable salt thereof is wherein ring B is imidazolyl, pyridazinyl, thiazolyl, pyridinyl, pyrimidinyl, pyrazinyl, or indazolyl, each ring substituted with R^{a}, R^{b}, and/or R^{c} where R^{a} is hydrogen, C₁₋₆ alkyl, hydroxy, C₁₋₆ alkoxy, halo, C₁₋₆ haloalkyl, hydroxyC₁₋₆ alkyl, heteroaryl, - C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is hydrogen or C₁₋₆ alkyl), -C(O)NR^{f}R^{g} (where R^{f} and R^{g} are independently selected from hydrogen, C₁₋₆ alkyl, C₁₋₆ haloalkyl, aminoC₁₋₆ alkyl, or hydroxyC₁₋₆ alkyl), and R^{b} and R^{c} are independently selected from hydrogen, C₁₋₆ alkyl, hydroxy, C₁₋₆ alkoxy, halo, C₁₋₆ haloalkyl, and C₁₋₆ haloalkoxy; and further wherein heteroaryl of R^{a}; phenyl, heteroaryl, and heterocyclyl of R^{d} are unsubstituted or substituted with one, two, or three substituents independently selected from C₁₋₆ alkyl, and C₁₋₆ haloalkyl. In a second subembodiment of embodiment 15, the compound of Formula (I) or a pharmaceutically acceptable salt thereof is wherein ring B is imidazolyl, pyridazinyl, thiazolyl, pyridinyl, pyrimidinyl, pyrazinyl, or indazolyl, each ring substituted with R^{a} wherein R^{a} is hydrogen, C₁₋₆ alkyl, or C₁₋₆ alkoxy, preferably hydrogen, methyl, or methoxy. In a third subembodiment of embodiment 15, the compound of Formula (I) or a pharmaceutically acceptable salt thereof is wherein ring B is imidazolyl, pyridazinyl, thiazolyl, pyridinyl, pyrimidinyl, pyrazinyl, or indazolyl, each ring substituted with R^{a} wherein R^{a} is C₁₋₆ alkyl, or C₁₋₆ alkoxy.
16. In embodiment 16, the compound of Formula (I) of any one of embodiments 1 to 12 and subembodiments contained therein, or a pharmaceutically acceptable salt thereof is wherein ring B is heterocyclyl substituted with R^{a}, R^{b}, and/or R^{c} as defined in the Summary. In a first subembodiment of embodiment 16, the compound of Formula (I) or a pharmaceutically acceptable salt thereof is wherein ring B is morpholinyl, 1,1-dioxothiomorpholinyl, azetinyl, pyrrolidinyl, piperidinyl, 6-oxo-1,6-dihydropyridinyl, or piperazinyl, each ring substituted with R^{a}, R^{b}, and/or R^{c} where R^{a} is hydrogen, C₁₋₆ alkyl, hydroxy, C₁₋₆ alkoxy, halo, C₁₋₆ haloalkyl, hydroxyC₁₋₆ alkyl, heteroaryl, -C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is hydrogen or C₁₋₆ alkyl), - C(O)NR^{f}R^{g} (where R^{f} and R^{g} are independently selected from hydrogen, C₁₋₆ alkyl, C₁₋₆ haloalkyl, aminoC₁₋₆ alkyl, and hydroxyC₁₋₆ alkyl), and R^{b} and R^{c} are independently selected from hydrogen, C₁₋₆ alkyl, hydroxy, C₁₋₆ alkoxy, halo, C₁₋₆ haloalkyl, and C₁₋₆ haloalkoxy; and further wherein heteroaryl of R^{a}; phenyl, heteroaryl, and heterocyclyl of R^{d} are unsubstituted or substituted with one, two, or three substituents independently selected from C₁₋₆ alkyl, and C₁₋₆ haloalkyl. In a first subembodiment of first subembodiment of embodiment 16, the compound of Formula (I) or a pharmaceutically acceptable salt thereof is wherein ring B is morpholin-4-yl, 1,1-dioxothiomorpholin-4-yl, azetin-1-yl, pyrrolidin-1-yl, piperidin-1-yl, 6-oxo-1,6-dihydropyridin-3-yl, or piperazin-1-yl and R^{a} is attached to ring atom that is para to the ring atom attaching each of the ring to the remainder of the molecule. In a second subembodiment of embodiment 16, the compound of Formula (I) or a pharmaceutically acceptable salt thereof, wherein R^{a} is hydrogen, C₁₋₆ alkyl, hydroxy, halo, C₁₋₆ haloalkyl, hydroxyC₁₋₆ alkyl, heteroaryl, - C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is C₁₋₆ alkyl), -C(O)NR^{f}R^{g} (where R^{f} and R^{g} are independently selected from hydrogen, C₁₋₆ alkyl, C₁₋₆ haloalkyl, and aminoC₁₋₆ alkyl), and R^{b} and R^{c} are independently selected from hydrogen, C₁₋₆ alkyl, and halo; and further wherein heteroaryl of R^{a}; heterocyclyl of R^{d} are unsubstituted or substituted with one, two, or three substituents independently selected from C₁₋₆ alkyl, and C₁₋₆ haloalkyl. In a third subembodiment of embodiment 16, the compound of Formula (I) or a pharmaceutically acceptable salt thereof, wherein R^{a} is hydrogen, C₁₋₆ alkyl, hydroxy, halo, C₁₋₆ haloalkyl, or hydroxyC₁₋₆ alkyl. In a fourth subembodiment of embodiment 16, the compound of Formula (I) or a pharmaceutically acceptable salt thereof, wherein R^{a} is hydrogen, C₁₋₆ alkyl, hydroxy, halo, C₁₋₆ haloalkyl, or hydroxyC₁₋₆ alkyl, ), and R^{b} and R^{c} are independently selected from hydrogen, C₁₋₆ alkyl, and halo. In a fourth subembodiment of embodiment 16, the compound of Formula (I) or a pharmaceutically acceptable salt thereof, wherein R^{a} is -C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is C₁₋₆ alkyl), -C(O)NR^{f}R^{g} (where R^{f} and R^{g} are independently selected from hydrogen, C₁₋₆ alkyl, C₁₋₆ haloalkyl, and aminoC₁₋₆ alkyl), and R^{b} and R^{c} are independently selected from hydrogen, C₁₋₆ alkyl, and halo; and further wherein heterocyclyl of R^{d} are unsubstituted or substituted with one, two, or three substituents independently selected from C₁₋₆ alkyl, and C₁₋₆ haloalkyl. In a fifth subembodiment of embodiment 16, the compound of Formula (I) or a pharmaceutically acceptable salt thereof, wherein R^{a} is heteroaryl, and R^{b} and R^{c} are independently selected from hydrogen, C₁₋₆ alkyl, and halo; and further wherein heteroaryl of R^{a} is unsubstituted or substituted with one, two, or three substituents independently selected from C₁₋₆ alkyl, and C₁₋₆ haloalkyl. In a sixth subembodiment of embodiment 16, the compound of Formula (I) or a pharmaceutically acceptable salt thereof, wherein R^{a} is -C(O)NR^{f}R^{g} (where R^{f} and R^{g} are independently selected from hydrogen, C₁₋₆ alkyl, C₁₋₆ haloalkyl, and aminoC₁₋₆ alkyl), and R^{b} and R^{c} are independently selected from hydrogen, C₁₋₆ alkyl, and halo.
17. In embodiment 17, the compound of Formula (I) of any one of embodiments 1 to 12 and subembodiments contained therein, or a pharmaceutically acceptable salt thereof is wherein ring B is bicyclic heterocylyl, fused heterocyclyl, spiro heterocyclyl, or bridged heterocyclyl, each ring substituted with R^{a}, R^{b}, and/or R^{c} as defined in the Summary. In some embodiments, ring B is a five to six membered heterocyclyl having 1 to 3 heteroatom ring vertices selected from N, O, and S substituted with R^{a}, R^{b}, and R^{c}, wherein R^{b} and R^{c} are on adjacent ring vertices and are combined to form a four to six membered heterocyclyl having 0 to 2 additional heteroatom ring vertices selected from N, O, and S. In a first subembodiment of embodiment 17, the compound of Formula (I) or a pharmaceutically acceptable salt thereof is wherein ring B is 2-oxaspiro[3.5]non-6-en-7-yl, 2-oxaspiro[3.5]non-7-yl, 2-oxa-8-azaspiro[4.5]dec-8-yl, 9-oxa-3-azaspiro[5.5]undec-3-yl, 2-oxa-6-azaspiro[3.4]oct-6-yl, 1-oxa-7-azaspiro[3.5]non-7-yl, 1-oxa-8-azaspiro[4.5]dec-8-yl, 6-oxa-2-azaspiro[3.3]hept-2-yl, 2,8-diazaspiro[4.5]dec-8-yl, 7-oxa-3-azabicyclo[3.3.0]oct-3-yl, 8-oxa-3-azabicyclo[4.3.0]non-3-yl, 2-oxa-6-azaspiro[3.5]non-6-yl, 7-oxo-3,6,8-triazabicyclo[4.3.0]non-3-yl, 3-pyrrolino[3,4-c]pyrazol-2-yl, 3,6-diazabicyclo[3.1.1]hept-3-yl, 2,7-diazaspiro[3.5]non-7-yl, each ring optionally substituted with R^{a} where R^{a} is hydrogen or alkyl. In some embodiments, ring B is selected from the group consisting of 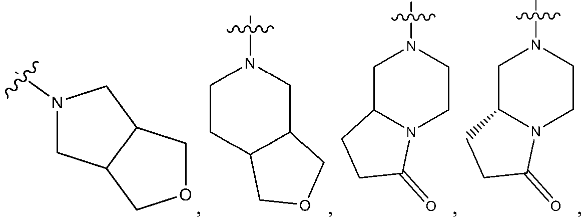 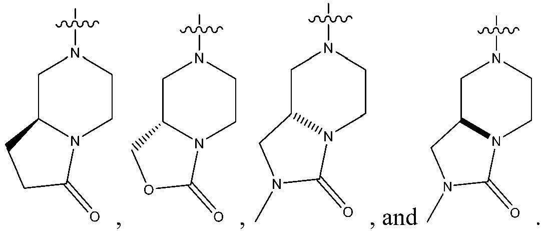
18. In embodiment 18, the compounds of Formula (I) of any one of embodiments 1 to 13, 16, and 17, and subembodiments contained therein, or a pharmaceutically acceptable salt thereof is wherein the compounds of Formula (I) are represented by Formula (Ia): 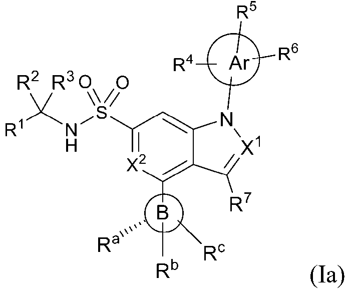 or a pharmaceutically acceptable salt thereof.
19. In embodiment 19, the compounds of Formula (I) of any one of embodiments 1 to 13, 16, and 17, and subembodiments contained therein, or a pharmaceutically acceptable salt thereof is wherein the compounds of Formula (I) are represented by Formula (Ib): 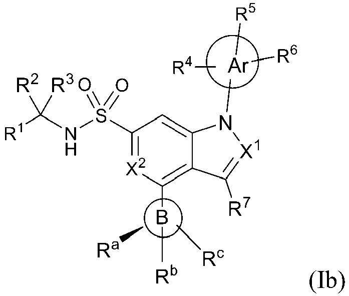 or a pharmaceutically acceptable salt thereof.
20. In embodiment 20, the compounds of Formula (I) of any one of embodiments 1 to 13, 16, and 17, and subembodiments contained therein, or a pharmaceutically acceptable salt thereof is wherein the compounds of Formula (I) are represented by Formula (Ic), Formula (Id), Formula (Ie), or Formula (If): 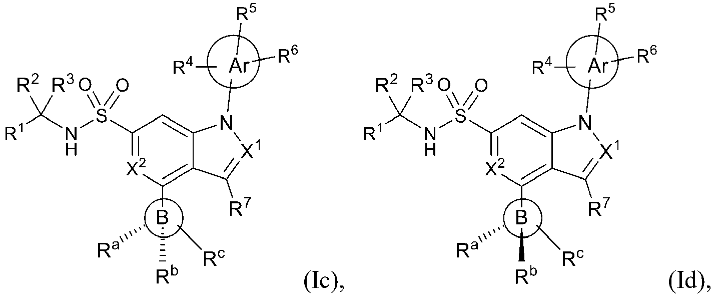 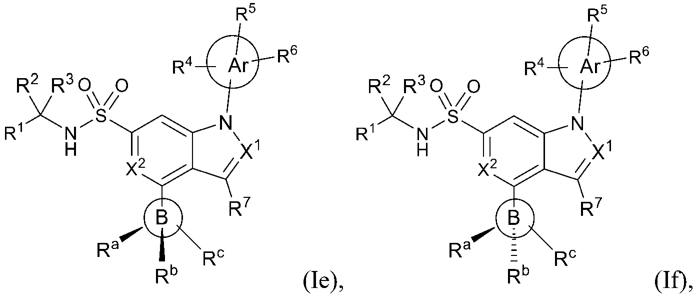 or a pharmaceutically acceptable salt thereof.
21. In embodiment 21, the compounds of Formula (I) of any one of embodiments 1 to 9, and 11 to 20 and subembodiments contained therein, or a pharmaceutically acceptable salt thereof is wherein Ar is not 1,3,4-oxadiazolyl.
22. In embodiment 22, the compounds of Formula (I) of any one of embodiments 1 to 9, and 11 to 20 and subembodiments contained therein, or a pharmaceutically acceptable salt thereof is wherein Ar is not 1,2,4-oxadiazolyl.
23. In embodiment 23, the compounds of Formula (I) of any one of embodiments 1 to 9 and 11 to 20 and subembodiments contained therein, or a pharmaceutically acceptable salt thereof is wherein Ar is not 1,2,4-oxadiazolyl and 1,3,4-oxadiazolyl.
24. In embodiment 24, the compounds of Formula (I) of any one of embodiments 1 to 9 and 11 to 23 and subembodiments contained therein, or a pharmaceutically acceptable salt thereof is wherein Ar is imidazolyl, isoxazolyl, pyrazolyl, 1,2,3-triazolyl, 1,2,4-triazolyl, thiazolyl, 1,2,4-thiadiazolyl, or 1,3,4-thiadiazolyl. In a first subembodiment of embodiment 24, the compound of Formula (I) or a pharmaceutically acceptable salt thereof is wherein Ar is 1,3,4-thiadiazol-2-yl.

### Biological Activity

The PARG enzyme and cell assays described in accompanying Example section may be used to measure the pharmacological effects of the compounds of the present invention.

Although the pharmacological properties of the compounds of Formula (I) vary with structural change, as expected, the compounds of the invention were found to be active in these PARG assays.

In general, the compounds of the invention demonstrate an IC₅₀ of 10 µM or less in the PARG enzyme assay described herein, with preferred compounds of the invention demonstrating an IC₅₀ of 1000nM or less, or 500 nM or less, and the most preferred compounds of the invention demonstrating an IC₅₀ of 200 nM or less.

In general, the compounds of the invention demonstrate an IC₅₀ of 1 µM or less in the PARG cell assay described herein, with preferred compounds of the invention demonstrating an IC₅₀ of 500 nM or less and the most preferred compounds of the invention demonstrating an IC₅₀ of 200 nM or less.

### PHARMACEUTICAL COMPOSITIONS

Also provided are pharmaceutical compositions which comprises a compound of Formula (I), or a pharmaceutically acceptable salt thereof, in association with a pharmaceutically acceptable diluent or carrier.

The compositions may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular, intraperitoneal or intramuscular dosing or as a suppository for rectal dosing).

The compositions may be obtained by conventional procedures using conventional pharmaceutical excipients, well known in the art. Thus, compositions intended for oral use may contain, for example, one or more coloring, sweetening, flavoring and/or preservative agents.

An effective amount of a compound of Formula (I) or a pharmaceutically salt thereof for use in therapy is an amount sufficient to treat or prevent a proliferative condition referred to herein, slow its progression and/or reduce the symptoms associated with the condition.

The amount of active ingredient that is combined with one or more excipients to produce a single dosage form will necessarily vary depending upon the individual treated and the particular route of administration. For example, a formulation intended for oral administration to humans will generally contain, for example, from 0.5 mg to 0.5 g of Formula (I) or a pharmaceutically salt thereof (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of the Formula (I) will naturally vary according to the nature and severity of the conditions, the age and sex of the animal or patient and the route of administration, according to well-known principles of medicine.

In using a compound of Formula (I) or a pharmaceutically salt thereof for therapeutic or prophylactic purposes it will generally be administered so that a daily dose in the range, for example, 0.1 mg/kg to 75 mg/kg body weight is received, given if required in divided doses. In general, lower doses will be administered when a parenteral route is employed. Thus, for example, for intravenous or intraperitoneal administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration may also be suitable, particularly in tablet form. Typically, unit dosage forms will contain about 0.5 mg to 0.5 g of a compound of this invention.

### THERAPEUTIC USES AND APPLICATIONS

Provided herein are compounds that function as inhibitors of PARG.

The present invention therefore provides a method of inhibiting PARG enzyme activity *in vitro* or *in vivo*, said method comprising contacting a cell with an effective amount of a of Formula (I) or a pharmaceutically salt thereof.

The present invention also provides a method of selectively inhibiting PARG enzyme activity over PARP1 or ARH3 enzyme activity *in vitro* or *in vivo*, said method comprising contacting a cell with an effective amount of compound of Formula (I) or a pharmaceutically salt thereof.

The present invention also provides a method of treating a disease or disorder in which PARG activity is implicated in a patient in need of such treatment, said method comprising administering to said patient a therapeutically effective amount of a compound of Formula (I) or a pharmaceutically salt thereof, or a pharmaceutical composition as defined herein.

Provided herein is a method of inhibiting cell proliferation, *in vitro* or *in vivo*, said method comprising contacting a cell with an effective amount of a compound of Formula (I) or a pharmaceutically salt thereof.

Provided herein is a method of treating a proliferative disorder in a patient in need of such treatment, said method comprising administering to said patient a therapeutically effective amount of a compound of Formula (I) or a pharmaceutically salt thereof, or a pharmaceutical composition as defined herein.

Provided herein is a method of treating cancer in a patient in need of such treatment, said method comprising administering to said patient a therapeutically effective amount of a compound of Formula (I) or a pharmaceutically salt thereof, or a pharmaceutical composition as defined herein.

Provided herein is a compound of Formula (I) or a pharmaceutically salt thereof, or a pharmaceutical composition as defined herein for use in therapy.

Provided herein is a compound of Formula (I) or a pharmaceutically salt thereof, or a pharmaceutical composition as defined herein for use in the treatment of a proliferative condition.

Provided herein is a compound of Formula (I) or a pharmaceutically salt thereof, or a pharmaceutical composition as defined herein for use in the treatment of cancer. In a particular embodiment, the cancer is human cancer.

Provided herein is a compound of Formula (I) or a pharmaceutically salt thereof, as defined herein for use in the inhibition of PARG enzyme activity.

Provided herein is a compound of Formula (I) or a pharmaceutically salt thereof, as defined herein for use in the treatment of a disease or disorder in which PARG activity is implicated.

Provided herein is a use of a compound of Formula (I) or a pharmaceutically salt thereof, as defined herein in the manufacture of a medicament for the treatment of a proliferative condition.

Provided herein is a use of a compound of Formula (I) or a pharmaceutically salt thereof, as defined herein in the manufacture of a medicament for the treatment of cancer. Suitably, the medicament is for use in the treatment of human cancers.

Provided herein is a use of a compound of Formula (I) or a pharmaceutically salt thereof in the manufacture of a medicament for the inhibition of PARG enzyme activity.

Provided herein is a use of a compound of Formula (I) or a pharmaceutically salt thereof in the manufacture of a medicament for the selective inhibition of PARG enzyme activity over PARP1 or ARH3 enzyme activity.

Provided herein is a use of a compound of Formula (I) or a pharmaceutically salt thereof in the manufacture of a medicament for the treatment of a disease or disorder in which PARG activity is implicated.

The term "proliferative disorder" are used interchangeably herein and pertain to an unwanted or uncontrolled cellular proliferation of excessive or abnormal cells which is undesired, such as, neoplastic or hyperplastic growth, whether in vitro or in vivo. Examples of proliferative conditions include, but are not limited to, pre-malignant and malignant cellular proliferation, including but not limited to, malignant neoplasms and tumors, cancers, leukemias, psoriasis, bone diseases, fibroproliferative disorders (e.g., of connective tissues), and atherosclerosis. Any type of cell may be treated, including but not limited to, lung, colon, breast, ovarian, prostate, liver, pancreas, brain, and skin.

The anti-proliferative effects of the compounds of Formula (I) or a pharmaceutically salt thereof have particular application in the treatment of human cancers (by virtue of their inhibition of PARG enzyme activity).

The anti-cancer effect may arise through one or more mechanisms, including but not limited to, the regulation of cell proliferation, the inhibition of angiogenesis (the formation of new blood vessels), the inhibition of metastasis (the spread of a tumor from its origin), the inhibition of invasion (the spread of tumor cells into neighboring normal structures), or the promotion of apoptosis (programmed cell death).

In a particular embodiment of the invention, the proliferative condition to be treated is cancer.

### ROUTES OF ADMINISTRATION

The compounds of Formula (I) or a pharmaceutically salt thereof or pharmaceutical compositions comprising these compounds may be administered to a subject by any convenient route of administration, whether systemically/ peripherally or topically (i.e., at the site of desired action).

Routes of administration include, but are not limited to, oral (e.g., by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eye drops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intra-arterial, intracardiac, intrathecal, intraspinal, intracapsular, subcapsular, intraorbital, intraperitoneal, intratracheal, subcuticular, intraarticular, subarachnoid, and intrasternal; by implant of a depot or reservoir, for example, subcutaneously or intramuscularly.

### COMBINATION THERAPIES

The antiproliferative treatment defined hereinbefore may be applied as a sole therapy or may involve, in addition to the compound of Formula (I) or a pharmaceutically salt thereof, conventional surgery or radiotherapy or chemotherapy. Such chemotherapy may include one or more of the following categories of anti-tumor agents: other antiproliferative/antineoplastic drugs and combinations thereof, as used in medical oncology, such as alkylating agents (for example cis-platin, oxaliplatin, carboplatin, cyclophosphamide, nitrogen mustard, melphalan, chlorambucil, busulphan, temozolamide and nitrosoureas); antimetabolites (for example gemcitabine and antifolates such as fluoropyrimidines like 5-fluorouracil and tegafur, raltitrexed, methotrexate, cytosine arabinoside, and hydroxyurea); antitumor antibiotics (for example anthracyclines like 30ecarbonat, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and 30ecarbo like taxol and taxotere and polokinase inhibitors); and topoisomerase inhibitors (for example epipodophyllotoxins like etoposide and teniposide, amsacrine, topotecan and camptothecin); cytostatic agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride; anti-invasion agents [for example c-Src kinase family inhibitors like 4-(6-chloro-2,3-methylenedioxyanilino)-7-[2-(4-methylpiperazin-1-yl)ethoxy]-5-tetrahydropyran-4-yloxyquinazoline (AZD0530; International Patent Application WO 01/94341), *N*-(2-chloro-6-methylphenyl)-2-{6-[4-(2-hydroxyethyl)piperazin-1-yl]-2-methylpyrimidin-4-ylamino}thiazole-5-carboxamide (30ecarbona, BMS-354825; J. Med. Chem., 2004, 47, 6658-6661) and bosutinib (SKI-606), and metalloproteinase inhibitors like marimastat, inhibitors of urokinase plasminogen activator receptor function or antibodies to Heparanase]; inhibitors of growth factor function: for example such inhibitors include growth factor antibodies and growth factor receptor antibodies (for example the anti-erbB2 antibody trastuzumab [Herceptin^{™}], the anti-EGFR antibody panitumumab, the anti-erbB1 antibody cetuximab [Erbitux, C225] and any growth factor or growth factor receptor antibodies disclosed by Stern et al. (Critical reviews in oncology/haematology, 2005, Vol. 54, pp11-29); such inhibitors also include tyrosine kinase inhibitors, for example inhibitors of the epidermal growth factor family (for example EGFR family tyrosine kinase inhibitors such as *N*-(3-chloro-4-fluorophenyl)-7-methoxy-6-(3-morpholinopropoxy)quinazolin-4-amine (gefitinib, ZD1839), *N*-(3-ethynylphenyl)-6,7-bis(2-methoxyethoxy)quinazolin-4-amine (erlotinib, OSI-774) and 6-acrylamido-*N*-(3-chloro-4-fluorophenyl)-7-(3-morpholinopropoxy)-quinazolin-4-amine (CI 1033), erbB2 tyrosine kinase inhibitors such as lapatinib); inhibitors of the hepatocyte growth factor family; inhibitors of the insulin growth factor family; inhibitors of the platelet-derived growth factor family such as imatinib and/or nilotinib (AMN107); inhibitors of serine/threonine kinases (for example Ras/Raf 31ecarbona inhibitors such as farnesyl transferase inhibitors, for example sorafenib (BAY 43-9006), tipifarnib (R115777) and lonafarnib (SCH66336)), inhibitors of cell 31ecarbona through MEK and/or AKT kinases, c-kit inhibitors, abl kinase inhibitors, PI3 kinase inhibitors, Plt3 kinase inhibitors, CSF-1R kinase inhibitors, IGF receptor (insulin-like growth factor) kinase inhibitors; aurora kinase inhibitors (for example AZD1152, PH739358, VX-680, MLN8054, R763, MP235, MP529, VX-528 AND AX39459) and cyclin dependent kinase inhibitors such as CDK2 and/or CDK4 inhibitors; antiangiogenic agents such as those which inhibit the effects of vascular endothelial growth factor, [for example the anti-vascular endothelial cell growth factor antibody bevacizumab (Avastin^{™}) and for example, a VEGF receptor tyrosine kinase inhibitor such as vandetanib (ZD6474), vatalanib (PTK787), sunitinib (SU11248), axitinib (AG-013736), pazopanib (GW 786034) and 4-(4-fluoro-2-methylindol-5-yloxy)-6-methoxy-7-(3-pyrrolidin-1-ylpropoxy)quinazoline (AZD2171; Example 240 within WO 00/47212), compounds such as those disclosed in International Patent Applications WO97/22596, WO 97/30035, WO 97/32856 and WO 98/13354 and compounds that work by other mechanisms (for example linomide, inhibitors of integrin αvβ3 function and angiostatin)]; vascular damaging agents such as Combretastatin A4 and compounds disclosed in International Patent Applications WO 99/02166, WO 00/40529, WO 00/41669, WO 01/92224, WO 02/04434 and WO 02/08213; an endothelin receptor antagonist, for example zibotentan (ZD4054) or atrasentan; antisense therapies, for example those which are directed to the targets listed above, such as ISIS 2503, an anti-ras antisense; gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and immunotherapy approaches, including for example *ex-vivo* and *in-vivo* approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

In a particular embodiment, the antiproliferative treatment defined hereinbefore may involve, in addition to the compound of Formula (I) or a pharmaceutically salt thereof, conventional surgery or radiotherapy or chemotherapy.

Such conjoint treatment may be achieved by way of the simultaneous, sequential or separate dosing of the individual components of the treatment. Such combination products employ the compounds of this invention within the dosage range described hereinbefore and the other pharmaceutically active agent within its approved dosage range.

According to this aspect of the invention there is provided a combination for use in the treatment of a cancer (for example a cancer involving a solid tumour) comprising a compound of Formula (I) or a pharmaceutically salt thereof, and another anti-tumour agent.

According to this aspect of the invention there is provided a combination for use in the treatment of a proliferative condition, such as cancer (for example a cancer involving a solid tumour), comprising a compound of Formula (I) or a pharmaceutically salt thereof, and any one of the anti-tumour agents listed herein above.

In a further aspect of the invention there is provided a compound of Formula (I) or a pharmaceutically salt thereof, for use in the treatment of cancer in combination with another anti-tumour agent, optionally selected from one listed herein above.

Herein, where the term "combination" is used it is to be understood that this refers to simultaneous, separate or sequential administration. In one aspect of the invention "combination" refers to simultaneous administration. In another aspect of the invention "combination" refers to separate administration. In a further aspect of the invention "combination" refers to sequential administration. Where the administration is sequential or separate, the delay in administering the second component should not be such as to lose the beneficial effect of the combination.

According to a further aspect of the invention there is provided a pharmaceutical composition which comprises a compound of Formula (I) or a pharmaceutically salt thereof, in combination with an anti-tumour agent (optionally selected from one listed herein above), in association with a pharmaceutically acceptable diluent or carrier.

### EXAMPLES

The following references (intermediates) and examples (final compounds) are put forth so as to provide those of ordinary skill in the art with a complete disclosure and description of how to make and use the present invention, and are not intended to limit the scope of what the inventors regard as their invention, nor are they intended to represent that the experiments below were performed or that they are all of the experiments that may be performed. It is to be understood that exemplary descriptions written in the present tense were not necessarily performed, but rather that the descriptions can be performed to generate data and the like of a nature described therein. Efforts have been made to ensure accuracy with respect to numbers used (e.g., amounts, temperature, etc.), but some experimental errors and deviations should be accounted for.

Unless indicated otherwise, parts are parts by weight, molecular weight is weight average molecular weight, temperature is in degrees Celsius (°C), and pressure is at or near atmospheric. Standard abbreviations are used, including the following: µg = microgram; µl or µL = microliter; mM = millimolar; µM = micromolar; aa = amino acid(s); Ac₂O = acetic anhydride; AcCl = acetylchloride; ACN = acetonitrile; AIBN = 2,2'-Azobis(2-methylpropionitrile); BID = twice daily; BINAP = 2,2'-bis(diphenylphosphino)-1 ,1 '-binaphthyl; Boc₂O or (Boc)₂O = di-tert-butyl carbonate; bp = base pair(s); BSA = bovine serum albumin; BW = body weight; d = doublet; dd = doublet of doublets; DEAD = diethyl azodicarboxylate; DIBAL = diisobutylaluminium hydride DIEA = N,N-diisopropylethylamine; DIPEA = N,N-diisopropylethylamine; dl or dL = deciliter; DMA = dimethylacetamide; DMAP = dimethylaminopyridine; DME = 1,2-dimethoxyethane; DMEM = Dulbeco's Modification of Eagle's Medium; DMF = N,N-dimethylformamide; DMSO = dimethylsulfoxide; dppf = 1,1'-Bis(diphenylphosphino)ferrocene; DTT = dithiothreitol; EDTA = ethylenediaminetetraacetic acid; ES = electrospray; EtOAc or EA = ethyl acetate; EtOH = ethanol; g = gram; h or hr = hour(s); HATU = 2-(1 H-7-azabenzotriazol-1-yl)-1,1,3,3-tetramethyluronium hexafluorophosphate; HEPES = 4-(2-hydroxyethyl)-1-piperazineethylanesulfonic acid; HOAc = acetic acid; HPLC = high performance liquid chromatography; HPLC = high pressure liquid chromatography; i.m. = intramuscular(ly); i.p. = intraperitoneal(ly); IHC = immunohistochemistry; IPA = isopropyl alcohol; kb = kilobase(s); kDa = kilodalton; kg = kilogram; l or L = liter; LC = liquid chromatography; LCMS = liquid chromatography and mass spectrometry; m / z = mass to charge ratio; M = molar; m = multiplet; MeCN = acetonitrile; MeOH = methanol; MeSO₂Cl = methanesulfonylchloride; mg = milligram; min = minute(s); min = minutes; ml or mL = milliliter; mM = millimolar; MS = mass spectrometry; MsCl = methanesulfonylchloride; N = normal; NADPH = nicotinamide adenine dinucleotide phosphate; NBS = N-bromosuccinamide; ng = nanogram; nm = nanometer; nM = nanomolar; NMP = N-methylpyrrolidone; NMR = nuclear magnetic resonance; ns = not statistically significant; nt = nucleotides(s); PBS = phosphate-buffered saline; Pd/C = palladium on carbon; Pd₂(dba)₃ = Tris(debenzylideneactone) dipalladium;Pd(dppf)Cl₂ = 1,1'-bis(diphenylphosphino)ferrocene-palladium(ll)dichloride; PE = petroleum ether; QD = daily; QM = monthly; QW = weekly; rac = racemic; Rt = retention time; s = singlet; s or sec = second(s); sat. = saturated; SC or SQ = subcutaneous(ly); t = triplet; TBAB = tetra-n-butylammonium bromide; TEA = triethylamine; TFA = trifluoroacetic acid; THF = tetrahydrofuran; TLC = thin layer chromatography; TMSCl = trimethylsilylchloride; TsOH = p-toluenesulfonic acid; U = unit; wt = wildtype.

### Reference 1

### Synthesis of 4-chloro-1-(5-(difluoromethyl)-1,3,4-thiadiazol-2-yl)-1H-indazole-6-sulfonyl chloride

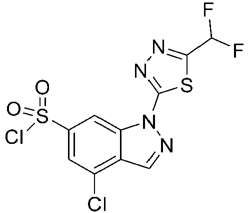

### Step 1: Synthesis of 6-(benzylthio)-4-chloro-1H-indazole

To a stirred mixture of 6-bromo-4-chloro-1H-indazole (15.00 g, 64.800 mmol, 1.00 equiv.) and phenylmethanethiol (24.14 g, 194.364 mmol, 3.00 equiv.) in 1,4-dioxane (150 mL) in a 500 mL 3-necked round-bottom flask, were added XantPhos (3.75 g, 6.481 mmol, 0.10 equiv.) and DIEA (25.13 g, 194.440 mmol, 3.00 equiv.) in portions at room temperature under nitrogen atmosphere. To the above mixture was added Pd₂(dba)₃₋CHCl₃ (2.97 g, 3.243 mmol, 0.05 equiv.) in portions at room temperature. The resulting mixture was stirred for additional 3h at 100 °C under nitrogen atmosphere. The residue was purified by silica gel column chromatography, eluted with DCM:MeOH (100:1), to afford the title compound (16.5 g).

### Step 2: Synthesis of 2-(6-(benzylthio)-4-chloro-1H-indazol-1-yl)-5-(difluoromethyl)-1,3,4-thiadiazole

To a stirred solution of 6-(benzylsulfanyl)-4-chloro-1H-indazole (5.1 g, 18.561 mmol, 1 equiv.) and 2-bromo-5-(difluoromethyl)-1,3,4-thiadiazole (7.98 g, 37.122 mmol, 2 equiv.) in DMF (100 mL) in a 250 mL 3-necked round-bottom flask, Cs₂CO₃ (6.05 g, 18.569 mmol, 1.00 equiv.) were added. The resulting mixture was stirred for 1h at 60 °C under nitrogen atmosphere. The reaction was quenched by the addition of water and the aqueous layer was extracted with EtOAc. The resulting mixture was concentrated under vacuum and the residue was purified by silica gel column chromatography, eluted with CH₂Cl₂ / MeOH (100/1), to afford the title compound (7 g).

### Step 3: Synthesis of 4-chloro-1-(5-(difluoromethyl)-1,3,4-thiadiazol-2-yl)-1H-indazole-6-sulfonyl chloride

To a stirred solution 2-(6-(benzylthio)-4-chloro-1H-indazol-1-yl)-5-(difluoromethyl)-1,3,4-thiadiazole (20.00 g, 48.915 mmol, 1.00 equiv.) and AcOH (2.5 mL) and H₂O(5.00 mL) in MeCN (200.00 mL) in a 500 mL 3-necked round-bottom flask, was added 1,3-dichloro-5,5-dimethylimidazolidine-2,4-dione (14.46 g, 73.394 mmol, 1.50 equiv.) in portions at 0 °C under nitrogen atmosphere. The resulting mixture was stirred for 2h at 0 °C under nitrogen atmosphere and then concentrated under vacuum to give the title compound as a brown solid (30 g, crude). The crude product was used in the next step directly without further purification.

### Reference 2

### Synthesis of 4-chloro-N-(1-cyanocyclopropyl)-1-[5-(difluoromethyl)-1,3,4-thiadiazol-2-yl]-1H-indazole-6-sulfonamide

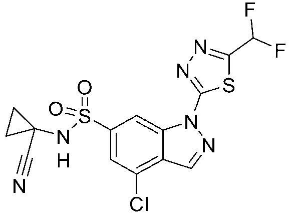

Into a 2L 3-necked round-bottom flask with pyridine (300 mL) were added 4-chloro-1-(5-(difluoromethyl)-1,3,4-thiadiazol-2-yl)-1H-indazole-6-sulfonyl chloride (30.00 g, 77.886 mmol, 1.00 equiv.) and 1-aminocyclopropane-1-carbonitrile hydrochloride salt (17.14 g, 77.904 mmol, 1.00 equiv.) at 0 °C. The resulting mixture was stirred overnight at room temperature under nitrogen atmosphere. The resulting mixture was concentrated under vacuum, then the pH value of the solution was adjusted to 5 with HCl (1 mol/L) and then extracted with ethyl acetate. The organic layers combined and concentrated under vacuum and the residue was purified by silica gel column chromatography, eluted with CH₂Cl₂:MeOH (50:1), to afford the title compound (17 g). LCMS (ES, m/z): [M+H]⁺ 431

### Reference 3

### Synthesis of 4-chloro-1-[5-(difluoromethyl)-1,3,4-thiadiazol-2-yl]-N-(1-methylcyclopropyl) indazole-6-sulfonamide

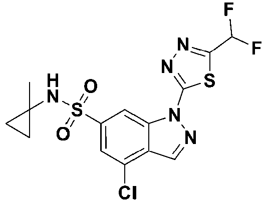

To a stirred solution of 1-methylcyclopropan-1-amine hydrochloride (0.50 g, 4.648 mmol, 1.20 equiv.) and pyridine (22.5 mL) in a 50 mL 3-necked round-bottom flask, 4-chloro-1-[5-(difluoromethyl)-1,3,4-thiadiazol-2-yl]indazole-6-sulfonyl chloride (1.50 g, 3.894 mmol, 1.00 equiv.) was added. The resulting mixture was stirred overnight at room temperature under nitrogen atmosphere and then concentrated under vacuum. pH value of the solution was adjusted to 5 with HCl (1 mol/L) and the resulting solution was extracted with ethyl acetate. The organic layers combined and concentrated under vacuum and the residue was purified by silica gel column chromatography, eluted with PE/EA (1/1), to afford the title compound (410 mg). LCMS (ES, m/z): [M+H]⁺ 420

### Reference 4

### Synthesis 4-chloro-N-(1-cyanocyclopropyl)-1-(5-methyl-1,3,4-thiadiazol-2-yl)-1H-indazole-6-sulfonamide

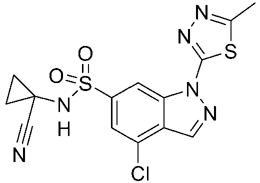

The title compound was prepared by following the proceeding as described in References 2 and 3 above replacing 2-bromo-5-(difluoromethyl)-1,3,4-thiadiazole with 2-bromo-5-methyl-1,3,4-thiadiazole in Step 2 of Reference 2.

### Reference 5

### Synthesis of 4-chloro-N-(1-cyanocyclopropyl)-1-(5-(difluoromethyl)-1,3,4-thiadiazol-2-yl)-1H-indole-6-sulfonamide

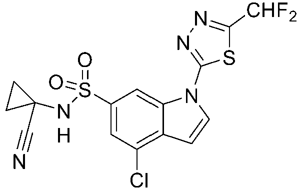

The title compound was prepared by proceeding as described in References 2 and 3 above but replacing 6-bromo-4-chloro-1H-indazole with 6-bromo-4-chloro-1H-indole in Step 2 of Reference 2.

### Example 1

### Synthesis of 1-[({1-[5-(difluoromethyl)(1,3,4-thiadiazol-2-yl)]-4-phenyl-1H-indazol-6-yl}sulfonyl)amino]cyclopropanecarbonitrile

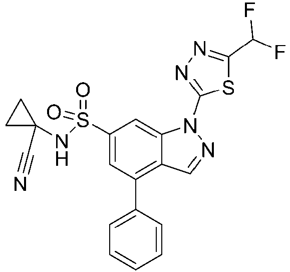

To a stirred solution of 4-chloro-N-(1-cyanocyclopropyl)-1-[5-(difluoromethyl)-1,3,4-thiadiazol-2-yl]-1H-indazole-6-sulfonamide (100 mg, 0.232 mmol, 1 equiv.), phenylboronic acid (141.51 mg, 1.161 mmol, 5.00 equiv.) and K₂CO₃ (64.16 mg, 0.464 mmol, 2 equiv.) in dioxane (1.5 mL) in an 8 mL vial, Pd(DtBPF)Cl₂ (15.13 mg, 0.023 mmol, 0.1 equiv.) was added at room temperature under a nitrogen atmosphere. The resulting mixture was stirred overnight at 80 °C under a nitrogen atmosphere and then quenched with water. The aqueous layer was extracted with EtOAc and the combined EtOAc extracts were concentrated under vacuum. The residue was purified by Prep-TLC (DCM:MeOH=70:1) to afford crude product (60 mg). The crude product was purified by Prep-HPLC (Column: XBridge Prep OBD C18 Column 30x150 mm 5 um; Mobile Phase A: water (0.05% NH₃·H₂O), Mobile Phase B: ACN; Flow rate: 60 mL/min; Gradient: 40% B to 53% B in 7 min; 254; 220 nm; RT 5.67 min.) to afford the title compound (29.4 mg,) as a white solid. LCMS (ES, m/z): [M+H]⁺ 473; ¹H-NMR (300MHz, DMSO-*d₆*, *ppm*) δ 1.29-1.51 (m, 4H), 7.46-7.71 (m, 4H), 7.7.83 (d, 2H), 7.98 (s, 1H), 8.93 (s, 1H), 9.04 (s, 1H), 9.50 (brs, 1H).

### Example 2

### Synthesis of 1-[({4-(1H-indazol-6-yl)-1-[5-(difluoromethyl)(1,3,4-thiadiazol-2-yl)]-1H-indazol-6-yl}sulfonyl)amino]cyclopropanecarbonitrile

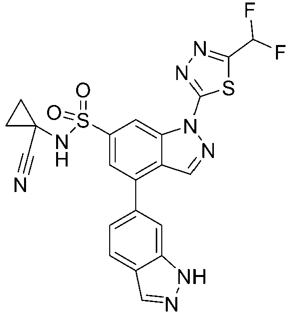

The title compound was prepared as described in Example 1 above by replacing phenylboronic acid with 1H-indazol-6-ylboronic acid. LCMS (ES, m/z): [M+H]⁺ 513; ¹H-NMR (300MHz, DMSO-*d₆*, *ppm*) δ 13.35 (s, 1H), 9.54 (s, 1H), 9.04 (s, 1H), 8.97 (s, 1H), 8.23 (s, 1H), 8.01-8.04 (m, 2H), 7.91(s, 1H), 7.46-7.82 (m, 2H), 1.34-1.54 (m, 4H).

### Example 3

### Synthesis of [4-(1-[5-(difluoromethyl)(1,3,4-thiadiazol-2-yl)]-6-{[(cyanocyclopropyl)amino]sulf onyl}(1H-indazol-4-yl))phenyl]-N,N-dimethylcarboxamide

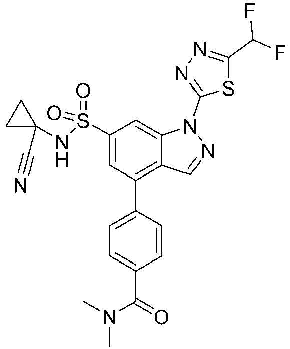

The title compound was prepared as described in Example 1 above by replacing phenylboronic acid with 4-(dimethylcarbamoyl) phenylboronic acid. LCMS (ES, m/z): [M+H]⁺ 544; ¹H-NMR (300MHz, DMSO-*d₆*, *ppm*) δ 9.52 (s, 1H), 9.06 (s, 1H), 8.98 (s, 1H), 8.00 (s, 1H), 7.90-7.87 (d, 2H), 7.82-7.46 (m, 3H), 3.04-3.01 (d, 6H), 1.52-1.34 (m, 4H).

### Example 4

### Synthesis of [3-(1-[5-(difluoromethyl)(1,3,4-thiadiazol-2-yl)]-6-{[(cyanocyclopropyl)amino] sulfonyl}(1H-indazol-4-yl))phenyl]-N,N-dimethylcarboxamide

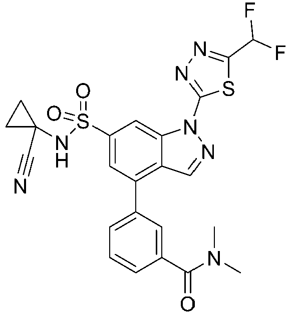

The title compound was prepared as described in Example 1 above by replacing phenylboronic acid with 3-(dimethylcarbamoyl)phenylboronic acid. LCMS (ES, m/z): [M+H]⁺ = 544; ¹H-NMR (400MHz, DMSO-d₆, *ppm*) δ 9.52 (s, 1H), 9.06 (s, 1H), 8.92 (s, 1H), 7.99 (s, 1H), 7.90-7.92 (d, 1H), 7.51-7.81 (m, 4H), 3.01-3.04 (d, 6H), 1.47-1.51 (m, 2H), 1.35-1.39 (m, 2H).

### Example 5

### Synthesis of N-(1-cyanocyclopropyl)-1-(5-(difluoromethyl)-1,3,4-thiadiazol-2-yl)-4-(1-methyl-6-oxo-1,6-dihydropyridin-3-yl)-1H-indazole-6-sulfonamide

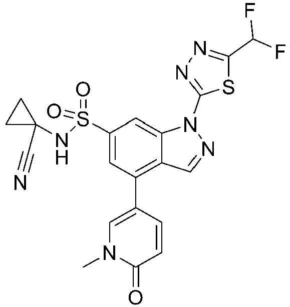

The title compound was prepared as described in Example 1 above by replacing phenylboronic acid with 1-methyl-6-oxopyridin-3-yl boronic acid. LCMS (ES, m/z): [M+H]⁺ 504; ¹H-NMR (300MHz, DMSO-d₆, *ppm*) δ 9.46 (s, 1H), 9.08 (d, 1H), 8.95 (s, 1H), 8.30 (d, 1H), 7.90-7.91 (m, 2H), 7.63-7.88 (t, 1H), 6.64 (d, 1H), 3.59 (s, 3H), 1.23-1.49 (m, 4H).

### Example 6

### Synthesis of 1-[({1-[5-(difluoromethyl)(1,3,4-thiadiazol-2-yl)]-4-cyclopropyl-1H-indazol-6-yl}sulfonyl)amino]cyclopropanecarbonitrile

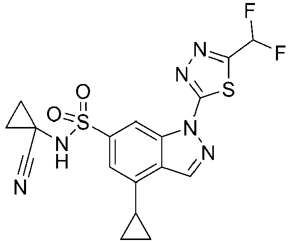

To a stirred solution of 4-chloro-N-(1-cyanocyclopropyl)-1-[5-(difluoromethyl)-1,3,4-thiadiazol-2-yl]-1H-indazole-6-sulfonamide (100 mg, 0.232 mmol, 1 equiv.) and cyclopropylboronic acid (99.69 mg, 1.161 mmol, 5 equiv.), K₂CO₃ (64.16 mg, 0.464 mmol, 2 equiv.) in dioxane (1.5 mL) in an 8 mL vial, Pd(DtBPF)Cl₂ (15.13 mg, 0.023 mmol, 0.1 equiv.) were added under nitrogen atmosphere. The resulting mixture was stirred overnight at 100 °C under nitrogen atmosphere and then quenched with water. The aqueous layer was extracted with EtOAc and the combined EtOAc extracts were concentrated under vacuum. The residue was purified by Prep-TLC (DCM:MeOH=80:1) to afford crude product (70 mg). The crude product was purified by Prep-HPLC (Column: XBridge Prep OBD C18 Column 30x150 mm 5 um; Mobile Phase A: water (0.05% NH₃·H₂O), Mobile Phase B: ACN; Flow rate: 60 mL/min; Gradient: 35% B to 47% B in 7 min; 254;220 nm; RT 5.68 min) to afford the title compound (31.3 mg). LCMS (ES, m/z): [M+H]⁺ 437; ¹H-NMR (300MHz, DMSO-d₆, *ppm*) δ 0.95-1.01 (m, 2H), 1.20-1.31 (m, 4H), 1.42-1.48 (d, 2H), 2.62-2.70 (m, 1H), 7.36 (s, 1H), 7.51-7.79 (t, 1H), 8.77 (s, 1H), 9.10 (s, 1H), 9.36 (brs, 1H).

### Example 7

### Synthesis of 1-[({1-[5-(difluoromethyl)(1,3,4-thiadiazol-2-yl)]-4-(2-oxaspiro[3.5]non-6-en-7-yl)-1H-indazol-6-yl}sulfonyl)amino]cyclopropanecarbonitrile

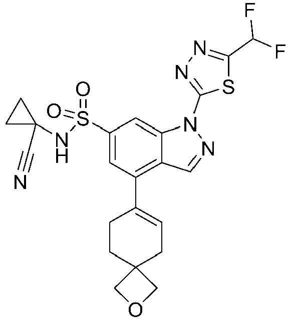

### Step 1: Synthesis of 2-oxaspiro[3.5]non-6-en-7-yl trifluoromethanesulfonate

To a stirred solution of 2-oxaspiro[3.5]nonan-7-one (1.00 g, 7.134 mmol, 1.00 equiv.) in THF (20 mL) were added LiHMDS (1432.36 mg, 8.560 mmol, 1.20 equiv.) dropwise at -78 °C under nitrogen atmosphere. After stirring the mixture for 2 h, 1,1,1-trifluoro-N-phenyl-N-trifluoromethanesulfonylmethanesulfonamide (3822.60 mg, 10.700 mmol, 1.50 equiv.) was added dropwise at -78 °C. The resulting mixture was stirred overnight at room temperature under nitrogen atmosphere and then quenched with saturated NH₄Cl (aq.). The product was extracted with EtOAc and dried over anhydrous Na₂SO₄. After filtration, the filtrate was concentrated under reduced pressure and the residue was purified by silica gel column chromatography, eluted with PE/EtOAc (10/1), to afford the title compound (1.2 g).

### Step 2: Synthesis of 4,4,5,5-tetramethyl-2-(2-oxaspiro[3.5]non-6-en-7-yl)-1,3,2-dioxaborolane

Into a 20-mL round-bottom flask purged and maintained with an inert atmosphere of nitrogen, was placed 2-oxaspiro[3.5]non-6-en-7-yl trifluoromethanesulfonate (200.00 mg, 0.735 mmol, 1.00 equiv.), bis(pinacolato)diboron (223.87 mg, 0.882 mmol, 1.2 equiv.), Pd(dppf)Cl₂ (53.75 mg, 0.073 mmol, 0.10 equiv.), KOAc (216.30 mg, 2.204 mmol, 3 equiv.) and dioxane (5.00 mL). The resulting solution was stirred for 12 h at 80 °C in an oil bath, concentrated under vacuum and used for next step directly without further purification.

### Step 3: Synthesis of 1-[({1-[5-(difluoromethyl)(1,3,4-thiadiazol-2-yl)]-4-(2-oxaspiro[3.5]non-6-en-7-yl)-1H-indazol-6-yl}sulfonyl)amino]cyclopropanecarbonitrile

Into an 8-mL round-bottom flask purged and maintained with an inert atmosphere of nitrogen, was placed 4,4,5,5-tetramethyl-2-(2-oxaspiro[3.5]non-6-en-7-yl)-1,3,2-dioxaborolane (200.00 mg, 0.800 mmol, 1.00 equiv.), 4-chloro-N-(1-cyanocyclopropyl)-1-[5-(difluoromethyl)-1,3,4-thiadiazol-2-yl]-1H-indazole-6-sulfonamide (344.46 mg, 0.800 mmol, 1 equiv.), Pd(PPh₃)₄ (92.39 mg, 0.080 mmol, 0.10 equiv.), KOAc (235.40 mg, 2.399 mmol, 3 equiv.), dioxane (2.00 mL), H₂O (2.00 mL). The resulting solution was stirred for 3h at 100 °C in an oil bath and then quenched with water. The resulting solution was extracted with EtOAc and the combined EtOAc extracts were washed with brine, dried over anhydrous sodium sulfate, filtered and concentrated. The residue was applied onto a silica gel column with ethyl acetate/petroleum ether (1/1). The crude product (170 mg) was purified by Prep-HPLC (Column: XBridge Prep OBD C₁₈ Column, 30×150 mm 5 um; Mobile Phase A: water (0.05% NH₃·H₂O), Mobile Phase B: ACN; Flow rate: 60 mL/min; Gradient:29% B to 50% B in 10 min; 254;220 nm; RT 8.28 min.) to afford the title compound (75 mg). LCMS (ES, *m*/*z*): [M+H]⁺ 519; ¹H-NMR-(300 MHz, DMSO-d₆, ppm) δ 9.43 (brs, 1H), 8.97 (s, 1H), 8.91 (s, 1H), 7.76 (s, 1H), 7.45-7.80 (t, 1H), 6.31 (s, 1H), 4.44 (dd, 4H), 2.66 (d, 4H), 2.08 (t, 2H), 1.32-1.52 (m, 4H).

### Example 8

### Synthesis of 1-[({1-[5-(difluoromethyl)(1,3,4-thiadiazol-2-yl)]-4-(2-oxaspiro[3.5]non-7-yl)-1H-indazol-6-yl}sulfonyl)amino]cyclopropanecarbonitrile

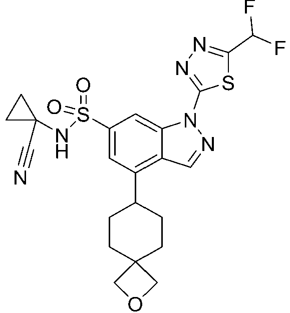

Into a 25-mL round-bottom flask purged and maintained with an inert atmosphere of nitrogen, was placed 1-[({1-[5-(difluoromethyl)(1,3,4-thiadiazol-2-yl)]-4-(2-oxaspiro[3.5]non-6-en-7-yl)-1H-indazol-6-yl}sulfonyl)amino]cyclopropanecarbonitrile (110.00 mg, 0.212 mmol, 1.00 equiv.), Pd/C (11.06 mg, 0.104 mmol, 0.49 equiv.), and MeOH (11.00 mL). The resulting solution was stirred for 12 h under hydrogen atmosphere at room temperature and then the solids were filtered out. The resulting mixture was concentrated and the residue was applied onto a silica gel column and eluted with ethyl acetate/petroleum ether (1/1). The crude product (75 mg) was purified by Prep-HPLC (Column: XBridge Prep OBD C18 Column, 30×150 mm 5 um; Mobile Phase A: water (10 mmol/L NH₄HCO₃), Mobile Phase B: ACN; Flow rate: 60 mL/min; Gradient: 40% B to 50% B in 7 min; 254;220 nm; RT 6.35 min.) to afford the title compound (2.9 mg). LCMS (ES, *m*/*z):* [M+H]⁺ 521; ¹H-NMR (300 MHz, DMSO-d₆, *ppm)* δ 9.29-9.44 (brs, 1H), 9.05 (s, 1H), 8.84 (s, 1H), 7.69 (s, 1H), 7.44-7.80 (t, 1H), 4.46 (s, 2H), 4.30 (s, 2H), 3.17-3.25 (m, 1H), 2.25 (d, 2H), 1.90 (d, 2H), 1.50-1.76 (m, 4H), 1.22-1.42 (m, 4H).

### Example 9

### Synthesis of 1-[({1-[5-(difluoromethyl)(1,3,4-thiadiazol-2-yl)]-4-imidazol-4-yl-1H-indazol-6-yl}sulfonyl)amino]cyclopropanecarbonitrile

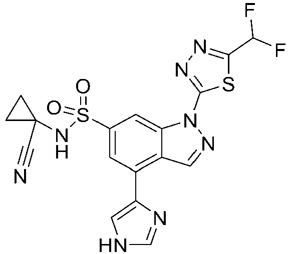

### Step 1: Synthesis of 1-[({1-[5-(difluoromethyl)(1,3,4-thiadiazol-2-yl)]-(1-trytyl-1H-imidazol-4-yl)-1H-indazol-6-yl}sulfonyl)amino]cyclopropanecarbonitrile

To a stirred solution of 4-chloro-N-(1-cyanocyclopropyl)-1-[5-(difluoromethyl)-1,3,4-thiadiazol-2-yl]-1H-indazole-6-sulfonamide (150 mg, 0.348 mmol, 1 equiv.) and 4-(tributylstannyl)-1-(triphenylmethyl)-1H-imidazole (417.42 mg, 0.696 mmol, 2 equiv.) in toluene (3 mL) in an 8 mL vial, Pd(PPh₃)₄ (80.47 mg, 0.070 mmol, 0.2 equiv.) was added under nitrogen atmosphere. The resulting mixture was stirred overnight at 110 °C under nitrogen atmosphere. The reaction was quenched by the addition of water/ice and the aqueous layer was extracted with EtOAc. The resulting mixture was concentrated under vacuum and the residue was purified by Prep-TLC (PE:EtOAc=2:1) to afford the title compound (200 mg).

### Step 2: Synthesis of 1-[({1-[5-(difluoromethyl)(1,3,4-thiadiazol-2-yl)]-4-imidazol-4-yl-1H-indazol-6-yl}sulfonyl)amino]cyclopropanecarbonitrile

To a stirred solution of 1-[({1-[5-(difluoromethyl)(1,3,4-thiadiazol-2-yl)]-(1-trytyl-1H-imidazol-4-yl)-1H-indazol-6-yl}sulfonyl)amino]cyclopropanecarbonitrile (190 mg, 0.270 mmol, 1 equiv.) in DCM (2 mL) and MeOH (2 mL) in an 8 mL vial, HCl (gas) in 1,4-dioxane (1 mL) was added at room temperature. The resulting mixture was stirred for 3h at room temperature and quenched with saturated NH₄Cl (aq.). The aqueous layer was extracted with DCM and the resulting mixture was concentrated under vacuum. The residue was purified by Prep-TLC (DCM:MeOH=25:1) to afford crude product (50 mg). The crude product was purified by Prep-HPLC (Column: XBridge Prep OBD C18 Column 30×150mm 5um;Mobile Phase A: water (0.05% NH₃·H₂O), Mobile Phase B: ACN; Flow rate: 60 mL/min; Gradient: 17% B to 27% B in 7 min; 254;220 nm; RT 6.08 min.) to afford the title compound (27.0 mg). LCMS (ES, m/z): [M+H]⁺ 463; ¹H-NMR (300MHz, DMSO-d₆, *ppm*) δ 1.24-1.34 (m, 2H), 1.44-1.49 (m, 2H), 7.63 (t, 1H), 7.97 (s, 1H), 8.17 (s, 1H), 8.32 (s, 1H), 8.84 (s, 1H), 9.35 (s, 1H), 9.48 (s, 1H), 12.70 (s, 1H).

### Example 10

### Synthesis of 1-[({1-[5-(difluoromethyl)(1,3,4-thiadiazol-2-yl)]-4-pyridazin-4-yl-1H-indazol-6-yl}sulfonyl)amino]cyclopropanecarbonitrile

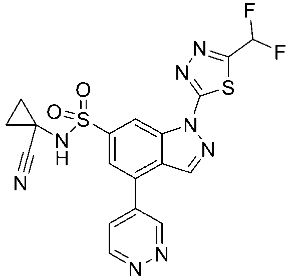

Into a 5-mL sealed tube, was placed 4-chloro-N-(1-cyanocyclopropyl)-1-[5-(difluoromethyl)-1,3,4-thiadiazol-2-yl]-1H-indazole-6-sulfonamide (200 mg, 0.464 mmol, 1 equiv.), DME (1.6 mL), H₂O (0.4 mL), 4-(tributylstannyl)pyridazine (342.72 mg, 0.928 mmol, 2 equiv.), and Pd(PPh₃)₄ (53.64 mg, 0.046 mmol, 0.1 equiv.). The final reaction mixture was irradiated with microwave radiation for 2h at 140 °C under nitrogen atmosphere. The resulting mixture was concentrated under vacuum and the residue was purified by Prep-TLC (CH₂Cl₂:MeOH=30:1) to afford crude product. The crude product (60 mg) was purified by Prep-HPLC (Column: XBridge Prep OBD C18 Column 30×150 mm 5 um; Mobile Phase A: water(0.05% NH₃·H₂O), Mobile Phase B: ACN; Flow rate: 60 mL/min; Gradient: 15% B to 26% B in 7 min; 254;220 nm; RT 6.55 min) to give the title compound (15 mg). LCMS (ES, m/z): [M+H] ⁺ 475; ¹H-NMR (300 MHz, DMSO-d₆, ppm) δ 9.73 (d, 1H), 9.48 (d, 1H), 9.15 (s, 1H), 9.10 (s, 1H), 8.15-8.18 (m, 2H), 7.64 (t, 1H), 1.45-1.50 (m, 2H), 1.23-1.36 (m, 2H).

### Example 11

### Synthesis of 1-[({1-[5-(difluoromethyl)(1,3,4-thiadiazol-2-yl)]-4-pyridazin-3-yl-1H-indazol-6-yl}sulfonyl)amino]cyclopropanecarbonitrile

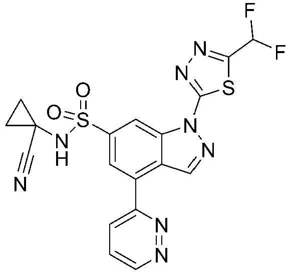

The title compound was prepared as described in Example 10 above by replacing 4-(tributylstannyl)pyridazine with 3-(tributylstannyl)pyridazine. LCMS (ES, m/z): [M+H]⁺ 475; ¹H-NMR (300 MHz, DMSO-d₆, *ppm*) δ 9.71 (s, 1H), 9.50 (d, 1H), 9.15 (s, 1H), 9.11 (d, 1H), 8.13-8.19 (m, 2H), 7.64 (t, 1H), 1.46-1.50 (m, 2H), 1.31-1.35 (m, 2H).

### Example 12

### Synthesis of 1-[({1-[5-(difluoromethyl)(1,3,4-thiadiazol-2-yl)]-4-(1-methylimidazol-4-yl)-1H-indazol-6-yl}sulfonyl)amino]cyclopropanecarbonitrile

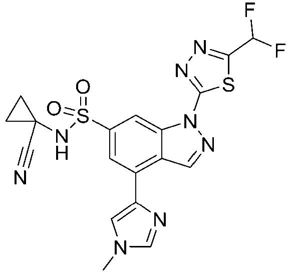

To a stirred solution of 4-chloro-N-(1-cyanocyclopropyl)-1-[5-(difluoromethyl)-1,3,4-thiadiazol-2-yl]-1H-indazole-6-sulfonamide (200 mg, 0.464 mmol, 1 equiv.) and 1-methyl-4-(tributylstannyl)-1H-imidazole (344.60 mg, 0.928 mmol, 2 equiv.) in toluene (4 mL) in an 8 mL vial and Pd(PPh₃)₄ (53.64 mg, 0.046 mmol, 0.1 equiv.) was added. The resulting mixture was stirred overnight at 110 °C under nitrogen atmosphere and the resulting mixture was concentrated under vacuum. The crude product was re-crystallized from DCM/PE=1/4 (5 mL) to afford crude product (200 mg, crude). The crude product was purified by Prep-HPLC (Column: XBridge Prep OBD C18 Column 19*250 mm,5 um; Mobile Phase A: water (0.05% NH₃·H₂O), Mobile Phase B: ACN; Flow rate: 20 mL/min; Gradient: 30% B to 30% B in 11 min; 254;220 nm; RT 9.08 min.) to afford the title compound (62.5 mg). LCMS (ES, m/z): [M+H]⁺ 477; ¹H-NMR (300MHz, DMSO-d₆, *ppm*) δ 1.22-1.43 (m, 4H), 3.78 (s, 3H),7.64 (t, 1H), 7.87 (s, 1H), 8.15 (s, 1H), 8.24 (s, 1H), 8.82 (s, 1H), 9.26 (s, 1H), 9.50 (brs, 1H).

### Example 13

### Synthesis of 1-[({1-[5-(difluoromethyl)(1,3,4-thiadiazol-2-yl)]-4-(1,3-thiazol-4-yl)-1H-indazol-6-yl}sulfonyl)amino]cyclopropanecarbonitrile

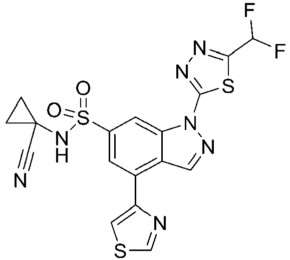

To a stirred solution of 4-chloro-N-(1-cyanocyclopropyl)-1-[5-(difluoromethyl)-1,3,4-thiadiazol-2-yl]-1H-indazole-6-sulfonamide (100 mg, 0.232 mmol, 1 equiv.) and 4-(tributylstannyl)-1,3-thiazole (173.70 mg, 0.464 mmol, 2 equiv.) in toluene (2 mL) in an 8 mL vial, Pd(PPh₃)₄ (26.82 mg, 0.023 mmol, 0.1 equiv.) was added. The resulting mixture was stirred overnight at 110 °C. The resulting mixture was concentrated under vacuum and the crude product was re-crystallized from DCM/PE (1:1, 2 mL) to crude product (70 mg, crude). The crude product was purified by Prep-HPLC (Column: SunFire C18 OBD Prep Column, 100Å, 5 µm, 19x250 mm; Mobile Phase A: water (1%HAC), Mobile Phase B: MeOH; Flow rate: 20 mL/min; Gradient: 75% B to 85% B in 7 min; 254/220 nm; RT 6.12 min.) to afford the title compound (36.1 mg). LCMS (ES, m/z): [M+H]⁺ 480; ¹H-NMR (300MHz, DMSO-d₆, *ppm*) δ 1.20-1.60 (m, 4H), 7.63 (t, 1H), 8.46 (s, 1H), 8.69 (s, 1H), 9.02 (s, 1H), 9.30 (s, 1H), 9.43 (s), 9.51-9.62 (brs, 1H).

### Example 14

### Synthesis of 1-[({1-[5-(difluoromethyl)(1,3,4-thiadiazol-2-yl)]-4-(2-pyridyl)-1H-indazol-6-yl}sulfonyl)amino]cyclopropanecarbonitrile

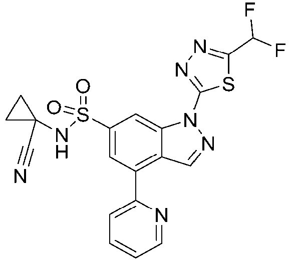

The title compound was prepared as described in Example 12 above by replacing1-methyl-4-(tributylstannyl)-1H-imidazole with 2-(tributylstannyl)pyridine. LCMS (ES, m/z): [M+H] ⁺ 474; ¹H-NMR (400 MHz, DMSO-d₆, *ppm*) δ 9.55 (s, 1H), 9.30 (s, 1H), 9.09 (s, 1H), 8.89 (d, 1H), 8.40 (d, 1H), 8.07-8.20 (m, 2H), 7.64 (t, 1H), 7.57-7.60 (m, 1H), 1.35-1.47 (m, 4H).

### Example 15

### Synthesis of 1-[({1-[5-(difluoromethyl)(1,3,4-thiadiazol-2-yl)]-4-pyrimidin-4-yl-1H-indazol-6-yl}sulfonyl)amino]cyclopropanecarbonitrile

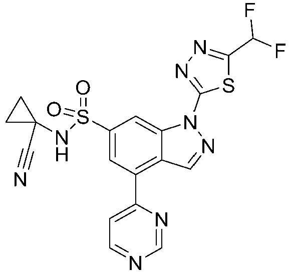

The title compound was prepared as described in Example 12 above by replacing 1-methyl-4-(tributylstannyl)-1H-imidazole with 4-(tributylstannyl)pyrimidine. LCMS (ES, m/z): [M+H] ⁺ 475; ¹H-NMR (300 MHz, DMSO-d₆, *ppm*) δ 1.33-1.51 (m, 4H), 7.64 (t, 1H), 8.30 (d, 1H), 8.53 (s, 1H), 9.09 (d, 1H), 9.20 (s, 1H), 9.36 (s, 1H), 9.47(s, 1H), 9.60 (s, 1H).

### Example 16

### Synthesis of 1-[({1-[5-(difluoromethyl)(1,3,4-thiadiazol-2-yl)]-4-(4-pyridyl)-1H-indazol-6-yl}sulfonyl)amino]cyclopropanecarbonitrile

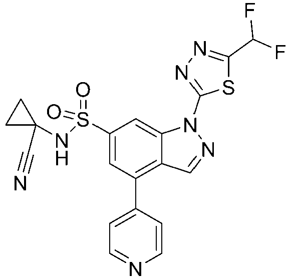

The title compound was prepared as described in Example 12 above by replacing 1-methyl-4-(tributylstannyl)-1H-imidazole with 4-(tributylstannyl)pyridine. LCMS (ES, m/z): [M+H] ⁺ 474; ¹H-NMR (300 MHz, DMSO-d₆, *ppm*) δ 9.10 (s, 1H), 9. 01 (s, 1H), 8.83 (s, 2H), 8.04 (s, 1H), 7.46-7.84 (m, 3H), 1.23-1.57(m, 4H).

### Example 17

### Synthesis of1-[({1-[5-(difluoromethyl)(1,3,4-thiadiazol-2-yl)]-4-(3-pyridyl)-1H-indazol-6-yl}sulfonyl)amino]cyclopropanecarbonitrile

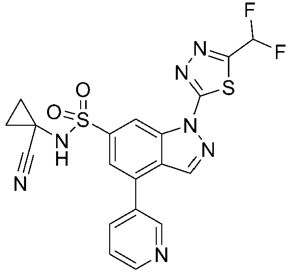

Into a 15-mL sealed tube purged and maintained with an inert atmosphere of nitrogen, were placed 4-chloro-N-(1-cyanocyclopropyl)-1-[5-(difluoromethyl)-1,3,4-thiadiazol-2-yl]-1H-indazole-6-sulfonamide (200 mg, 0.464 mmol, 1 equiv.), 3-(tributylstannyl)pyridine (341.81 mg, 0.928 mmol, 2 equiv.), toluene (2 mL), and Pd(PPh₃)₄ (53.64 mg, 0.046 mmol, 0.1 equiv.) under nitrogen atmosphere. The resulting solution was stirred for 1 overnight at 110°C under nitrogen atmosphere and then concentrated under reduced pressure. The residue was dissolved in CH₂Cl₂ (5 mL) and purified by Prep-TLC (CH₂Cl₂:MeOH = 50:1) to afford crude product (100 mg, crude) as a white solid. The crude product was purified by Prep-HPLC (Column: xbridge Prep OBD C18 Column 19*250 mm, 5 um; mobile phase A: water(10 mmol/L NH₄HCO₃), mobile phase B: ACN; flow rate: 20 mL/min; gradient: 39% B to 46% B in 10 min; 254;220 nm; rt: 8.38 min) to afford the title compound (15.1 mg). LCMS (ES, m/z): [M+H]⁺ 474; ¹H-NMR (400 MHz, DMSO-d₆, ppm) δ 9.54 (s, 1H), 9.08 (s, 1H), 9.03-9.01 (m, 2H), 8.79 (d, 1H), 8.26 (d, 1H), 8.02 (s, 1H), 7.67-7.70 (m, 1H), 7.65 (t, 1H), 1.50-1.47 (m, 2H), 1.37-1.34 (m, 2H).

### Example 18

### Synthesis of 1-[({1-[5-(difluoromethyl)(1,3,4-thiadiazol-2-yl)]-4-pyrazin-2-yl-1H-indazol-6-yl}sulfonyl)amino]cyclopropanecarbonitrile

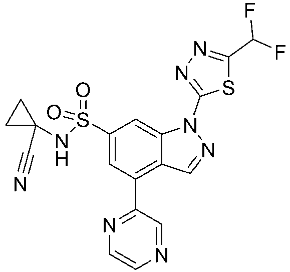

To a stirred solution of 4-chloro-N-(1-cyanocyclopropyl)-1-[5-(difluoromethyl)-1,3,4-thiadiazol-2-yl]-1H-indazole-6-sulfonamide (200 mg, 0.464 mmol, 1 equiv.) and 2-(tributylstannyl)pyrazine (342.72 mg, 0.928 mmol, 2 equiv.) in toluene (4 mL) in an 8 mL vial, Pd(PPh₃)₄ (53.64 mg, 0.046 mmol, 0.1 equiv.) was added under nitrogen atmosphere. The resulting mixture was stirred overnight at 110 °C under nitrogen atmosphere. The reaction was quenched with water and the aqueous layer was extracted with EtOAc. The resulting mixture was concentrated under vacuum and the residue was taken up in DCM/hexane (1:9, 10 mL) to provide a crude product as crystalline solid (100 mg). The crude product was purified by Prep-HPLC (Column: XSelect CSH Prep C18 OBD Column, 5 um, 19*150 mm; Mobile Phase A: water (1%HAC), Mobile Phase B: ACN; Flow rate: 20 mL/min; Gradient: 38% B to 63% B in 7 min; 254/220 nm; RT 6.83 min.) to afford the title compound (54.7 mg). LCMS (ES, m/z): [M+H]⁺ 475; ¹H-NMR (300MHz, DMSO-d₆, *ppm*) δ 1.32-1.39 (m, 2H), 1.43-1.52 (m, 2H), 7.65 (t, 1H), 8.51 (d, 1H), 8.85 (d, 1H), 8.95 (m, 1H), 9.16 (s, 1H), 9.28 (s, 1H), 9.46 (d, 1H), 9.56 (s, 1H).

### Example 19

### Synthesis of 1-[({1-[5-(difluoromethyl)(1,3,4-thiadiazol-2-yl)]-4-(6-methoxypyrimidin-4-yl)-1H-indazol-6-yl}sulfonyl)amino]cyclopropanecarboxamide

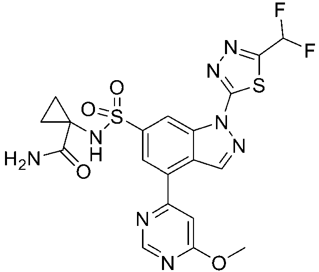

Into a 15-mL sealed tube purged and maintained with an inert atmosphere of nitrogen, were placed 4-methoxy-6-(tributylstannyl)pyrimidine (1389.76 mg, 3.482 mmol, 3.00 equiv.), 1-[1-[5-(difluoromethyl)-1,3,4-thiadiazol-2-yl]-4-(2-methoxypyrimidin-4-yl)indazole-6-sulfonamido]cyclopropane-1-carboxamide (500 mg, 1.161 mmol, 1.00 equiv.), toluene (5 mL) and Pd(PPh₃)₄ (201.16 mg, 0.174 mmol, 0.15 equiv.). The resulting solution was stirred for 1 overnight at 110 °C and then quenched with water. The resulting solution was extracted with ethyl acetate, washed with water and dried over anhydrous sodium sulfate. The resulting mixture was concentrated under vacuum and the residue was purified by Prep-TLC (CH₂Cl₂:MeOH=50:1) to afford crude product. The crude product (20 mg) was purified by Prep-HPLC (Column: XBridge Prep OBD C18 Column 19*250 mm, 5 um; Mobile Phase A: water (10 mmol/L NH₄HCO₃), Mobile Phase B: ACN; Flow rate: 20 mL/min; Gradient: 47% B to 57% B in 8 min; 254;220 nm; RT 5.83 min) to afford the title compound (3.3 mg). LCMS (ES, m/z): [M+H] ⁺ 523; ¹H-NMR (300 MHz, DMSO-d₆, *ppm*) δ 9.28 (s, 1H), 9.06 (s, 1H), 8.92 (brs, 1H), 8.41 (s, 1H), 7.86 (d, 1H), 7.64 (t, 1H), 7.08 (d, 2H), 4.05 (s, 3H), 1.14-1.18 (m, 2H), 0.88-0.98 (m, 2H).

### Example 20

### Synthesis of 1-[({1-[5-(difluoromethyl)(1,3,4-thiadiazol-2-yl)]-4-(2-methoxypyrimidin-4-yl)-1H-indazol-6-yl}sulfonyl)amino]cyclopropanecarboxamide

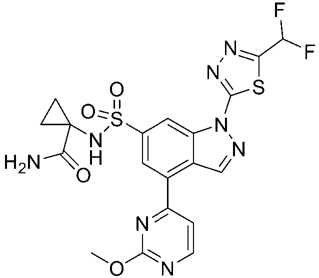

The title compound was prepared as described in Example 19 above by replacing 4-methoxy-6-(tributylstannyl)pyrimidine with 2-methoxy-4-(tributylstannyl)pyrimidine. LCMS (ES, m/z): [M+H] ⁺ 523; ¹H-NMR (300 MHz, DMSO-d₆, *ppm)* δ 9.31 (s, 1H), 9.09 (s, 1H), 8.87 (d, 1H), 8.42 (s, 1H), 7.86 (d, 1H), 7.64 (t, 1H), 7.12 (d, 2H), 4.10 (s, 3H), 1.14-1.18 (m, 2H), 0.88-0.98 (m, 2H).

### Example 21

### Synthesis of 1-[({1-[5-(difluoromethyl)(1,3,4-thiadiazol-2-yl)]-4-(2-methoxypyrimidin-4-yl)-1H-indazol-6-yl}sulfonyl)amino]cyclopropanecarbonitrile

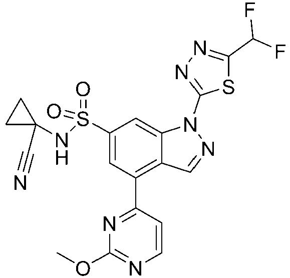

Into an 8-mL sealed tube, were placed 1-[1-[5-(difluoromethyl)-1,3,4-thiadiazol-2-yl]-4-(2-methoxypyrimidin-4-yl)indazole-6-sulfonamido]cyclopropane-1-carboxamide (14.00 mg, 0.033 mmol, 1.00 equiv.), piperidine (0.20 mL), and TFAA (20.50 mg, 0.098 mmol, 3 equiv.). The resulting solution was stirred for 4h at 0 °C under nitrogen atmosphere.
The resulting mixture was concentrated under vacuum and the residue was purified by Prep-TLC (CH₂Cl₂:MeOH=30:1) to afford crude product. The crude product (12 mg) was purified by Prep-HPLC (Column: XBridge Prep OBD C18 Column 30×150 mm 5 um; Mobile Phase A: water (0.05% NH₃·H₂O), Mobile Phase B: ACN; Flow rate: 60 mL/min; Gradient: 20% B to 40% B in 7 min; 254;220 nm; RT 6.22 min) to afford the title compound (2.1 mg). LCMS (ES, m/z): [M+H]⁺ 505; ¹H-NMR (300 MHz, DMSO-d₆, *ppm*) δ 9.48-9.70 (brs, 1H), 9.35 (s, 1H), 9.19 (s, 1H), 8.90 (d, 1H), 8.50 (s, 1H), 7.91(d, 1H), 7.65 (t, 1H), 4.11(s, 3H), 1.32-1.50(m, 4H).

### Example 22

### Synthesis of 1-[({1-[5-(difluoromethyl)(1,3,4-thiadiazol-2-yl)]-4-(6-methoxypyrimidin-4-yl)-1H-indazol-6-yl}sulfonyl)amino]cyclopropanecarbonitrile

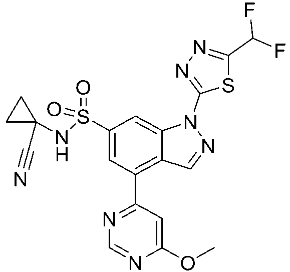

Into an 8-mL sealed tube, were placed 1-[1-[5-(difluoromethyl)-1,3,4-thiadiazol-2-yl]-4-(6-methoxypyrimidin-4-yl)-1H-indazole-6-sulfonamido]cyclopropane-1-carboxamide (90.00 mg, 0.172 mmol, 1.00 equiv.), DCM (0.90 mL), Et₃N (104.3 mg, 1.033 mmol, 6 equiv.), and TFAA (108.36 mg, 0.516 mmol, 3 equiv.). The resulting solution was stirred for 3 h at room temperature under nitrogen atmosphere and the resulting mixture was concentrated under vacuum. The residue was purified by Prep-TLC (CH₂Cl₂:MeOH=30:1) to afford crude product. The crude product (50 mg) was purified by Prep-HPLC (Column: XBridge Prep OBD C18 Column 30×150 mm 5 um; Mobile Phase A: water (10 mmol/L NH₄HCO₃), Mobile Phase B: ACN; Flow rate: 20 mL/min; Gradient: 55% B to 68% B in 8 min; 254;220 nm; RT 6.22 min) to afford the title compound (3.2 mg, 4.14%) as a white solid. LCMS (ES, m/z): [M+H]⁺ 505; ¹H-NMR (300 MHz, DMSO-d₆, *ppm*) δ 9.32 (s, 1H), 9.17 (s, 1H), 9.06 (s, 1H), 8.49 (s, 1H), 7.46-7.81 (m, 2H), 4.06 (s, 3H), 1.30-1.53 (m, 4H).

### Example 23

### Synthesis of ({1-[5-(difluoromethyl)(1,3,4-thiadiazol-2-yl)]-4-(2-oxa-7-azaspiro[3.5]non-7-yl)(1H-indazol-6-yl)}sulfonyl)(methylcyclopropyl)amine

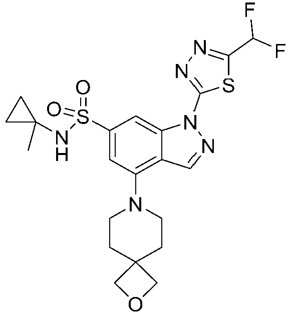

To a stirred solution of 4-chloro-1-[5-(difluoromethyl)-1,3,4-thiadiazol-2-yl]-N-(1-methylcyclopropyl)indazole-6-sulfonamide (250.00 mg, 0.595 mmol, 1.00 equiv.) and 2-oxa-7-azaspiro[3.5]nonane oxalic acid salt (258.69 mg, 1.191 mmol, 2.00 equiv.) in dioxane (2.50 mL) in an 8 mL vial, BINAP (185.39 mg, 0.298 mmol, 0.5 equiv.), Cs₂CO₃ (873.04 mg, 2.680 mmol, 4.5 equiv.), and Pd(AcO)₂ (53.47 mg, 0.238 mmol, 0.4 equiv.) were added. The resulting mixture was stirred for overnight at 100 °C under nitrogen atmosphere and then quenched with saturated NH₄Cl (aq.). The aqueous layer was extracted with EA and the resulting mixture was concentrated under vacuum. The residue was purified by Prep-TLC (DCM:MeOH=25:1) to afford 150 mg crude product as a yellow solid. The crude product was purified by Prep-HPLC (Column: XBridge Prep OBD C₁₈ Column, 30×150 mm 5 um; Mobile Phase A: water (10 mmol/l NH₄HCO₃), Mobile Phase B: ACN; Flow rate: 60 mL/min; Gradient:44% B to 54% B in 7 min; 254;220 nm; RT 7.02 min.) to afford the title compound (9.4 mg). LCMS (ES, m/z): [M+H]⁺: 511; ¹H-NMR (300 MHz, DMSO-*d*₆, *ppm)* δ 8.86 (s, 1H), 8.43 (s, 1H), 8.30 (s,1H), 7.61 (t, 1H), 7.16 (s, 1H), 4.10 (s, 4H), 3.34-3.36 (m, 4H), 2.02-2.08 (m, 4H), 1.07 (s, 3H), 0.65(t, 2H), 0.40 (t, 2H).

### Example 24

### Synthesis of [4-(1-[5-(difluoromethyl)(1,3,4-thiadiazol-2-yl)]-6-{[(methylcyclopropyl)amino] sulfonyl}(1H-indazol-4-yl))piperazinyl]-N,N-dimethylcarboxamide

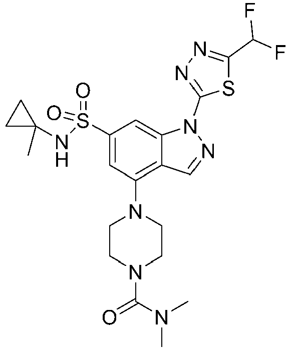

To a stirred solution of 4-chloro-1-[5-(difluoromethyl)-1,3,4-thiadiazol-2-yl]-N-(1-methylcyclopropyl)indazole-6-sulfonamide (500.00 mg, 1.191 mmol, 1.00 equiv.) and N,N-dimethylpiperazine-1-carboxamide (374.46 mg, 2.382 mmol, 2.00 equiv.) in THF (3.00 mL) in an 8 mL vial, Pd(OAc)₂ (106.95 mg, 0.476 mmol, 0.4 equiv.), Ruphos (222.29 mg, 0.476 mmol, 0.4 equiv.) and t-BuONa (286.12 mg, 2.977 mmol, 2.5 equiv.) were added under nitrogen atmosphere. The resulting mixture was stirred for overnight at 70 °C under nitrogen atmosphere and then quenched with saturated NH₄Cl (aq.). The aqueous layer was extracted with EA and the organics were concentrated under vacuum, The residue was purified by Prep-TLC (DCM:MeOH=25:1) to afford 100 mg crude product as a yellow solid, The crude product was purified by Prep-HPLC (Column: XBridge Shield RP18 OBD Column, 30*150 mm, 5 um; Mobile Phase A: (sater10 mmol/l NH₄HCO₃), Mobile Phase B:ACN; Flow rate: 60 mL/min; Gradient:45% B to 55% B in 7 min; 254;220 nm; RT 6.38 min) afford the title compound (2.4 mg). LCMS (ES, m/z): [M+H]⁺: 541; ¹H-NMR (300 MHz, DMSO-*d*₆, *ppm*) δ 8.94 (s, 1H), 8.47 (s, 1H), 8.32 (s, 1H), 7.61 (t, 1H), 7.18 (s, 1H), 3.35-3.50 (m, 8H), 2.81 (s, 6H), 1.08 (s, 3H), 0.66 (t, 2H), 0.41 (t, 2H).

### Example 25

### Synthesis of 1-[({1-[5-(difluoromethyl)(1,3,4-thiadiazol-2-yl)]-4-(2-oxa-7-azaspiro[3.5]non-7-yl)-1H-indazol-6-yl}sulfonyl)amino]cyclopropanecarbonitrile

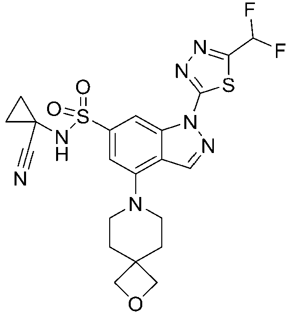

To a stirred solution of 4-chloro-N-(1-cyanocyclopropyl)-1-[5-(difluoromethyl)-1,3,4-thiadiazol-2-yl]indazole-6-sulfonamide (200.00 mg, 0.464 mmol, 1.00 equiv.) and 2-oxa-7-azaspiro[3.5]nonane; formic acid (160.82 mg, 0.928 mmol, 2.00 equiv.) in dioxane (2.00 mL) in an 8 mL vial, Cs₂CO₃ (605.01 mg, 1.857 mmol, 4.00 equiv.) , RuPhos (43.32 mg, 0.093 mmol, 0.20 equiv.) and RuPhos Palladacycle Gen.3 (38.83 mg, 0.046 mmol, 0.10 equiv.) were added under nitrogen atmosphere. The resulting mixture was stirred for 4h at 100 °C under nitrogen atmosphere and then quenched with saturated NH₄Cl (aq.). The aqueous layer was extracted with EtOAc and the organics were concentrated under reduced pressure. The residue was purified by Prep-TLC (DCM:MeOH=30:1). The crude product was purified by Prep-HPLC (Column: XBridge Prep OBD C18 Column 19*250 mm, 5 um; Mobile Phase A: water (10 mmol/L NH₄HCO₃), Mobile Phase B: ACN; Flow rate: 20 mL/min; Gradient: 50% B to 65% B in 8 min; 254; 220 nm; RT 6.88 min.) to afford the title compound (13.1 mg). LCMS (ES, m/z): [M+H]⁺ 522; ¹H-NMR (400MHz, DMSO-d₆, *ppm*) δ 1.30-1.33 (m, 2H), 1.43-1.46 (m, 2H), 2.04-2.05 (m, 4H), 3.34-3.37 (m, 4H), 4.40 (s, 4H), 7.17 (s, 1H), 7.61 (t, 1H), 8.46 (s, 1H), 8.90 (s, 1H), 9.34 (s, 1H).

### Example 26

### Synthesis of 1-[({4-((2R)-2-methylmorpholin-4-yl)-1-[5-(difluoromethyl)(1,3,4-thiadiazol-2-yl)]-1H-indazol-6-yl}sulfonyl)amino]cyclopropanecarbonitrile

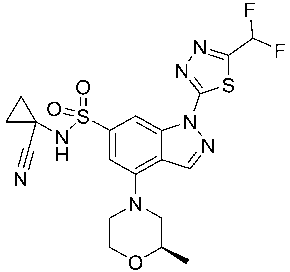

Into a 15-mL sealed tube, were placed 4-chloro-N-(1-cyanocyclopropyl)-1-[5-(difluoromethyl)-1,3,4-thiadiazol-2-yl]-1H-indazole-6-sulfonamide (500.00 mg, 1.161 mmol, 1.00 equiv.), (2R)-2-methylmorpholine (234.78 mg, 2.321 mmol, 2 equiv.), dioxane (5.00 mL), Cs₂CO₃ (756.26 mg, 2.321 mmol, 2 equiv.), RuPhos (108.31 mg, 0.232 mmol, 0.2 equiv.), and RuPhos Palladacycle Gen.3 (97.06 mg, 0.116 mmol, 0.10 equiv.) under nitrogen atmosphere. The resulting solution was stirred for 1 overnight at 100 °C under nitrogen atmosphere and then quenched with saturated NH₄Cl (aq.). The resulting mixture was extracted with EtOAc and the combined organic layers were concentrated under reduced pressure. The residue was purified by Prep-TLC (CH₂Cl₂:MeOH=30:1) to afford the crude product. The crude product (60 mg) was purified by Prep-HPLC (Column: Xbridge Prep OBD C18 Column 30×150 mm 5 um; mobile phase A: water (0.05% NH₃·H₂O), mobile phase B: ACN; flow rate: 60 mL/min; gradient: 27% B to 37% B in 7 min; 254;220 nm; rt: 6.50 min) to afford the title compound (19.3 mg). LCMS (ES, m/z): [M+H]⁺ 496; ¹H-NMR (300MHz, DMSO-d₆, *ppm*) δ 9.34 (brs, 1H), 9.00(s, 1H), 8.52 (s, 1H), 7.61 (t, 1H), 7.18 (s, 1H), 3.96-4.02 (m, 1H), 3.68-3.84 (m, 4H), 3.04 (t, 1H), 2.73 (t, 1H), 1.50-1.41 (m, 2H), 1.36-1.28 (m, 2H), 1.21 (d, 3H).

### Example 27

### Synthesis of 1-[({1-[5-(difluoromethyl)(1,3,4-thiadiazol-2-yl)]-4-(4-hydroxypiperidyl)-1H-indazol-6-yl}sulfonyl)amino]cyclopropanecarbonitrile

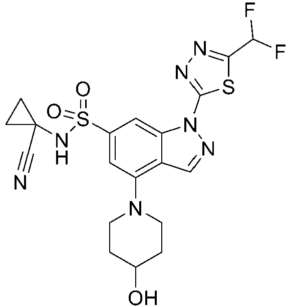

The title compound was prepared as described in Example 26 by replacing (2R)-2-methylmorpholine with piperidin-4-ol. LCMS (ES, *m*/*z*): [M+H]⁺ 496; ¹H-NMR (300MHz, DMSO-d₆, *ppm*) δ 1.31 (t, 2H), 1.41-1.46 (m, 2H), 1.62-1.68 (m, 2H), 1.90-1.98 (m, 2H), 3.12-3.23 (m, 2H), 3.70-3.79 (m, 3H), 4.82 (d, 1H), 7.15 (d, 1H), 7.61 (t, 1H), 8.44 (s, 1H), 8.89 (s, 1H), 9.32 (s, 1H).

### Example 28

### Synthesis of 1-[({1-[5-(difluoromethyl)(1,3,4-thiadiazol-2-yl)]-4-[4-(2-methylpropanoyl) piperazinyl]indol-6-yl}sulfonyl)amino]cyclopropanecarbonitrile

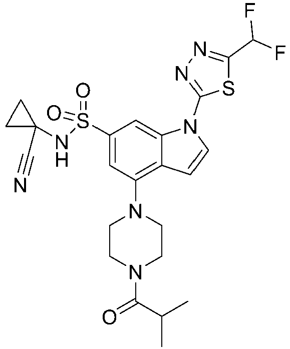

To a stirred solution of 4-chloro-N-(1-cyanocyclopropyl)-1-(5-(difluoromethyl)-1,3,4-thiadiazol-2-yl)-1H-indole-6-sulfonamide (600.00 mg, 1.396 mmol, 1.00 equiv.) and 2-methyl-1-(piperazin-1-yl)propan-1-one TFA salt (709.81 mg, 2.792 mmol, 2.00 equiv.) in 1,4-dioxane (10 mL) in a 20 mL vial, were added RuPhos (260.54 mg, 0.558 mmol, 0.40 equiv.), RuPhos Palladacycle Gen.3 (466.98 mg, 0.558 mmol, 0.40 equiv.) and Cs₂CO₃ (1591.81 mg, 4.886 mmol, 3.50 equiv.) under nitrogen atmosphere. The resulting mixture was stirred for overnight at 90 °C and then quenched with saturated NH₄Cl (aq.) (6 mL), extracted with EtOAc. The combined extracts were concentrated under vacuum and the residue was purified by Prep-TLC (CH₂Cl₂:MeOH=25:1) to afford of crude product (95 mg). The crude product was purified by Prep-HPLC (Column: XBridge Prep OBD C18 Column, 30×150 mm, 5 um; Mobile Phase A: water (10 mmol/L NH₄HCO₃ + 0.1% NH₃·H₂O), Mobile Phase B: ACN; Flow rate: 60 mL/min; Gradient:40% B to 50% B in 7 min; 254/220 nm; RT: 6.22 min) to afford the title compound (2.5 mg). LCMS (ES, *m*/*z*): [M+H]⁺ 550; ¹H-NMR (400 MHz , DMSO-d₆, *ppm*) δ 9.10 (s, 1H), 8.62 (s, 1H), 8.17 (d, 1H), 7.64 (t, 1H), 7.22 (s, 1H), 7.13 (d, 1H), 3.76 (d, 4H), 3.21 (d, 4H), 2.96 (sep, 1H), 1.39 (dd, 2H), 1.26 (dd, 2H), 1.06 (d, 6H).

### Example 29

### Synthesis of 1-[({1-[5-(difluoromethyl)(1,3,4-thiadiazol-2-yl)]-4-(2-oxa-8-azaspiro[4.5]dec-8-yl)-1H-indazol-6-yl}sulfonyl)amino]cyclopropanecarbonitrile

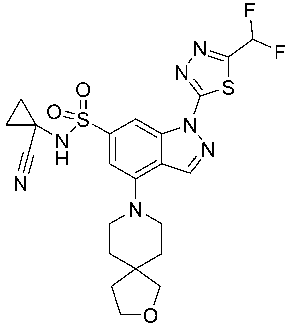

To a stirred solution of 4-chloro-N-(1-cyanocyclopropyl)-1-[5-(difluoromethyl)-1,3,4-thiadiazol-2-yl]indazole-6-sulfonamide (400.00 mg, 0.928 mmol, 1.00 equiv.) and 2-oxa-8-azaspiro[4.5]decane hydrochloride (329.91 mg, 1.857 mmol, 2.00 equiv.) in 1,4-dioxane (4 mL) in an 8 mL vial, Cs₂CO₃ (1058.76 mg, 3.250 mmol, 3.50 equiv.) RuPhos Palladacycle Gen.3 (155.30 mg, 0.186 mmol, 0.2 equiv.) and RuPhos (43.32 mg, 0.093 mmol, 0.10 equiv.) were added under nitrogen atmosphere. The solution was stirred for overnight at 90 °C and then quenched with saturated NH₄Cl (aq.). The resulting mixture was extracted with EtOAc and the combined organic layers were concentrated under reduced pressure. The residue was purified by Prep-TLC (CH₂Cl₂:MeOH=20:1) to afford the crude product. The crude product (60 mg) was purified by Prep-HPLC (Column: XBridge Phenyl OBD Column, 5 um, 19*150 mm; mobile phase A: water(10 mmol/L NH₄HCO₃), mobile phase B: ACN; flow rate: 20 mL/min; gradient: 42% B to 52% B in 8 min; 254;220 nm; rt: 8.13 min) to afford the title compound (10.7 mg). LCMS (ES, m/z): [M+H]⁺ 536; ¹H-NMR (300MHz, DMSO-d₆, *ppm*) δ 9.34 (s, 1H), 8.90 (s, 1H), 8.46 (s,1H), 7.61 (t, 1H), 7.18 (d, 1H), 3.80 (t, 2H), 3.43-3.55 (m, 6H), 1.77-1.84 (m, 6H), 1.40-1.50 (m, 2H), 1.28-1.35 (m, 2H).

### Example 30

### Synthesis of 1-[({1-[5-(difluoromethyl)(1,3,4-thiadiazol-2-yl)]-4-(9-oxa-3-azaspiro[5.5]undec-3-yl)-1H-indazol-6-yl}sulfonyl)amino]cyclopropanecarbonitrile

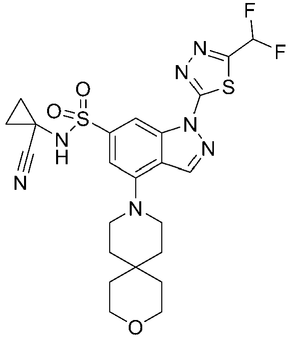

Into a 15-mL sealed tube, were placed 4-chloro-N-(1-cyanocyclopropyl)-1-[5-(difluoromethyl)-1,3,4-thiadiazol-2-yl]-1H-indazole-6-sulfonamide (500.00 mg, 1.161 mmol, 1.00 equiv.), 3-oxa-9-azaspiro[5.5]undecane (360.33 mg, 2.321 mmol, 2 equiv.), dioxane (5 mL) , Cs₂CO₃ (756.26 mg, 2.321 mmol, 2 equiv.), RuPhos Palladacycle Gen.3 (97.06 mg, 0.116 mmol, 0.1 equiv.), and RuPhos (108.31 mg, 0.232 mmol, 0.2 equiv.). The resulting solution was stirred for 1 overnight at 90 °C and then quenched with saturated NH₄Cl (aq.). The resulting mixture was extracted with EtOAc and the combined organic layers were concentrated under reduced pressure. The residue was purified by Prep-TLC (CH₂Cl₂:MeOH=40:1) to afford the crude product. The crude product (18 mg) was purified by Prep-HPLC (Column: XBridge Prep OBD C18 Column 19*250 mm, 5 um; mobile phase A: water (10 mmol/L NH₄HCO₃), mobile phase B: MeOH-HPLC; flow rate: 20 mL/min; gradient: 65% B to 79% B in 8 min; 254;220 nm; rt: 6.87 min) to afford the title compound (2.1 mg). LCMS (ES, m/z): [M+H]⁺ 550; ¹H-NMR (300MHz, DMSO-d₆, *ppm*) δ 9.31 (brs, 1H), 8.89 (s, 1H), 8.42 (s, 1H), 7.60 (t, 1H), 7.16 (s, 1H), 3.56-3.62 (m, 4H), 3.41-3.49 (m, 4H), 1.69-1.81 (m, 4H), 1.45-1.55 (m, 4H), 1.40-1.42 (m, 2H), 1.25-1.37 (m, 2H).

### Example 31

### Synthesis of [4-(1-[5-(difluoromethyl)(1,3,4-thiadiazol-2-yl)]-6-{[(cyanocyclopropyl)amino] sulfonyl}(1H-indazol-4-yl))(1,4-diazaperhydroepinyl)]-N-methylcarboxamide

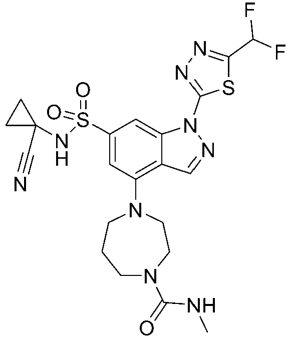

The title compound was prepared as described in Example 30 above by replacing 3-oxa-9-azaspiro[5.5]undecane with N-methyl-1,4-diazepane-1-carboxamide TFA salt. LCMS (ES, m/z): [M+H]⁺ 555; ¹H-NMR (300 MHz, DMSO-*d*₆, *ppm*) δ 9.28 (brs, 1H), 8.85 (s, 1H), 8.27 (s, 1H), 7.60 (t, 1H), 6.96 (s, 1H), 6.30-6.32 (d, 1H), 3.84 (s, 4H), 3.68 (s, 2H), 3.32 (s, 2H), 2.51-2.52 (m, 3H), 1.93 (s, 2H), 1.24-1.44 (m, 4H).

### Example 32

### Synthesis of [4-(1-[5-(difluoromethyl)(1,3,4-thiadiazol-2-yl)]-6-{[(cyanocyclopropyl)amino] sulfonyl}(1H-indazol-4-yl))piperazinyl]-N-ethyl-N-methylcarboxamide

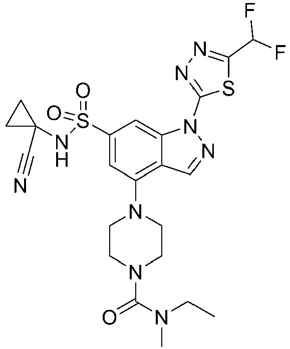

The title compound was prepared as described in Example 30 above by replacing 3-oxa-9-azaspiro[5.5]undecane with N-ethyl-N-methylpiperazine-1-carboxamide TFA salt. LCMS (ES, m/z): [M+H]⁺ 566; ¹H-NMR (300MHz, DMSO-d₆, *ppm*) δ 9.32 (s, 1H), 8.96 (s, 1H), 8.50 (s, 1H), 7.60 (t, 1H), 7.18 (d, 1H), 3.447 (d, 4H), 3.39(d, 4H), 3.18 (q, 2H), 2.80 (s, 3H), 1.43-1.46 (m, 2H), 1.23-1.29 (m, 2H), 1.10 (t, 3H).

### Example 33

### Synthesis of 1-[({4-[4-(azetidinylcarbonyl)piperazinyl]-1-[5-(difluoromethyl)(1,3,4-thiadiazol-2-yl)]-1H-indazol-6-yl}sulfonyl)amino]cyclopropanecarbonitrile

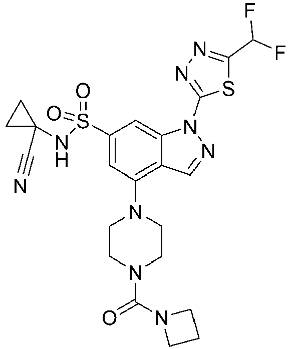

The title compound was prepared as described in Example 30 above by replacing 3-oxa-9-azaspiro[5.5]undecane with azetidin-1-yl(piperazin-1-yl)methanone TFA salt. LCMS (ES, m/z): [M+H]⁺ = 564; ¹H-NMR (300MHz, DMSO-d₆, *ppm*) δ 9.32 (brs, 1H), 8.95 (s, 1H), 8.49 (s, 1H), 7.60 (t, 1H), 7.15 (s, 1H), 3.95(t, 4H), 3.73-3.45 (m, 8H), 2.18 (p, 2H), 1.40-1.47 (m, 2H), 1.28-1.35 (m, 2H).

### Example 34

### Synthesis of1-[({1-[5-(difluoromethyl)(1,3,4-thiadiazol-2-yl)]-4-(2-oxa-6-azaspiro[3.4]oct-6-yl)-1H-indazol-6-yl}sulfonyl)amino]cyclopropanecarbonitrile

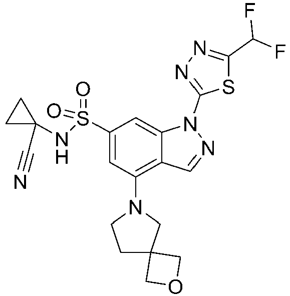

The title compound was prepared as described in Example 30 above by replacing 3-oxa-9-azaspiro[5.5]undecane with 2-oxa-6-azaspiro[3.4]octane oxalic acid salt. LCMS (ES, m/z): [M+H]⁺ 508; ¹H-NMR (300 MHz, DMSO-*d*₆, *ppm*) δ 9.27 (s, 1H), 8.90 (s, 1H), 8.20 (s, 1H), 7.60 (t, 1H), 6.77 (d, 1H), 4.64 (dd, 4H), 3.95 (s, 2H), 3.69 (t, 2H), 2.37(t, 2H), 1.42-1.51(m, 2H), 1.20-1.32 (m, 2H).

### Example 35

### Synthesis of [(2R)-4-(1-[5-(difluoromethyl)(1,3,4-thiadiazol-2-yl)]-6-{[(cyanocyclopropyl) amino]sulfonyl}(1H-indazol-4-yl))-2-methylpiperazinyl]-N,N-dimethylcarboxamide

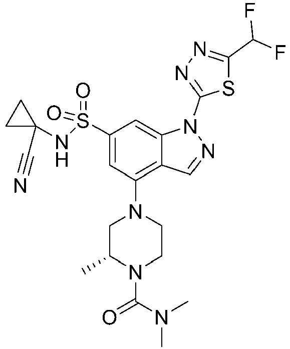

The title compound was prepared as described in Example 30 above by replacing 3-oxa-9-azaspiro[5.5]undecane with (R)-N,N,2-trimethylpiperazine-1-carboxamide TFA salt. LCMS (ES, m/z): [M+H]⁺: 566; ¹H-NMR (400 MHz, DMSO-d₆, *ppm*) δ 9.31 (s, 1H), 8.86 (s, 1H), 8.49 (s, 1H), 7.62 (t, 1H), 7.17 (s, 1H), 3.97 (dd, 1H), 3.69 (d, 1H), 3.60 (d, 1H), 3.42-3.44 (m, 2H), 3.30-3.32 (m, 1H), 3.18-3.24 (m, 1H), 2.81 (s, 6H), 1.46-1.47 (m, 2H), 1.28-1.39 (m, 5H).

### Example 36

### Synthesis of 1-[({1-[5-(difluoromethyl)(1,3,4-thiadiazol-2-yl)]-4-[4-(4-methyl(1,2,4-triazol-3-yl))piperazinyl]-1H-indazol-6-yl}sulfonyl)amino]cyclopropanecarbonitrile

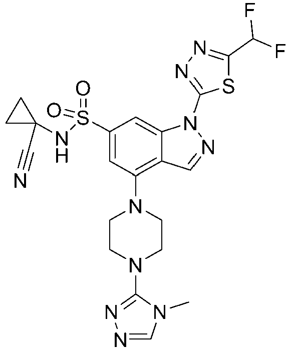

The title compound was prepared as described in Example 30 above by replacing 3-oxa-9-azaspiro[5.5]undecane with 1-(4-methyl-1,2,4-triazol-3-yl)piperazine. LCMS (ES, m/z): [M+H]⁺: 562; ¹H-NMR (400 MHz, DMSO-d₆, *ppm*) δ 9.26 (brs, 1H), 9.0 3(s, 1H), 8.55 (s, 1H), 8.27 (s, 1H), 7.62 (t, 1H), 7.25 (s, 1H), 3.56-3.62 (m, 4H), 3.55 (s, 3H), 3.37-3.38 (m, 4H), 1.43-1.46 (m, 2H), 1.30-1.33 (m, 2H).

### Example 37

### Synthesis of 1-[({4-[(1S,5R)-8-(2-methylpropanoyl)-3,8-diazabicyclo[3.2.1]oct-3-yl]-1-[5-(difluoromethyl)(1,3,4-thiadiazol-2-yl)]-1H-indazol-6-yl}sulfonyl)amino]cyclopropane carbonitrile

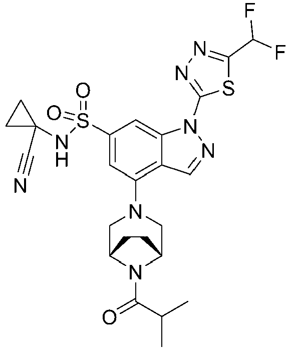

The title compound was prepared as described in Example 30 above by replacing 3-oxa-9-azaspiro[5.5]undecane with 1-((1R,5S)-3,8-diazabicyclo[3.2.1]octan-8-yl)-2-methylpropan-1-one TFA salt. LCMS (ES, m/z): [M+H]⁺ 577; ¹H-NMR (400 MHz, DMSO-d₆, *ppm*) δ 8.91 (s, 1H), 8.42 (s, 1H), 7.60 (t, 1H), 7.12 (s, 1H), 4.71 (s, 1H), 4.58 (s, 1H), 3.78 (t, 2H), 3.23 (d, 1H), 3.15 (d, 1H), 2.86 (p, 1H), 1.84-2.00 (m, 4H), 1.22-1.32 (m, 2H), 1.12-1.21 (m, 2H), 1.06 (dd, 6H).

### Example 38

### Synthesis of1-[({1-[5-(difluoromethyl)(1,3,4-thiadiazol-2-yl)]-4-[4-(2-methylpropanoyl) piperazinyl]-1H-indazol-6-yl}sulfonyl)amino]cyclopropanecarbonitrile

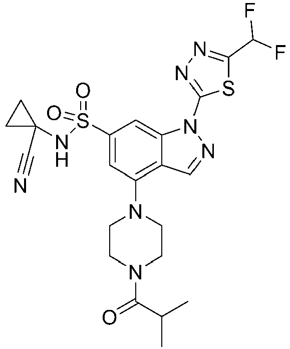

The title compound was prepared as described in Example 30 above by replacing 3-oxa-9-azaspiro[5.5]undecane with 2-methyl-1-(piperazin-1-yl)propan-1-one. LCMS (ES, m/z): [M+H]+ 551; ¹H-NMR (300 MHz, DMSO-d₆, *ppm*) δ 9.34 (s, 1H), 8.98 (s, 1H), 8.50 (s, 1H), 7.61 (t, 1H), 7.17 (s, 1H), 3.77 (d, 4H), 3.48 (brs, 4H), 2.95 (p, 1H), 1.39-1.47 (m, 2H), 1.29-1.34 (m, 2H), 1.06 (d, 6H).

### Example 39

### Synthesis of 1-[({1-[5-(difluoromethyl)(1,3,4-thiadiazol-2-yl)]-4-[4-(2,2-dimethylpropanoyl) piperazinyl]-1H-indazol-6-yl}sulfonyl)amino]cyclopropanecarbonitrile

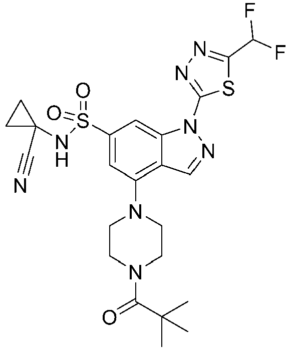

Into a 25-mL round-bottom flask purged and maintained with an inert atmosphere of nitrogen, were placed 4-chloro-N-(1-cyanocyclopropyl)-1-[5-(difluoromethyl)-1,3,4-thiadiazol-2-yl]indazole-6-sulfonamide (400.00 mg, 0.928 mmol, 1.00 equiv.), 2,2-dimethyl-1-(piperazin-1-yl)propan-1-one hydrochloride (575.75 mg, 2.785 mmol, 3 equiv.), RuPhos (173.30 mg, 0.371 mmol, 0.4 equiv.), Cs₂CO₃ (1210.02 mg, 3.714 mmol, 4 equiv.), dioxane (8.00 mL), and RuPhos Palladacycle Gen.3 (310.61 mg, 0.371 mmol, 0.4 equiv.). The resulting solution was stirred for 12 h at 90 °C in an oil bath and then quenched with water. The resulting solution was extracted with ethyl acetate and the organics were washed with brine, dried over anhydrous sodium sulfate and concentrated. The residue was purified by flash chromatography [silica gel, ethyl acetate / petroleum ether (1/1)] to provide the title compound (14 mg). LCMS (ES, m/z): [M+H]⁺ 565; ¹H-NMR (400 MHz, DMSO-d₆, *ppm*) δ 9.28 (brs, 1H), 8.98 (s, 1H), 8.51 (s, 1H), 7.62 (m, 1H), 7.18 (s, 1H), 3.84 (s, 4H), 3.47 (s, 4H), 1.46-1.40 (m, 2H), 1.33-1.29 (m, 2H), 1.26 (s, 9H).

### Example 40

### Synthesis of [1-(1-[5-(difluoromethyl)(1,3,4-thiadiazol-2-yl)]-6-{[(cyanocyclopropyl)amino] sulfonyl}(1H-indazol-4-yl))(4-piperidyl)]-N-methylcarboxamide

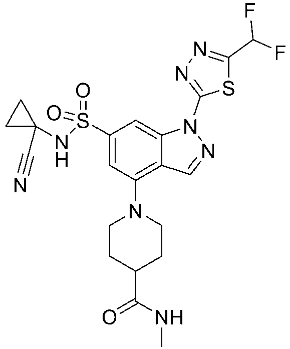

Into a 15-mL sealed tube purged and maintained with an inert atmosphere of nitrogen, were placed 4-chloro-N-(1-cyanocyclopropyl)-1-[5-(difluoromethyl)-1,3,4-thiadiazol-2-yl]indazole-6-sulfonamide (300.00 mg, 0.696 mmol, 1.00 equiv.), N-methylpiperidine-4-carboxamide (197.81 mg, 1.393 mmol, 2.00 equiv.), Cs₂CO₃ (680.63 mg, 2.089 mmol, 3 equiv.), dioxane (3.00 mL), RuPhos (32.49 mg, 0.070 mmol, 0.1 equiv.), and RuPhos Pd G3 (232.96 mg, 0.279 mmol, 0.40 equiv.). The resulting solution was stirred for 1 overnight at 90 °C. The reaction was quenched with water at room temperature and extracted with EtOAc. The organics were concentrated under vacuum and the residue was purified by Prep-TLC (CH₂Cl₂: MeOH=20:1) to afford crude product. The crude product (150 mg) was purified by Prep-HPLC (Column: Ultimate XB-C4 21.2*250 mm 5 um; Mobile Phase A: water (0.1%FA), Mobile Phase B: ACN; Flow rate: 60 mL/min; Gradient:38% B to 46% B in 7 min; 254/220 nm; RT 6.33 min) to afford the title compound (41.7mg, 11.16%). LCMS (ES, m/z): [M+H]⁺ 537; ¹H-NMR (300 MHz, DMSO-d₆, *ppm*) δ 9.33 (brs, 1H), 8.89 (s, 1H), 8.45 (s, 1H), 7.81 (d, 1H), 7.60 (t, 1H), 7.16 (s, 1H), 3.97 (d, 2H), 3.03-3.09 (m, 2H) , 2.60 (d, 3H) , 2.36-2.41(m, 1H), 1.78-1.82 (m, 4H), 1.39-1.47 (m, 2H), 1.26-1.34 (m, 2H).

### Example 41

### Synthesis of 1-[({1-[5-(difluoromethyl)(1,3,4-thiadiazol-2-yl)]-4-(1-oxa-7-azaspiro[3.5]non-7-yl)-1H-indazol-6-yl}sulfonyl)amino]cyclopropanecarbonitrile

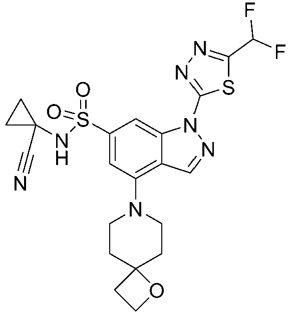

To a stirred solution/mixture of 4-chloro-N-(1-cyanocyclopropyl)-1-[5-(difluoromethyl)-1,3,4-thiadiazol-2-yl]indazole-6-sulfonamide (400 mg, 0.928 mmol, 1.00 equiv.) and bis(1-oxa-7-azaspiro[3.5]nonane) and oxalic acid (319.76 mg, 0.928 mmol, 1.00 equiv.) in 1,4-dioxane (4 mL) in an 8 mL vial, Cs₂CO₃ (1058.76 mg, 3.250 mmol, 3.50 equiv.), RuPhos Palladacycle Gen.3 (77.65 mg, 0.093 mmol, 0.10 equiv.) and RuPhos (86.65 mg, 0.186 mmol, 0.20 equiv.) were added under nitrogen atmosphere. The solution was stirred at 90 °C for overnight under nitrogen atmosphere. The reaction was quenched with saturated NH₄Cl (aq.) and the resulting mixture was extracted with EtOAc. The combined organic layers were concentrated under reduced pressure and the residue was purified by Prep-TLC (CH₂Cl₂:EtOAc=30:1) to afford the crude product. The crude product (120 mg) was re-crystallized from PE/EtOAc (4:1 5mL) to afford the title compound (61.6 mg). LCMS (ES, m/z): [M+H]⁺ 522; ¹H-NMR (400MHz, DMSO-d₆, *ppm*) δ 9.35 (s, 1H), 8.92 (s, 1H), 8.46 (s, 1H), 7.61 (t, 1H), 7.18(s, 1H), 4.46 (t, 2H), 3.46-3.50 (m, 2H), 3.37-3.42 (m, 2H), 2.43-2.45 (m, 2H), 2.00-2.08 (m, 4H), 1.44-1.49 (m, 2H), 1.27-1.31(m, 2H).

### Example 42

### Synthesis of 1-[({1-[5-(difluoromethyl)(1,3,4-thiadiazol-2-yl)]-4-(1,4-oxazaperhydroepin-4-yl)-1H-indazol-6-yl}sulfonyl)amino]cyclopropanecarbonitrile

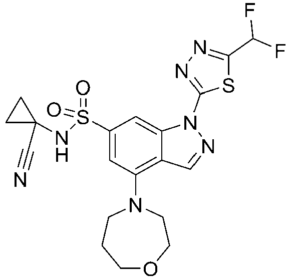

Into a 20-mL vial, were placed 4-chloro-N-(1-cyanocyclopropyl)-1-[5-(difluoromethyl)-1,3,4-thiadiazol-2-yl]indazole-6-sulfonamide (300.00 mg, 0.696 mmol, 1.00 equiv.), dioxane (5.00 mL), 1,4-oxazepane hydrochloride (191.64 mg, 1.393 mmol, 2.00 equiv.), Cs₂CO₃ (680.63 mg, 2.089 mmol, 3.00 equiv.), RuPhos (129.97 mg, 0.279 mmol, 0.40 equiv.), and RuPhos Palladacycle Gen.3 (232.96 mg, 0.279 mmol, 0.40 equiv.). The resulting solution was stirred for 1 overnight at 90 °C under nitrogen atmosphere. The resulting mixture was washed with H2O and extracted with ethyl acetate. The organic layer was concentrated under vacuum and the residue was purified by Prep TLC (CH₂Cl₂:MeOH=30:1) to afford the title compound (40.7 mg). LCMS (ES, m/z): [M+H]⁺ 496; ¹H-NMR (300MHz, DMSO-d₆, *ppm*) δ 9.29 (s, 1H), 8.83 (s, 1H), 8.28 (s, 1H), 7.60 (t, 1H), 6.97-6.96 (d, 1H), 3.92-3.89 (m, 6H), 3.69 (t, 2H), 2.04-2.01 (m, 2H), 1.46-1.41 (m, 2H), 1.38-1.31 (m, 2H).

### Example 43

### Synthesis of [4-(1-[5-(difluoromethyl)(1,3,4-thiadiazol-2-yl)]-6- {[(cyanocyclopropyl)amino] sulfonyl}(1H-indazol-4-yl))piperazinyl]-N,N-dimethylcarboxamide

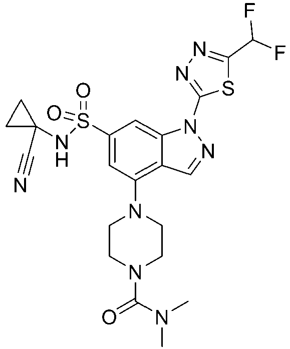

Into a 15-mL sealed tube purged and maintained with an inert atmosphere of nitrogen, were placed 4-chloro-N-(1-cyanocyclopropyl)-1-[5-(difluoromethyl)-1,3,4-thiadiazol-2-yl]indazole-6-sulfonamide (300.00 mg, 0.696 mmol, 1.00 equiv.), Cs₂CO₃ (680.63 mg, 2.089 mmol, 3 equiv.), dioxane (3.00 mL), N,N-dimethylpiperazine-1-carboxamide (131.37 mg, 0.836 mmol, 1.2 equiv.), RuPhos (32.49 mg, 0.070 mmol, 0.1 equiv.), and RuPhos Palladacycle Gen.3 (58.24 mg, 0.070 mmol, 0.1 equiv.). The resulting solution was stirred for 1 overnight at 90 °C. The reaction was quenched with water at room temperature and the aqueous layer was extracted with EtOAc. The resulting mixture was concentrated under vacuum and the residue was purified by Prep-TLC (CH₂Cl₂:MeOH=20:1) to afford crude product. The crude product (150 mg) was purified by Prep-HPLC (Column: XBridge Prep OBD C18 Column, 30×150 mm 5 um; Mobile Phase A: water (0.05% NH₃.H₂O), Mobile Phase B: ACN; Flow rate:60 mL/min; Gradient:20% B to 35% B in 7 min; 254;220 nm; RT 6.77 min) to afford the title compound (57.4 mg). LCMS (ES, m/z): [M+H]⁺ 552; ¹H-NMR (300 MHz, DMSO-d₆, ppm) δ 9.33 (brs, 1H), 8.94 (s, 1H), 8.48 (m, 1H), 7.59 (t, 1H), 7.16 (s, 1H), 3.31-3.43 (m, 8H), 2.79 (s, 6H), 1.39-1.44 (t, 2H), 1.26-1.36 (m, 2H).

### Example 44

### Synthesis of 1-[({1-[5-(difluoromethyl)(1,3,4-thiadiazol-2-yl)]-4-(1-oxa-8-azaspiro[4.5]dec-8-yl)-1H-indazol-6-yl}sulfonyl)amino]cyclopropanecarbonitrile

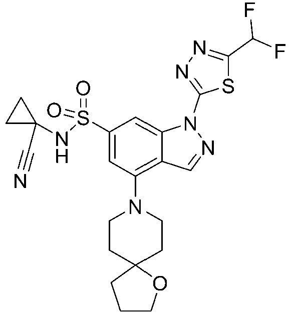

The title compound was prepared as described in Example 43 above by replacing N,N-dimethyl-piperazine-1-carboxamide with 1-oxa-8-azaspiro[4.5]decane. LCMS (ES, m/z): [M+H]⁺ 536; ¹H-NMR (300 MHz, DMSO-d₆, *ppm*) δ 9.33 (s, 1H), 8.90 (s, 1H), 8.45 (s, 1H), 7.61 (t, 1H), 7.17 (d, 1H), 3.78 (t, 2H), 3.41-3.56 (m, 4H), 1.68-1.97 (m, 8H), 1.40-1.50 (m, 2H), 1.20-1.36 (m, 2H).

### Example 45

### Synthesis of 1-[({1-[5-(difluoromethyl)(1,3,4-thiadiazol-2-yl)]-4-(6-oxa-2-azaspiro[3.3]hept-2-yl)-1H-indazol-6-yl}sulfonyl)amino]cyclopropanecarbonitrile

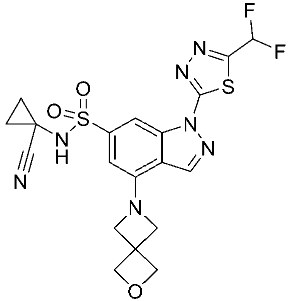

The title compound was prepared as described in Example 43 above by replacing N,N-dimethylpiperazine-1-carboxamide with bis(2-oxa-6-azaspiro[3.3]heptane) oxalic acid salt. LCMS (ES, *m*/*z*): [M+H]⁺ 494; ¹H-NMR (300MHz, DMSO-d₆, *ppm*) δ 9.30 (brs, 1H), 8.71 (s, 1H), 8.19 (s, 1H), 7.59 (t, 1H), 6.58 (s, 1H), 4.88 (s, 4H), 4.47 (s, 4H), 1.40-1.46 (m, 2H), 1.25-1.33 (m, 2H).

### Example 46

### Synthesis of 1-[({1-[5-(difluoromethyl)(1,3,4-thiadiazol-2-yl)]-4-(2-methyl-2,8-diazaspiro[4.5]d ec-8-yl)-1H-indazol-6-yl}sulfonyl)amino]cyclopropanecarbonitrile

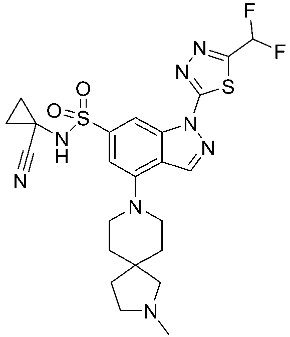

The title compound was prepared as described in Example 43 above by replacing N,N-dimethylpiperazine-1-carboxamide with 2-methyl-2,8-diazaspiro[4.5]decane hemioxalate salt. LCMS (ES, m/z): [M+H]⁺ 549; ¹H-NMR (300MHz, DMSO-d₆, *ppm*) δ 9.20-9.50 (brs, 1H), 8.89 (s, 1H), 8.45 (s, 1H), 7.61 (t, 1H), 7.17 (s, 1H), 3.25-3.47 (m, 6H), 2.41 (s, 2H), 2.26 (s, 3H), 1.65-1.83 (m, 6H), 1.29-1.46 (m, 4H).

### Example 47

### Synthesis of 1-[({1-[5-(difluoromethyl)(1,3,4-thiadiazol-2-yl)]-4-[4-(hydroxymethyl)piperidyl]-1H-indazol-6-yl}sulfonyl)amino]cyclopropanecarbonitrile

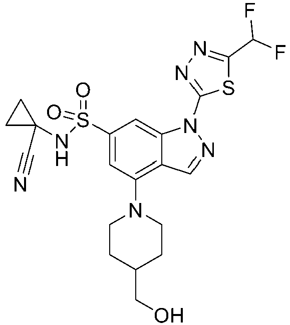

The title compound was prepared as described in Example 43 above by replacing N,N-dimethylpiperazine-1-carboxamide with piperidin-4-ylmethanol. LCMS-PH (ES, m/z): [M+H]⁺ 510; ¹H-NMR (400 MHz, DMSO-*d*₆, *ppm*) δ 9.32 (s, 1H), 8.87 (s, 1H), 8.43 (s, 1H), 7.60 (t, 1H), 7.16 (s, 1H), 4.56 (t, 1H), 3.89 (d, 2H), 3.33-3.35 (m, 2H), 3.04 (t, 2H), 1.85 (d, 2H), 1.63-1.66 (m, 1H), 1.40-1.45 (m, 4H), 1.32-1.37 (t, 2H).

### Example 48

### Synthesis of 1-[({4-((1S,5R)-7-oxa-3-azabicyclo[3.3.0]oct-3-yl)-1-[5-(difluoromethyl)(1,3,4-thiadiazol-2-yl)]-1H-indazol-6-yl}sulfonyl)amino]cyclopropanecarbonitrile

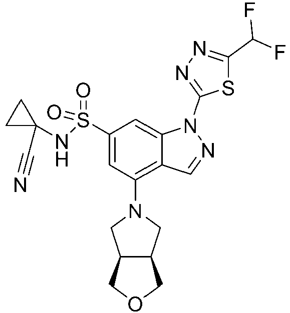

The title was prepared as described in Example 43 above by replacing N,N-dimethylpiperazine-1-carboxamide with *cis*-hexahydro-1H-furo[3,4-c]pyrrole hydrochloride. LCMS (ES, m/z): [M+H]⁺ 508; ¹H-NMR (400 MHz, DMSO-*d*₆, *ppm*) δ 9.27 (s, 1H), 8.90 (s, 1H), 8.23 (s, 1H), 7.60 (t, 1H), 6.79 (s, 1H), 3.85-3.93 (m, 4H), 3.73 (dd, 2H), 3.61 (dd, 2H), 3.17 (s, 2H), 1.42-1.45 (m, 2H), 1.28-1.30 (t, 2H).

### Example 49

### Synthesis of 1-[({1-[5-(difluoromethyl)(1,3,4-thiadiazol-2-yl)]-4-(4-formylpiperazinyl)-1H-indazol-6-yl}sulfonyl)amino]cyclopropanecarbonitrile

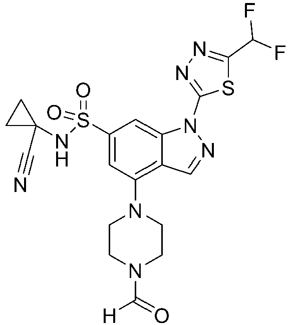

The title compound was prepared as described in Example 43 above by replacing N,N-dimethylpiperazine-1-carboxamide with piperazine-1-carbaldehyde. LCMS (ES, m/z): [M+H]⁺: 509; ¹H-NMR (400 MHz, DMSO-*d*₆, *ppm*) δ 9.27-9.42 (brs, 1H), 8.98 (brs, 1H), 8.52 (brs, 1H), 8.13 (brs, 1H), 7.49-7.74 (m, 1H), 7.19 (brs, 1H), 3.65 (s, 4H), 3.39-3.58 (m, 4H), 1.22-1.48 (m, 4H).

### Example 50

### Synthesis of 1-[({4-(4-acetylpiperazinyl)-1-[5-(difluoromethyl)(1,3,4-thiadiazol-2-yl)]-1H-indazol-6-yl}sulfonyl)amino]cyclopropanecarbonitrile

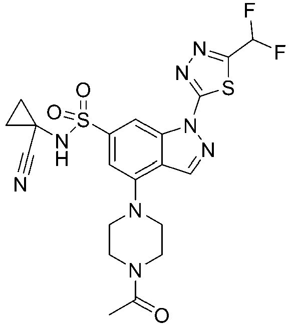

The title compound was prepared as described in Example 43 above by replacing N,N-dimethylpiperazine-1-carboxamide with 1-(piperazin-1-yl)ethanone. LCMS (ES, m/z): [M+H]⁺ 509; ¹H-NMR (400 MHz, DMSO-d6, ppm) δ 9.43 (s, 1H), 8.97 (s, 1H), 8.50 (s, 1H), 7.60 (t, 1H), 7.16 (s, 1H), 3.73 (s, 4H), 3.47 (d, 4H), 2.08 (s, 3H), 1.44 (s, 2H), 1.32 (s, 2H).

### Example 51

### Synthesis of [1-(1-[5-(difluoromethyl)(1,3,4-thiadiazol-2-yl)]-6- {[(cyanocyclopropyl)amino] sulfonyl} (1H-indazol-4-yl))(4-piperidyl)]-N,N-dimethylcarboxamide

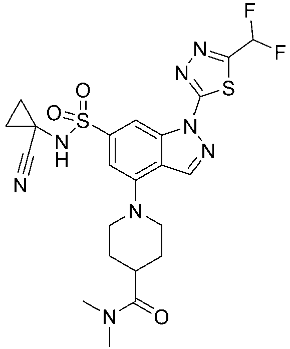

The title compound was prepared as described in Example 43 above by replacing N,N-dimethylpiperazine-1-carboxamide with N,N-dimethylpiperidine-4-carboxamide. LCMS (ES, m/z): [M+H]⁺ 551; ¹H-NMR (400 MHz, DMSO-d₆, *ppm)* δ 9.32 (s, 1H), 8.91 (s, 1H), 8.46 (s, 1H), 7.60 (t, 1H), 7.17 (s, 1H), 3.89 (d, 2H), 3.13-3.17 (m, 2H), 3.08 (s, 3H), 2.90-2.97 (m, 1H), 2.85 (s, 3H), 1.81-1.90 (m, 4H), 1.43-1.46 (m, 2H), 1.25-1.30 (m, 2H).

### Example 52

### Synthesis of N-(1-cyanocyclopropyl)-1-(5-(difluoromethyl)-1,3,4-thiadiazol-2-yl)-4-(1,1-dioxidothiomorpholino)-1H-indazole-6-sulfonamide

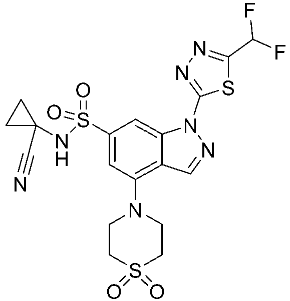

The title compound was prepared as described in Example 43 above by replacing N,N-dimethylpiperazine-1-carboxamide with thiomorpholine 1,1-dioxide. LCMS (ES, m/z): [M+H]⁺ 530; ¹H-NMR (300 MHz, DMSO-*d*₆, *ppm*) δ 9.24 (brs, 1H), 8.95 (s, 1H), 8.56 (s, 1H), 7.61 (t, 1H), 7.26 (s, 1H), 3.92 (s, 4H), 3.40 (s, 4H), 1.40-1.44 (t, 2H), 1.27-1.34 (m, 2H).

### Example 53

### Synthesis of[1-(1-[5-(difluoromethyl)(1,3,4-thiadiazol-2-yl)]-6-{[(cyanocyclopropyl)amino] sulfonyl}(1H-indazol-4-yl))pyrrolidin-3-yl]-N-methylcarboxamide

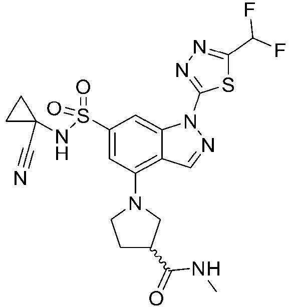

The title compound was prepared as described in Example 43 above by replacing N,N-dimethylpiperazine-1-carboxamide with N-methylpyrrolidine-3-carboxamide. LCMS (ES, m/z): [M+H]⁺ 523; ¹H-NMR (400MHz, DMSO-d₆, *ppm*) δ 9.26 (s, 1H), 8.87 (s, 1H), 8.20 (s, 1H), 8.07 (dd, 1H), 7.60 (t, 1H), 6.76 (s, 1H), 3.70-3.82 (m, 4H), 3.19 (p, 1H), 2.64-2.65 (d, 3H), 2.15-2.38 (m, 2H), 1.42-1.45 (m, 2H), 1.29-1.30 (m, 2H).

### Example 54

### Synthesis of [1-(1-[5-(difluoromethyl)(1,3,4-thiadiazol-2-yl)]-6-{[(cyanocyclopropyl)amino] sulfonyl}(1H-indazol-4-yl))pyrrolidin-3-yl]-N,N-dimethylcarboxamide

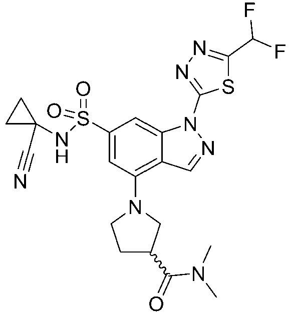

The title compound was prepared as described in Example 43 above by replacing N,N-dimethylpiperazine-1-carboxamide with N,N-dimethylpyrrolidine-3-carboxamide. LCMS (ES, m/z): [M+H]⁺ 537; ¹H-NMR (300 MHz, DMSO-*d*₆, *ppm*) δ 9.25 (s, 1H), 8.87 (s, 1H), 8.19 (s, 1H), 7.59 (t, 1H), 6.75 (d, 1H), 3.62-3.89 (m, 5H), 3.12 (s, 3H), 2.88 (s, 3H), 2.27-2.37 (m, 1H), 2.12-2.24 (m, 1H), 1.39-1.48 (m, 2H), 1.22-1.38 (m, 2H).

### Example 55

### Synthesis of [1-(1-[5-(difluoromethyl)(1,3,4-thiadiazol-2-yl)]-6-{[(cyanocyclopropyl)amino] sulfonyl}(1H-indazol-4-yl))azetidin-3-yl]-N,N-dimethylcarboxamide

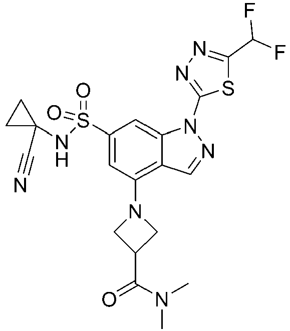

The title compound was prepared as described in Example 43 above by replacing N,N-dimethylpiperazine-1-carboxamide with N,N-dimethylazetidine-3-carboxamide hydrochloride salt. LCMS (ES, m/z): [M+H]⁺ 523; ¹H-NMR (400 MHz, DMSO-*d*₆, *ppm*) δ 9.23 (brs, 1H), 8.76 (s, 1H), 8.21 (s, 1H), 7.59 (t, 1H), 6.62 (d, 1H), 4.49 (t, 2H), 4.36 (t, 2H), 3.95-4.10 (m, 1H), 2.95 (s, 3H), 2.89 (s, 3H), 1.41-1.43 (m, 2H), 1.27-1.29 (m, 2H).

### Example 56

### Synthesis of 1-[({1-[5-(difluoromethyl)(1,3,4-thiadiazol-2-yl)]-4-(cis-8-oxa-3-azabicyclo-[4.3.0]non-3-yl)-1H-indazol-6-yl}sulfonyl)amino]cyclopropanecarbonitrile

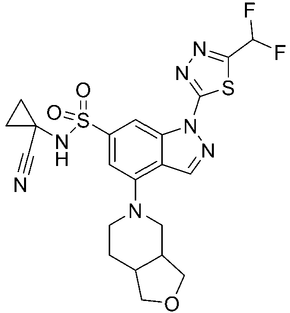

The title compound was prepared as described in Example 43 above by replacing N,N-dimethyl piperazine-1-carboxamide with octahydrofuro[3,4-c]pyridine hydrochloride salt. LCMS (ES, m/z): [M+H]⁺ 522; ¹H-NMR (400 MHz, DMSO-*d*₆, *ppm*) δ 9.28 (s, 1H), 8.86 (s, 1H), 8.35 (s, 1H), 7.60 (t, 1H), 7.07 (d, 1H), 3.84 (dd, 2H), 3.69 (dd, 1H), 3.58-3.63 (m, 3H), 3.42-3.51 (m, 2H), 2.64-2.67 (m, 1H), 2.50-2.51 (m, 1H), 2.00-2.04 (m, 1H), 1.77-1.79 (m, 1H), 1.42-1.45 (m, 2H), 1.29-1.32(m, 2H).

### Example 57

### Synthesis of 1-[({1-[5-(difluoromethyl)(1,3,4-thiadiazol-2-yl)]-4-[4-(pyrrolidinylcarbonyl)-piperazinyl]-1H-indazol-6-yl}sulfonyl)amino]cyclopropanecarbonitrile

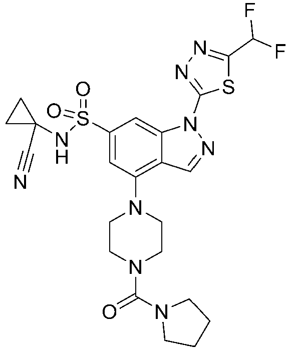

The title compound was prepared as described in Example 43 above by replacing N,N-dimethylpiperazine-1-carboxamide with 1-(pyrrolidine-1-carbonyl)piperazine hydrochloride salt. LCMS (ES, m/z): [M+H] ⁺ 578; ¹H-NMR (400 MHz, DMSO-d₆, ppm) δ 9.33 (s, 1H),8.96-8.97 (d, 1H), 8.50 (s, 1H),7.61 (t, 1H), 7.18 (d, 1H), 3.47 (s, 8H), 3.34-3.35 (m,4H), 1.79 (t, 4H), 1.44-1.46 (t, 2H), 1.22-1.30 (m, 2H).

### Example 58

### Synthesis of methyl 4-(1-[5-(difluoromethyl)(1,3,4-thiadiazol-2-yl)]-6-{[(cyanocyclopropyl) amino]sulfonyl}-1H-indazol-4-yl)piperazinecarboxylate

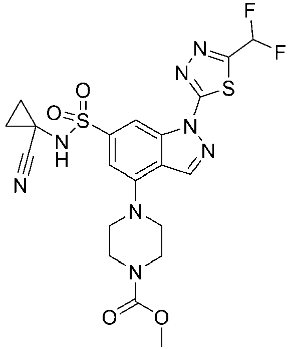

The title compound was prepared as described in Example 43 above by replacing N,N-dimethylpiperazine-1-carboxamide with methyl piperazine-1-carboxylate. LCMS (ES, m/z): [M+H]⁺ 539; ¹H-NMR (300 MHz, DMSO-*d*₆, *ppm*) δ 9.33 (s, 1H), 8.95 (s, 1H), 8.50 (s, 1H), 7.60 (t, 1H), 7.17 (s, 1H), 3.66 (s, 7H), 3.47 (d, 4H), 1.44 (dd, 2H), 1.32 (dd, 2H).

### Example 59

### Synthesis of 1-[({4-((1S)-7-oxo-3,6-diazabicyclo[4.3.0]non-3-yl)-1-[5-(difluoromethyl)(1,3,4-thiadiazol-2-yl)]-1H-indazol-6-yl}sulfonyl)amino]cyclopropanecarbonitrile

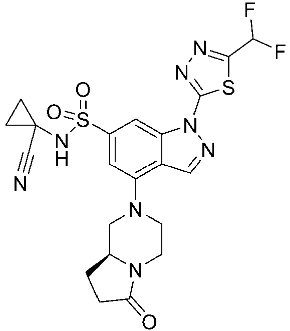

The title compound was prepared as described in Example 43 above by replacing N,N-dimethylpiperazine-1-carboxamide with (S)-hexahydropyrrolo[1,2-a]pyrazin-6(2H)-one hydrochloride salt. LCMS (ES, m/z): [M+H]⁺ 535; ¹H-NMR (400 MHz, DMSO-d₆, *ppm*) δ 9.35 (s, 1H), 8.99 (s, 1H), 8.52 (s, 1H), 7.61 (t, 1H), 7.22(d, 1H), 3.97 (d, 2H), 3.85-3.89 (m, 2H), 3.14 (dt, 1H), 2.94 (dt, 1H), 2.80 (t, 1H), 2.29-3.33 (m, 2H), 2.20-2.26 (m, 1H), 1.65-1.70 (m, 1H), 1.44 (dd, 2H), 1.32 (dd, 2H).

### Example 60

### Synthesis of 1-[({4-((1R)-7-oxo-3,6-diazabicyclo[4.3.0]non-3-yl)-1-[5-(difluoromethyl)(1,3,4-thiadiazol-2-yl)]-1H-indazol-6-yl}sulfonyl)amino]cyclopropanecarbonitrile

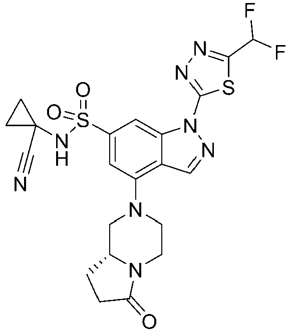

The title compound was prepared as described in Example 43 above by replacing N,N-dimethylpiperazine-1-carboxamide with (R)-hexahydropyrrolo[1,2-a]pyrazin-6(2H)-one hydrochloride salt. LCMS (ES, m/z): [M+H]⁺ 535; ¹H-NMR (400 MHz, DMSO-*d*₆, *ppm*) δ 9.35 (s, 1H), 8.99 (s, 1H), 8.53 (s, 1H), 7.61 (t, 1H), 7.22(d, 1H), 3.97(d, 2H), 3.85-3.89 (m, 2H), 3.13 (dt, 1H), 2.94 (dt, 1H), 2.80 (t, 1H), 2.29-2.35 (m, 2H), 2.20-2.26 (m, 1H), 1.67-1.70 (m, 1H), 1.45 (dd, 2H), 1.32 (dd, 2H).

### Example 61

### Synthesis of 1-[({4-((1R)-7-oxo-8-oxa-3,6-diazabicyclo[4.3.0]non-3-yl)-1-[5-(difluoromethyl) (1,3,4-thiadiazol-2-yl)]-1H-indazol-6-yl}sulfonyl)amino]cyclopropanecarbonitrile

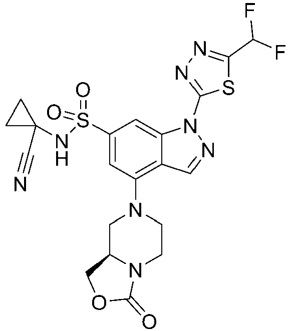

The title compound was prepared as described in Example 43 above by replacing N,N-dimethylpiperazine-1-carboxamide with (R)-hexahydro-3H-oxazolo[3,4-a]pyrazin-3-one hydrochloride salt. LCMS (ES, m/z): [M+H]⁺ 537; ¹H-NMR (400MHz, DMSO-d₆, *ppm*) δ 9.37 (s, 1H), 8.99 (s, 1H), 8.55 (s, 1H), 7.62 (t, 1H), 7.23 (s, 1H), 4.47 (t, 1H), 4.16-4.23 (m, 1H), 4.08 (dd, 1H), 4.01 (dd, 1H), 3.79 (t, 2H), 3.35-3.40 (m, 1H), 2.95-3.07 (m, 2H), 1.46 (dd, 2H), 1.32 (dd, 2H).

### Example 62

### Synthesis of 1-[({4-((1S)-7-oxo-8-oxa-3,6-diazabicyclo[4.3.0]non-3-yl)-1-[5-(difluoromethyl) (1,3,4-thiadiazol-2-yl)]-1H-indazol-6-yl}sulfonyl)amino]cyclopropanecarbonitrile

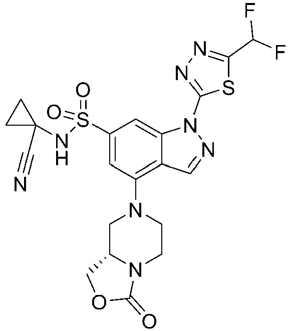

The title compound was prepared as described in Example 43 above by replacing N,N-dimethylpiperazine-1-carboxamide with (S)-hexahydro-3H- oxazolo[3,4-a]pyrazin-3-one hydrochloride salt. LCMS (ES, m/z): [M+H]⁺ 537; ¹H-NMR (400MHz, DMSO-d₆, *ppm*) δ 9.36 (s, 1H), 8.98 (s, 1H), 8.55 (s, 1H), 7.61 (t, 1H), 7.22 (s, 1H), 4.47 (t, 1H), 4.16-4.23 (m, 1H), 4.08 (dd, 1H), 4.01 (dd, 1H), 3.79 (t, 2H), 3.35-3.40 (m, 1H), 2.95-3.07 (m, 2H), 1.46 (dd, 2H), 1.32 (dd, 2H).

### Example 63

### Synthesis of1-[({1-[5-(difluoromethyl)(1,3,4-thiadiazol-2-yl)]-4-(4-methyl(1,4-diazaperhydro-epinyl))-1H-indazol-6-yl}sulfonyl)amino]cyclopropanecarbonitrile

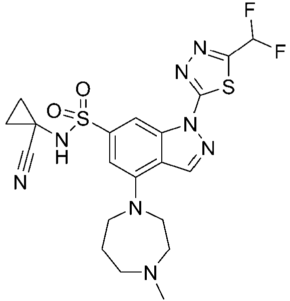

The title compound was prepared as described in Example 43 above by replacing N,N-dimethylpiperazine-1-carboxamide with N-methylhomopiperazine. LCMS (ES, m/z): [M+H]⁺: 509; ¹H-NMR (400 MHz, DMSO-*d*₆, *ppm*) δ 9.28 (s, 1H), 8.78 (s, 1H), 8.25 (s, 1H), 7.59 (t, 1H), 6.93 (s, 1H), 3.82 (t, 2H), 3.77 (t, 2H), 2.79-2.81 (m, 2H), 2.54-2.56 (m, 2H), 2.33 (s, 3H), 2.03-2.04 (m, 2H), 1.443 (dd, 2H), 1.26 (dd, 2H).

### Example 64

### Synthesis of 1-[({1-[5-(difluoromethyl)(1,3,4-thiadiazol-2-yl)]-4-[4-(difluoromethyl)piperidyl]-1H-indazol-6-yl}sulfonyl)amino]cyclopropanecarbonitrile

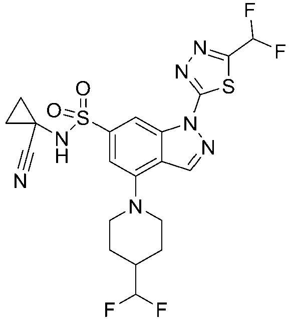

The title compound was prepared as described in Example 43 above by replacing N,N-dimethylpiperazine-1-carboxamide with 4-(difluoromethyl)piperidine hydrochloride salt. LCMS (ES, m/z): [M+H]⁺: 535; ¹H-NMR (300 MHz, DMSO-*d*₆, *ppm*) δ 9.33 (s, 1H), 8.91 (s, 1H), 8.47 (s, 1H), 7.60 (t, 1H), 7.18 (d, 1H), 6.02 (td, 1H), 3.93(d, 2H), 3.06 (t, 2H), 2.13-2.28 (m, 1H), 1.86-1.94 (m, 2H), 1.67 (qd, 2H), 1.43 (dd, 2H), 1.32 (dd, 2H).

### Example 65

### Synthesis of 1-[({1-[5-(difluoromethyl)(1,3,4-thiadiazol-2-yl)]-4-(2-oxa-6-azaspiro[3.5]non-6-yl)-1H-indazol-6-yl}sulfonyl)amino]cyclopropanecarbonitrile

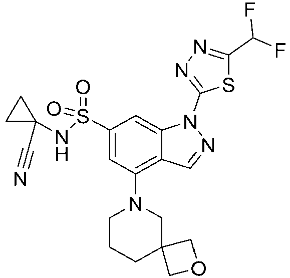

The title compound was prepared as described in Example 43 above by replacing N,N-dimethylpiperazine-1-carboxamide with 2-oxa-6-azaspiro[3.5]nonane oxalic acid salt. LCMS (ES, m/z): [M+H]⁺ 522; ¹H-NMR (400 MHz, DMSO-*d*₆, *ppm*) δ 9.36 (s, 1H), 8.87 (s, 1H), 8.51 (s, 1H), 7.62 (t, 1H), 7.24 (s, 1H), 4.38 (dd, 4H), 3.66 (s, 2H), 3.29-3.38 (m, 2H), 1.91 (t, 2H), 1.75 (brs, 2H), 1.47 (dd, 2H), 1.34 (dd, 2H).

### Example 66

### Synthesis of 1-[({1-[5-(difluoromethyl)(1,3,4-thiadiazol-2-yl)]-4-[4-(cyclopentylcarbonyl) piperazinyl]-1H-indazol-6-yl}sulfonyl)amino]cyclopropanecarbonitrile

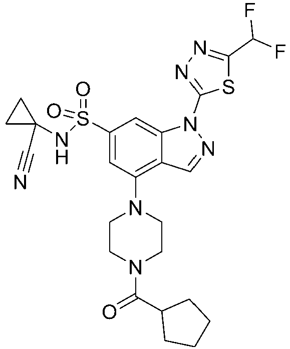

The title compound was prepared as described in Example 43 above by replacing N,N-dimethylpiperazine-1-carboxamide with 1-cyclopentanecarbonylpiperazine TFA salt. LCMS (ES, m/z): [M+H] ⁺ 577; ¹H-NMR (300 MHz, DMSO-d₆, *ppm*) δ 9.29 (brs, 1H), 8.97 (s, 1H), 8.50 (s, 1H), 7.61 (t, 1H), 7.16 (s, 1H), 3.75-3.86 (m, 4H), 3.46 (s, 4H), 3.01-3.11 (m, 1H) , 1.72-1.90 (m, 4H), 1.57-1.70 (m, 4H), 1.45-1.51 (m, 2H) , 1.20-1.30 (m, 2H).

### Example 67

### Synthesis of N-(2,2-difluoroethyl)[4-(1-[5-(difluoromethyl)(1,3,4-thiadiazol-2-yl)]-6-{[(cyano-cyclopropyl)amino]sulfonyl}(1H-indazol-4-yl))piperazinyl]-N-methylcarboxamide

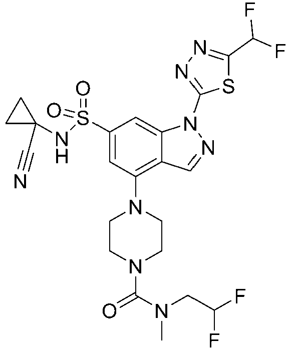

Into a 20-mL vial, were placed 4-chloro-N-(1-cyanocyclopropyl)-1-[5-(difluoromethyl)-1,3,4-thiadiazol-2-yl]indazole-6-sulfonamide (250.00 mg, 0.580 mmol, 1.00 equiv.), dioxane (5.00 mL), N-(2,2-difluoroethyl)-N-methylpiperazine-1-carboxamide (144.30 mg, 0.696 mmol, 1.20 equiv.), Cs₂CO₃ (283.60 mg, 0.870 mmol, 1.50 equiv.), RuPhos (108.31 mg, 0.232 mmol, 0.40 equiv.), and RuPhos Palladacycle Gen.3 (194.13 mg, 0.232 mmol, 0.40 equiv.). The resulting solution was stirred for 1 overnight at 90 °C under nitrogen atmosphere and then washed with H₂O. The resulting solution was extracted with ethyl acetate and the organic layers were combined and concentrated under vacuum. Purified by Prep-TLC (CH₂Cl₂:MeOH=30:1) provided crude product which was purified by Prep-HPLC (Column: XBridge Shield RP18 OBD Column, 5 um, 19*150 mm; Mobile Phase A:water (10 mmol/L NH₄HCO₃ + 0.1% NH₃·H₂O), Mobile Phase B:ACN; Flow rate:20 mL/min; Gradient: 43% B to 47% B in 10 min; 254/220 nm; RT 9.32 min) to afford the title compound (55.6 mg). LCMS (ES, m/z): [M+H]⁺ 602; ¹H-NMR (300MHz, DMSO-d₆, *ppm*) δ 9.33 (s, 1H), 8.97 (s, 1H), 8.51 (s, 1H), 7.61 (t, 1H), 7.18 (s, 1H), 6.21 (tt, 1H), 3.62 (td, 2H), 3.47-3.45 (m, 8H), 2.99 (s, 3H), 1.44 (dd, 2H), 1.33 (dd, 2H).

### Example 68

### Synthesis of 1-{[(4-{4-[((3S)-1-methylpyrrolidin-3-yl)carbonyl]piperazinyl}-1-[5-(difluoro methyl)(1,3,4-thiadiazol-2-yl)]-1H-indazol-6-yl)sulfonyl]amino}cyclopropanecarbonitrile

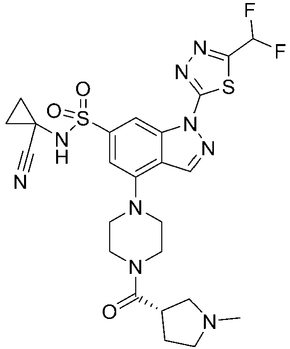

The title compound was prepared as described in Example 67 above by replacing N-(2,2-difluoroethyl)-N-methylpiperazine-1-carboxamide with (S)-(1-methylpyrrolidin-3-yl)(piperazin-1-yl)methanone TFA salt. LCMS (ES, m/z): [M+H]⁺ 592; ¹H-NMR (400 MHz, DMSO-*d*₆, *ppm*) δ 9.33 (brs, 1H), 8.96 (s, 1H), 8.49 (s, 1H), 7.61 (t, 1H), 7.16 (d, 1H), 3.76 (d, 4H), 3.44(d, 4H), 2.78 (t, 1H), 2.51-2.60 (m, 3H), 2.30-2.38 (m, 1H), 2.24 (s, 3H), 1.95-2.01 (m, 2H), 1.40 (dd, 2H), 1.27 (dd, 2H).

### Example 69

### Synthesis of 1-[({1-[5-(difluoromethyl)(1,3,4-thiadiazol-2-yl)]-4-[4-(1-methylimidazol-2-yl)piperazinyl]-1H-indazol-6-yl}sulfonyl)amino]cyclopropanecarbonitrile

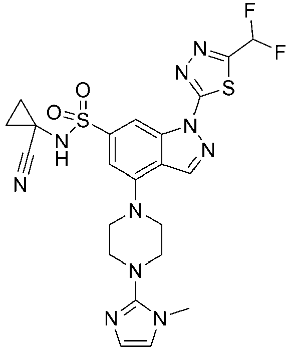

The title compound was prepared as described in Example 67 above by replacing N-(2,2-difluoroethyl)-N-methylpiperazine-1-carboxamide with 1-(1-methylimidazol-2-yl)piperazine hydrochloride salt. LCMS (ES, m/z): [M+H]⁺ 561; ¹H-NMR (400 MHz, DMSO-*d*₆, *ppm*) δ 9.36 (s, 1H), 9.02 (s, 1H), 8.54 (s, 1H), 7.62 (t, 1H), 7.25 (s, 1H), 6.93 (d, 1H), 6.65 (d, 1H), 3.58 (t, 4H), 3.53 (s, 3H), 3.27(t, 4H), 1.46 (dd, 2H), 1.33 (dd, 2H).

### Example 70

### Synthesis of 1-[({1-[5-(difluoromethyl)(1,3,4-thiadiazol-2-yl)]-4-(7-oxa-3,9-diazabicyclo-[3.3.1]non-3-yl)-1H-indazol-6-yl}sulfonyl)amino]cyclopropanecarbonitrile

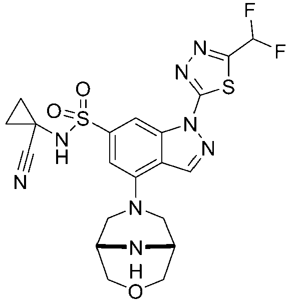

### Step 1: Synthesis of 1-[({1-[5-(difluoromethyl)(1,3,4-thiadiazol-2-yl)]-4-(N-Boc-7-oxa-3,9-diazabicyclo[3.3.1]non-3-yl)-1H-indazol-6-yl}sulfonyl)amino]cyclopropanecarbonitrile

To a stirred solution of 4-chloro-N-(1-cyanocyclopropyl)-1-[5-(difluoromethyl)-1,3,4-thiadiazol-2-yl]-indazole-6-sulfonamide (400.00 mg, 0.929 mmol, 1.00 equiv.) and tert-butyl 3-oxa-7,9-diazabicyclo[3.3.1]-nonane-9-carboxylate (423.91 mg, 1.858 mmol, 2.00 equiv.) in dioxane (4.00 mL) in an 8 mL vial, were added RuPhos (173.30 mg, 0.372 mmol, 0.40 equiv.), Cs₂CO₃ (756.26 mg, 2.322 mmol, 2.50 equiv.), RuPhos Palladacycle Gen.3 (310.61 mg, 0.372 mmol, 0.40 equiv.) under nitrogen atmosphere. The resulting mixture was stirred for overnight at 90 °C under nitrogen atmosphere and then quenched with saturated NH₄Cl (aq.). The aqueous layer was extracted with EA and the combined fractions were concentrated under vacuum. The residue was purified by Prep-TLC (DCM:MeOH=25:1) to afford 100 mg crude product as a yellow solid. The crude product (150 mg) was purified by Prep-HPLC(Column: XBridge Prep OBD C18 Column, 19*250 mm, 5 um; Mobile Phase A: water (10 mmol/L NH₄HCO₃), Mobile Phase B: ACN; Flow rate: 25 mL/min; Gradient: 56% B to 66% B in 7 min; 254;220 nm; RT 5.83 min.) to afford the title compound (15 mg) as a light yellow solid.

### Step 2: Synthesis of 1-[({1-[5-(difluoromethyl)(1,3,4-thiadiazol-2-yl)]-4-(7-oxa-3,9-diaza-bicyclo[3.3.1]non-3-yl)-1H-indazol-6-yl}sulfonyl)amino]cyclopropanecarbonitrile

To a stirred solution of 1-[({1-[5-(difluoromethyl)(1,3,4-thiadiazol-2-yl)]-4-(N-Boc-7-oxa-3,9-diazabicyclo[3.3.1]non-3-yl)-1H-indazol-6-yl}sulfonyl)amino]cyclopropanecarbonitrile (15 mg, 0.024 mmol, 1equiv) in an 8 mL vial, were added DCM (0.3 mL) and TFA (0.1 mL) under nitrogen atmosphere. The resulting mixture was stirred for overnight under nitrogen atmosphere for 12 h. The reaction was quenched with saturated NaCl (aq) and the aqueous layer was extracted with EA. The resulting mixture was concentrated under vacuum and the crude product (20 mg) was purified by Prep-HPLC with the following conditions (Column: XBridge Prep OBD C18 Column, 30×150 mm 5 um; Mobile Phase A: water (10 mmol/L NH₄HCO₃), Mobile Phase B:ACN; Flow rate: 60 mL/min; Gradient: 27% B to 40% B in 7 min; 254;220 nm; RT 5.63 min) to afford the title compound (5.2 mg). LCMS (ES, m/z): [M+H]⁺ 523; ¹H-NMR (400 MHz, DMSO-*d*₆, *ppm*) δ 9.22 (brs, 1H), 8.98 (s, 1H), 8.35 (s, 1H), 7.61 (t, 1H), 7.07 (s, 1H), 3.98 (dd, 4H), 3.80 (d, 2H), 3.51 (dd, 2H), 3.04 (s, 2H), 1.44 (dd, 2H), 1.28 (dd, 2H).

### Examples 71 and 72

### Synthesis of 1-[({4-(8-methyl-7-oxo-3,6,8-triazabicyclo[4.3.0]non-3-yl)-1-[5-(difluoromethyl)-(1,3,4-thiadiazol-2-yl)]-1H-indazol-6-yl}sulfonyl)amino]cyclopropane carbonitrile

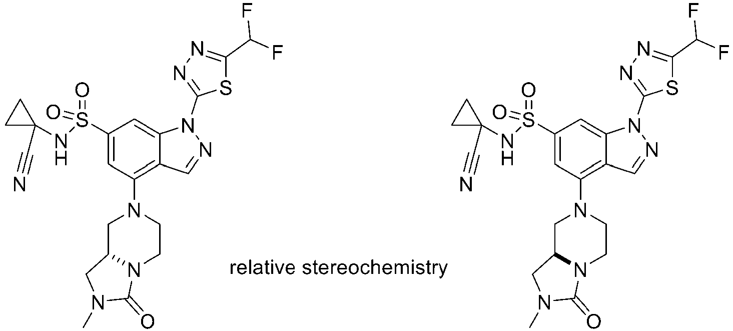

To a stirred solution of 4-chloro-N-(1-cyanocyclopropyl)-1-[5-(difluoromethyl)-1,3,4-thiadiazol-2-yl]indazole-6-sulfonamide (700.00 mg, 1.625 mmol, 1.00 equiv.) and 2-methyl-hexahydroimidazo[1,5-a]pyrazin-3-one hydrochloride (622.81 mg, 3.250 mmol, 2.00 equiv.) in dioxane (7.00 mL) in a 20 mL vial, and RuPhos (303.27 mg, 0.650 mmol, 0.40 equiv.), Cs₂CO₃ (1852.84 mg, 5.687 mmol, 3.50 equiv.) and RuPhos Palladacycle Gen.3 (543.56 mg, 0.650 mmol, 0.40 equiv.) were added under nitrogen atmosphere. The resulting mixture was stirred for overnight at 90 °C under nitrogen atmosphere. The reaction was quenched with saturated NH₄Cl (aq.) and the aqueous layer was extracted with EA. The resulting mixture was concentrated under vacuum and the residue was purified by Prep-TLC (DCM:MEOH=25:1) to afford 220 mg of crude product as a yellow solid. The crude product was purified by Prep-SFC (Column: CHIRALPAK IA-3, 4.6*50 mm, 3 um; Mobile Phase A: Hex (0.1%DEA), Mobile Phase B: EtOH; Flow rate:1 mL/min; Gradient:50 %B to 50 %; enantiomer A RT 2.77 min; enantiomer B RT 2.40 min)to afford 1-[({4-(8-methyl-7-oxo-3,6,8-triazabicyclo[4.3.0]non-3-yl)-1-[5-(difluoromethyl)(1,3,4-thiadiazol-2-yl)]-1H-indazol-6-yl}sulfonyl)amino]cyclopropane carbonitrile enantiomer A (37.7 mg) and enantiomer B( 33.4 mg

Characterization Example 71 (enantiomer A): LCMS (ES, m/z): [M+H]⁺ 550; ¹H-NMR (300 MHz, DMSO-*d*₆, *ppm*) δ 9.38 (brs, 1H), 8.98 (s, 1H), 8.53 (s, 1H), 7.61 (t, 1H), 7.21(d, 1H), 3.73-3.98 (m, 4H), 3.46 (t, 1H), 3.22 (td, 1H), 3.09 (dd, 1H), 2.85-2.95 (m, 2H), 2.72 (s, 3H), 1.44 (dd, 2H),1.32 (dd, 2H).

Characterization Example 72 (enantiomer B): LCMS (ES, m/z): [M+H]⁺ 550; ¹H-NMR (300 MHz, DMSO-d₆, *ppm*) 9.35 (s, 1H), 8.99 (s, 1H), 8.53 (s, 1H), 7.62 (t, 1H), 7.21 (d, 1H), 3.67-3.98 (m, 4H), 3.46 (t, 1H), 3.31 (td, 1H), 3.09 (dd, 1H), 2.85-2.95 (m, 2H), 2.72 (s, 3H), 1.45 (dd, 2H), 1.32 (dd, 2H).

### Example 73

### Synthesis of 1-[({1-[5-(difluoromethyl)(1,3,4-thiadiazol-2-yl)]-4-(5-methyl(3-pyrrolino[3,4-c]pyrazol-2-yl))-1H-indazol-6-yl}sulfonyl)amino]cyclopropanecarbonitrile

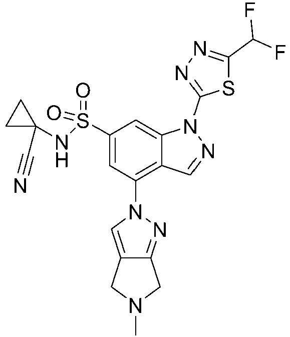

To a stirred solution of 4-chloro-N-(1-cyanocyclopropyl)-1-[5-(difluoromethyl)-1,3,4-thiadiazol-2-yl]indazole-6-sulfonamide (400.00 mg, 0.928 mmol, 1.00 equiv.) and 5-methyl-2H,4H,6H-pyrrolo[3,4-c]pyrazole (228.69 mg, 1.857 mmol, 2.00 equiv.) in dioxane (5.00 mL) in an 8 mL vial, Cs₂CO₃ (756.26 mg, 2.321 mmol, 2.50 equiv.), t-BuXPhos (78.85 mg, 0.186 mmol, 0.20 equiv.) and t-BuXPhosPd-G3 (73.72 mg, 0.093 mmol, 0.10 equiv.) were added at room temperature under nitrogen atmosphere. The resulting mixture was stirred overnight at 60 °C under nitrogen atmosphere and then quenched with NH₄Cl (aq). The aqueous layer was extracted with EtOAc and the combined extracts were concentrated under vacuum. The residue was purified by Prep-TLC (CH₂Cl₂:MeOH=30:1) to afford crude product (300 mg). The crude product was purified by Prep-HPLC (Column: XBridge Prep OBD C18 Column, 30×150 mm 5 um; Mobile Phase A: water (0.05% NH₃·H₂O), Mobile Phase B: ACN; Flow rate: 60 mL/min; Gradient: 22% B to 33% B in 7 min; 254;220 nm; RT 5.33 min) to afford the title compound (82 mg). LCMS (ES, m/z): [M+H]⁺ 518; ¹H-NMR (300MHz, DMSO-d₆, *ppm*) δ 9.54 (brs, 1H), 9.15 (s, 1H), 8.86 (s, 1H), 8.43 (s, 1H), 8.09 (d, 1H), 7.62 (t, 1H), 3.77 (d, 4H), 2.52-2.60 (m, 3H), 1.45 (dd, 2H), 1.33 (dd, 2H).

### Example 74

### Synthesis of 4-(6-(N-(1-cyanocyclopropyl)sulfamoyl)-1-(5-(difluoromethyl)-1,3,4-thiadiazol-2-yl)-1H-indazol-4-yl)-N-methylpiperazine-1-carboxamide

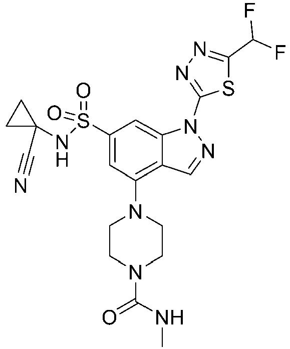

To a stirred solution of 4-chloro-N-(1-cyanocyclopropyl)-1-[5-(difluoromethyl)-1,3,4-thiadiazol-2-yl]indazole-6-sulfonamide (300.00 mg, 0.696 mmol, 1.00 equiv.) and N-methylpiperazine-1-carboxamide TFA salt (335.93 mg, 1.393 mmol, 2.00 equiv.) in dioxane (3.00 mL) in an 8 mL vial, were added RuPhos (129.97 mg, 0.279 mmol, 0.40 equiv.), K₂CO₃ (336.83 mg, 2.437 mmol, 3.50 equiv.), and RuPhos Palladacycle Gen.3 (232.96 mg, 0.279 mmol, 0.40 equiv.) under nitrogen atmosphere. The resulting mixture was stirred for overnight at 90 °C under nitrogen atmosphere for 12 h and then quenched with saturated NH₄Cl (aq). The aqueous layer was extracted with EtOAc and the organic layer was concentrated under vacuum. The residue was purified by Prep-TLC (DCM:MeOH=25:1) to afford 100 mg crude product as a yellow solid. The crude product was purified by Prep-HPLC (Column: Xbridge Phenyl OBD Column, 5 um,19*150 mm; Mobile Phase A: water (10 mmol/L NH₄HCO₃), Mobile Phase B: ACN; Flow rate: 20 mL/min; Gradient: 42% B to 52% B in 8 min; 254;220 nm; Rt: 8.13 min) to afford the title compound (40.7 mg). LCMS (ES, m/z): [M+H]⁺ 538; ¹H-NMR (300 MHz, DMSO-d₆, ppm) δ 9.35 (brs, 1H), 8.97 (s, 1H), 8.51 (d, 1H), 7.60 (t, 1H), 7.16 (d, 1H), 6.57 (d, 1H), 3.57-3.64 (m, 4H), 3.43-4.44 (m, 4H), 2.62 (d, 3H), 1.44 (dd, 2H), 1.32 (dd, 2H).

### Example 75

### Synthesis of 1-(6-(N-(1-cyanocyclopropyl)sulfamoyl)-1-(5-(difluoromethyl)-1,3,4-thiadiazol-2-yl)-1H-indazol-4-yl)-N-methylazetidine-3-carboxamide

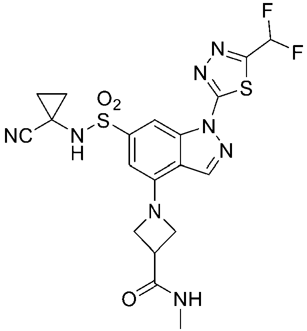

The title compound was prepared as described in Example 74 above by replacing N-methylpiperazine-1-carboxamide TFA salt with N-methylazetidine-3-carboxamide TFA salt. LCMS (ES, m/z): [M+H]⁺ 509; ¹H-NMR (300 MHz, DMSO-*d*₆, *ppm*) δ 9.24 (s, 1H), 8.72 (s, 1H), 8.20 (s, 1H), 8.09 (d, 1H), 7.60 (t, 1H), 6.59 (s, 1H), 4.42 (t, 2H), 4.29 (t, 2H), 3.52-3.65 (m, 1H), 2.65 (d, 3H), 1.25-1.45 (m, 4H).

### Example 76

### Synthesis of [4-(1-[5-(difluoromethyl)(1,3,4-thiadiazol-2-yl)]-6-{[(cyanocyclopropyl)amino] sulfonyl}(1H-indazol-4-yl))piperazinyl]-N-[2-(dimethylamino)ethyl]-N-methylcarboxamide

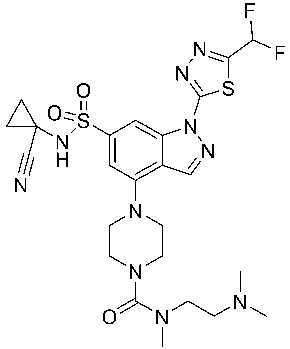

The title compound was prepared as described in Example 74 above by replacing N-methylpiperazine-1-carboxamide TFA salt with N-[2-(dimethylamino)ethyl]-N-methylpiperazine-1-carboxamide TFA salt. LCMS (ES, m/z): [M+H]⁺ 609; ¹H-NMR (300 MHz, DMSO-*d*₆, *ppm*) δ 9.32(brs, 1H), 8.97 (s, 1H), 8.50 (s, 1H), 7.61 (t, 1H), 7.18 (s, 1H), 3.45 (s, 4H), 3.38 (s, 4H), 3.27 (t, 2H), 2.86 (s, 3H), 2.41 (t, 2H), 2.17 (s, 6H), 1.30-1.46 (m, 4H).

### Example 77

### Synthesis of [4-(1-[5-(difluoromethyl)(1,3,4-thiadiazol-2-yl)]-6-{[(cyanocyclopropyl)amino] sulfonyl}(1H-indazol-4-yl))piperazinyl]-N-methyl-N-propylcarboxamide

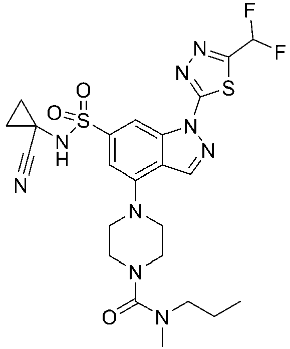

The title compound was prepared as described in Example 74 above by replacing N-methylpiperazine-1-carboxamide TFA salt with N-methyl-N-propylpiperazine-1-carboxamide TFA salt. LCMS (ES, m/z): [M+H]⁺ 580; ¹H-NMR (300 MHz, DMSO-d₆, *ppm*) δ 9.33 (s, 1H), 8.96 (s, 1H), 8.50 (s, 1H), 7.61 (t, 1H), 7.18 (s, 1H), 3.37-3.47 (m, 8H), 3.10-3.15 (t, 2H), 2.82 (s, 3H), 1.54 (q, 2H), 1.44 (dd, 2H), 1.33 (dd, 2H), 0.84 (t, 3H).

### Example 78

### Synthesis of [4-(1-[5-(difluoromethyl)(1,3,4-thiadiazol-2-yl)]-6-{[(cyanocyclopropyl)amino]-sulfonyl}(1H-indazol-4-yl))(1,4-diazaperhydroepinyl)]-N,N-dimethylcarboxamide

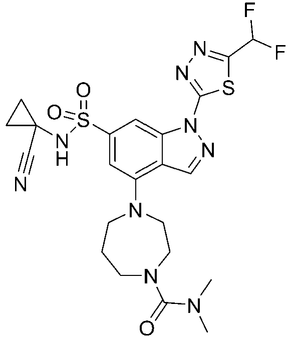

The title compound was prepared as described in Example 74 above by replacing N-methylpiperazine-1-carboxamide TFA salt with N,N-dimethyl-1,4-diazepane-1-carboxamide TFA salt. LCMS (ES, m/z): [M+H]⁺ 566; ¹H-NMR (300MHz, DMSO-d₆, *ppm*) δ 9.30 (s,1H), 8.86 (s, 1H), 8.27 (s, 1H), 7.60 (t, 1H), 6.97 (s, 1H), 3.87-3.91 (m, 4H), 3.59 (s, 2H), 3.20-3.29 (m, 2H), 2.65 (s, 6H), 2.03-2.08 (d, 2H), 1.26-1.45 (m, 4H).

### Example 79

### Synthesis of 1-[({1-[5-(difluoromethyl)(1,3,4-thiadiazol-2-yl)]-4-[4-(3-methyl(2-pyridyl))-piperazinyl]-1H-indazol-6-yl}sulfonyl)amino]cyclopropanecarbonitrile

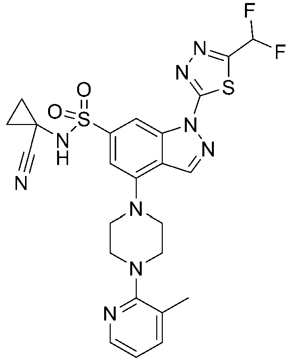

The title compound was prepared as described in Example 74 above by replacing N-methylpiperazine-1-carboxamide TFA salt with 1-(3-methylpyridin-2-yl)piperazine dihydrochloride hydrochloride salt. LCMS (ES, m/z): [M+H]⁺ 572; ¹H-NMR (400 MHz, DMSO-d₆, *ppm*) δ 9.01 (brs, 1H), 9.01 (s, 1H), 8.53 (s, 1H), 8.17 (d, 1H), 7.62 (t, 1H), 7.56 (d, 1H), 7.26 (s, 1H), 6.99 (dd, 1H), 3.61 (t, 4H), 3.38 (t, 4H), 2.33 (s, 3H), 1.45 (t, 2H), 1.32 (dd, 2H).

### Example 80

### Synthesis of 1-[({1-[5-(difluoromethyl)(1,3,4-thiadiazol-2-yl)]-4-[4-(cyclopropylcarbonyl) piperazinyl]-1H-indazol-6-yl}sulfonyl)amino]cyclopropanecarbonitrile

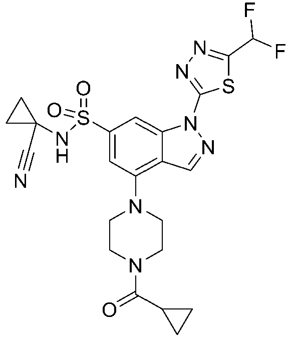

The title compound was prepared as described in Example 74 above by replacing N-methylpiperazine-1-carboxamide TFA salt with 1-cyclopropanecarbonylpiperazine. LCMS (ES, m/z): [M+H]⁺ 549; ¹H -NMR (400MHz, DMSO-d₆, *ppm)* δ 9.34 (s, 1H), 8.98 (s, 1H), 8.50 (s, 1H), 7.61 (t, 1H), 7.17 (d, 1H), 3.98 (s, 2H), 3.76 (s, 2H), 3.48-3.55 (m, 4H), 3.17 (d, 1H), 2.02-2.08 (m, 1H), 1.44 (dd, 2H), 1.32 (dd, 2H), 0.70-0.80 (m, 4H).

### Example 81

### Synthesis1-[({1-[5-(difluoromethyl)(1,3,4-thiadiazol-2-yl)]-4-[4-(cyclobutylcarbonyl)piperazinyl ]-1H-indazol-6-yl}sulfonyl)amino]cyclopropanecarbonitrile

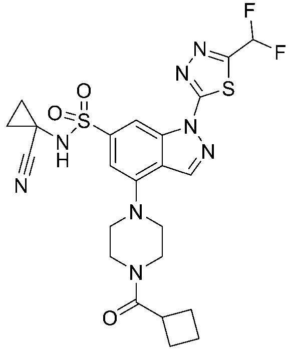

The title compound was prepared as described in Example 74 above by replacing N-methylpiperazine-1-carboxamide TFA salt with 1-cyclobutanecarbonylpiperazine TFA salt. LCMS (ES, m/z): [M+H]⁺ 563; ¹H -NMR (400MHz, DMSO-d₆, *ppm*) δ 9.33 (s, 1H), 8.96 (s, 1H), 8.50 (s, 1H), 7.47-7.74 (t, 1H), 7.16 (s, 1H), 3.73 (s, 2H), 3.61 (s, 2H), 3.41-3.47 (m, 5H), 2.12-2.27 (m, 4H), 1.89-1.96 (m, 1H), 1.77-1.81 (m, 1H), 1.44 (dd, 2H), 1.32 (dd, 2H).

### Example 82

### Synthesis of 1-[({4-[(3R)-3-methyl-4-(2-methylpropanoyl)piperazinyl]-1-[5-(difluoromethyl) (1,3,4-thiadiazol-2-yl)]-1H-indazol-6-yl}sulfonyl)amino]cyclopropanecarbonitrile

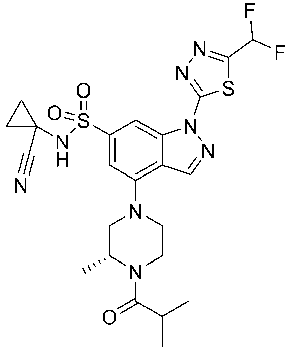

The title compound was prepared as described in Example 74 above by replacing N-methylpiperazine-1-carboxamide TFA salt with (R)-2-methyl-1-(2-methylpiperazin-1-yl)propan-1-one TFA salt. LCMS (ES, m/z): [M+H]⁺ 565; ¹H -NMR (300 MHz, DMSO-d₆, *ppm*) δ 9.32 (s, 1H), 8.86 (s, 1H), 8.7 (s, 1H), 7.60 (t, 1H), 7.14 (brs, 1H), 4.70 (brs, 1H), 4.20-4.55 (m, 1H), 3.97 (brs, 1H), 3.86 (d, 1H), 33.73 (d, 2H), 3.00-3.20 (m, 1H), 2.91 (p, 1H), 1.32-1.50 (m, 3H), 1.20-1.30 (m, 4H), 1.04-1.06 (d, 6H).

### Example 83

### Synthesis of [(2S)-4-(1-[5-(difluoromethyl)(1,3,4-thiadiazol-2-yl)]-6-{[(cyanocyclopropyl) amino]sulfonyl}(1H-indazol-4-yl))-2-methylpiperazinyl]-N,N-dimethylcarboxamide

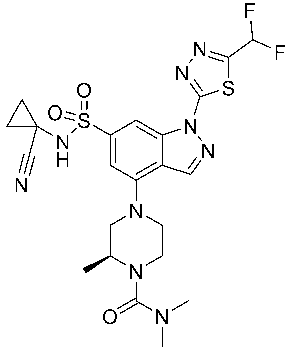

To a stirred solution of 4-chloro-N-(1-cyanocyclopropyl)-1-[5-(difluoromethyl)-1,3,4-thiadiazol-2-yl]indazole-6-sulfonamide (250.00 mg, 0.580 mmol, 1.00 equiv.) and (S)-N,N,2-trimethylpiperazine-1-carboxamide TFA salt (312.50 mg, 1.161 mmol, 2.00 equiv.) in dioxane (2.50 mL) in an 8 mL vial, RuPhos (108.31 mg, 0.232 mmol, 0.40 equiv.), K₂CO₃ (280.69 mg, 2.031 mmol, 3.5 equiv.) and RuPhos Palladacycle Gen.3 (194.13 mg, 0.232 mmol, 0.40 equiv.) were added under nitrogen atmosphere. The resulting mixture was stirred for overnight at 90 °C under nitrogen atmosphere and then quenched with saturated NH₄Cl (aq.). The aqueous layer was extracted with EA and the combined extracts were concentrated under vacuum. The residue was purified by Prep-TLC (DCM:MeOH=25:1) to afford 30 mg crude product as a yellow solid. The crude product was purified by Prep-HPLC (Column: XBridge Prep OBD C₁₈ Column, 30×150 mm 5 um; Mobile Phase A: water (10 mmol/L NH₄HCO₃), Mobile Phase B: ACN; Flow rate: 60 mL/min; Gradient:35% B to 45 %B in 7 min; 254;220 nm; RT: 5.92 min) afford the title compound (3.2 mg). LCMS (ES, m/z): [M+H]⁺ 566; ¹H -NMR (400 MHz, DMSO-*d*₆, *ppm*) δ 9.30 (brs, 1H), 8.87 (s, 1H), 8.49 (s, 1H), 7.62 (t, 1H), 7.16 (s, 1H), 3.97 (dd, 1H), 3.69 (d, 1H), 3.60 (d, 1H), 3.44 (d, 2H), 3.28-3.30 (m, 1H), 3.18-3.24 (m, 1H), 2.81 (s, 6H), 1.44 (d, 2H), 1.31-1.33 (m, 5H).

### Example 85

### Synthesis of 1-{[(1-[5-(difluoromethyl)(1,3,4-thiadiazol-2-yl)]-4-{4-[(1-methyl(4-piperidyl))-carbonyl]piperazinyl}-1H-indazol-6-yl)sulfonyl]amino}cyclopropanecarbonitrile

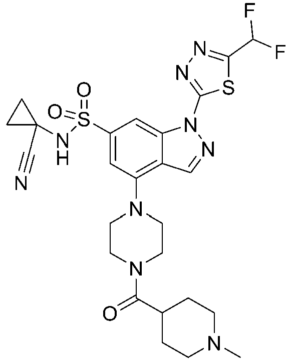

The title compound was prepared as described in Example 83 above by replacing (S)-N,N,2-trimethylpiperazine-1-carboxamide TFA salt with (1-methylpiperidin-4-yl)(piperazin-1-yl)methanone TFA salt. LCMS (ES, m/z): [M+H]⁺ 606; ¹H -NMR (400 MHz, DMSO-*d*₆, *ppm*) δ 9.33 (brs, 1H), 8.96 (s, 1H), 8.49 (s, 1H), 7.61 (t, 1H), 7.16 (s, 1H), 3.76 (d, 4H), 3.46 (d, 4H), 2.79 (d, 2H), 2.55-2.64 (m, 1H), 2.16 (s, 3H), 1.92 (td, 2H), 1.59-1.65 (m, 4H), 1.40 (dd, 2H), 1.29 (dd, 2H).

### Example 84

### Synthesis of 1-[({1-[5-(difluoromethyl)(1,3,4-thiadiazol-2-yl)]-4-[4-(4-methyl(3-pyridyl)) piperazinyl]-1H-indazol-6-yl}sulfonyl)amino]cyclopropanecarbonitrile

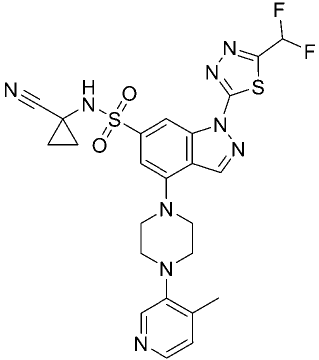

Into a 25-mL round-bottom flask purged and maintained with an inert atmosphere of nitrogen, were placed 4-chloro-N-(1-cyanocyclopropyl)-1-[5-(difluoromethyl)-1,3,4-thiadiazol-2-yl]indazole-6-sulfonamide (170.00 mg, 0.395 mmol, 1.00 equiv.), 1-(4-methylpyridin-3-yl)piperazine TFA salt (210 mg, 1.184 mmol, 3 equiv.), RuPhos Palladacycle Gen.3 (132.01 mg, 0.158 mmol, 0.4 equiv.), RuPhos (73.65 mg, 0.158 mmol, 0.4 equiv.), and Cs₂CO₃ (514.26 mg, 1.578 mmol, 4 equiv.), dioxane (3.40 mL). The resulting solution was stirred for 12 h at 90 °C in an oil bath and then quenched with water. The resulting solution was extracted with ethyl acetate and the organics were washed with brine, dried over anhydrous sodium sulfate and concentrated. The residue was applied onto a silica gel column and eluted with ethyl acetate/petroleum ether (1/1). The crude product was purified by Prep-HPLC (Column: XBridge Shield RP18 OBD Column, 19*250 mm, 10 um; Mobile Phase A: water (0.1%FA), Mobile Phase B: ACN; Flow rate:20 mL/min; Gradient: 33% B to 50% B in 7 min; 254/220 nm; RT: 5.87 min) to afford the title compound (2.7 mg). LCMS (ES, m/z): [M+H]⁺ 572; ¹H -NMR (300 MHz, DMSO-d₆, *ppm*) δ 9.00 (s, 1H), 8.54(s, 1H), 8.33 (s, 1H), 8.20 (d, 1H), 7.62 (t, 1H), 7.26 (s, 1H), 7.24 (d, 1H), 3.62 (s, 4H), 3.24 (s, 4H), 2.35 (s, 3H), 1.43 (dd, 2H), 1.33 (dd, 4H).

### Example 86

### Synthesis of [4-(1-[5-(difluoromethyl)(1,3,4-thiadiazol-2-yl)]-6-{[(cyanocyclopropyl)amino] sulfonyl}(1H-indazol-4-yl))piperazinyl]-N-methyl-N-(2,2,2-trifluoroethyl)carboxamide

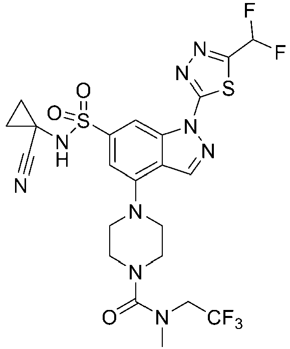

To a stirred solution of 4-chloro-N-(1-cyanocyclopropyl)-1-[5-(difluoromethyl)-1,3,4-thiadiazol-2-yl]indazole-6-sulfonamide (250.00 mg, 0.580 mmol, 1.00 equiv.) and N-methyl-N-(2,2,2-trifluoroethyl)piperazine-1-carboxamide TFA salt (375.14 mg, 1.161 mmol, 2.00 equiv.) in 1,4-dioxane (3 mL) in a 20 mL sealed tube were added K₂CO₃ (280.69 mg, 2.031 mmol, 3.50 equiv.), RuPhos (108.31 mg, 0.232 mmol, 0.40 equiv.) and RuPhos Palladacycle Gen.3 (194.13 mg, 0.232 mmol, 0.40 equiv.) under nitrogen atmosphere. The resulting mixture was stirred for overnight at 90 °C under nitrogen atmosphere. The reaction was quenched with saturated NH₄Cl (aq.) and then diluted with water. The resulting mixture was extracted with EtOAc and the combined organic layers were concentrated under vacuum. The residue was purified by Prep-TLC (CH₂Cl₂:MeOH = 30:1) to afford the crude product (50 mg) which was purified by crystallization from CH₂Cl₂:hexane (1:10, 1 mL) to give the title compound (20.7 mg). LCMS (ES, m/z): [M+H]⁺ 620; ¹H-NMR (400MHz, DMSO-d₆, *ppm*) δ 9.35 (s, 1H), 8.98 (d, 1H), 8.52 (s, 1H), 7.61 (t, 1H), 7.19 (d, 1H), 4.12 (q, 2H), 3.48 (s, 8H), 3.05 (s, 3H), 1.45 (dd, 2H), 1.33 (dd, 2H).

### Example 87

### Synthesis of 1-[({1-[5-(difluoromethyl)(1,3,4-thiadiazol-2-yl)]-4-[4-(2-pyridylcarbonyl) piperazinyl]-1H-indazol-6-yl}sulfonyl)amino]cyclopropanecarbonitrile

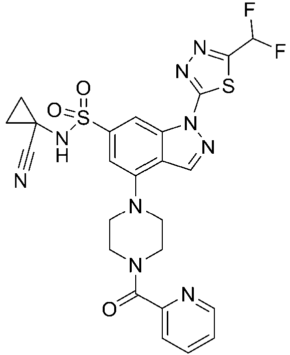

Into a 20-mL vial, was placed 4-chloro-N-(1-cyanocyclopropyl)-1-[5-(difluoromethyl)-1,3,4-thiadiazol-2-yl]indazole-6-sulfonamide (300.00 mg, 0.696 mmol, 1.00 equiv.), dioxane (5.00 mL), 1-(pyridine-2-carbonyl)piperazine (266.32 mg, 1.393 mmol, 2.00 equiv.), K₂CO₃ (288.71 mg, 2.089 mmol, 3.00 equiv.), RuPhos (129.97 mg, 0.279 mmol, 0.40 equiv.), RuPhos Palladacycle Gen.3 (232.96 mg, 0.279 mmol, 0.40 equiv.). The resulting solution was stirred for 1 overnight at 90 degrees C under nitrogen atmosphere. The resulting mixture was washed with 3x5 mL of H₂O. The resulting solution was extracted with 2x5 mL of ethyl acetate and the combined extracts were concentrated under vacuum. The residue was purified by Prep-TLC (CH₂Cl₂: MeOH=30:1) to afford 1-[({1-[5-(difluoromethyl)(1,3,4-thiadiazol-2-yl)]-4-[4-(2-pyridyl carbonyl)piperazinyl]-1H-indazol-6-yl}sulfonyl)amino]cyclopropanecarbonitrile (9.7 mg). LCMS (ES, m/z): [M+H]⁺ 586; ¹H-NMR (300 MHz, DMSO-d₆, *ppm*) δ 9.33 (s, 1H), 8.97 (s, 1H), 8.64 (d, 1H), 8.51 (s, 1H), 7.98 (td, 1H), 7.67 (d, 1H), 7.61 (t, 3H), 7.53 (dd, 1H), 7.19 (d, 1H), 3.95 (s, 2H), 3.75 (s, 2H), 3.58 (s, 2H), 3.48 (s, 2H), 1.45 (dd, 2H), 1.32 (dd, 2H).

### Example 88

### Synthesis of 1-[({4-[(3S)-3-methyl-4-(2-methylpropanoyl)piperazinyl]-1-[5-(difluoromethyl) (1,3,4-thiadiazol-2-yl)]-1H-indazol-6-yl}sulfonyl)amino]cyclopropanecarbonitrile

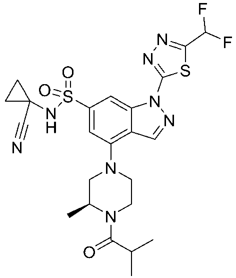

Into a 20-mL sealed tube were placed 4-chloro-N-(1-cyanocyclopropyl)-1-[5-(difluoromethyl)-1,3,4-thiadiazol-2-yl]indazole-6-sulfonamide (300.00 mg, 0.696 mmol, 1.00 equiv.), K₂CO₃ (336.83 mg, 2.437 mmol, 3.5 equiv.), dioxane (3.00 mL) in an 8 mL vial, (S)-2-methyl-1-(2-methylpiperazin-1-yl)propan-1-one TFA salt (373.62 mg, 1.393 mmol, 2 equiv.), RuPhos Pd Gen.3 (232.96 mg, 0.279 mmol, 0.4 equiv.) and RuPhos (129.97 mg, 0.279 mmol, 0.40 equiv.) was added. The mixture was stirred for 1 overnight at 90 °C under nitrogen atmosphere and then quenched with saturated NH₄Cl (aq.) at room temperature. The aqueous layer was extracted with EtOAc and the resulting mixture was concentrated under vacuum. The residue was purified by Prep-TLC (DCM:MeOH=30:1) to afford crude product (120 mg). The crude product was purified by Prep-HPLC (Column: XBridge Prep OBD C18 Column, 19*250 mm, 5 um; Mobile Phase A: water (10 mmol/L NH₄HCO₃ + 0.1% NH₃.H₂O), Mobile Phase B: ACN; Flow rate: 20 mL/min; Gradient: 45% B to 60% B in 7 min; 254/220 nm; RT: 9.50 min) to afford the title compound (3.3 mg). LCMS (ES, m/z): [M+H]⁺ 565; ¹H-NMR (300 MHz, DMSO-d₆, *ppm*) δ 9.15 (brs, 1H), 8.84 (s, 1H), 8.46 (s, 1H), 7.61 (t, 1H), 7.13 (s, 1H), 4.65-4.75 (m, 1H), 4.20-4.55 (m, 1H), 3.88-3.95 (m, 1H), 3.84 (d, 1H), 3.72 (d, 2H), 3.10-3.20 (m,1H), 2.91 (p, 1H), 1.24-1.45 (m, 7H), 1.04 (d, 6H).

### Example 89

### Synthesis of 1-[({4-[cis-3,5-dimethyl-4-(2-methylpropanoyl)piperazinyl]-1-[5-(difluoromethyl)-(1,3,4-thiadiazol-2-yl)]-1H-indazol-6-yl}sulfonyl)amino]cyclopropanecarbonitrile

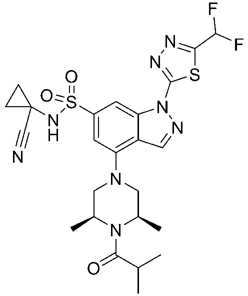

Into a 20-mL round-bottom flask purged and maintained with an inert atmosphere of nitrogen, were placed 4-chloro-N-(1-cyanocyclopropyl)-1-[5-(difluoromethyl)-1,3,4-thiadiazol-2-yl]indazole-6-sulfonamide (600.00 mg, 1.393 mmol, 1.00 equiv.), 1-(*cis*-2,6-dimethyl-piperazin-1-yl)-2-methylpropan-1-one TFA salt (513.29 mg, 2.785 mmol, 2.00 equiv.), RuPhos (259.95 mg, 0.557 mmol, 0.40 equiv.), K₂CO₃ (577.42 mg, 4.178 mmol, 3.00 equiv.), dioxane (10.00 mL), and RuPhos Pd Gen.3 (465.91 mg, 0.557 mmol, 0.40 equiv.). The resulting solution was stirred for 12h at 90 °C in an oil bath and then quenched with water. The resulting solution was extracted with ethyl acetate. The organic layer was washed with brine, dried over anhydrous sodium sulfate and concentrated. The residue was applied onto a silica gel column and eluted with dichloromethane/methanol (20/1) to provide crude product (8 mg). The crude product was purified by Prep-HPLC (Column: XBridge Prep OBD C18 Column, 30×150 mm 5 um; Mobile Phase A: water (10 mmol/L NH₄HCO₃), Mobile Phase B:ACN; Flow rate: 60 mL/min; Gradient: 43% B to 55% B in 7 min; 254;220 nm; RT:6.38 min) to afford the title compound (1.3 mg). LCMS (ES, m/z): [M+H]⁺ 579; ¹H-NMR (300 MHz, DMSO-d₆, *ppm*) δ 9.23 (brs, 1H), 8.71 (s, 1H), 8.50 (s, 1H), 7.61 (t, 1H), 7.20 (s, 1H), 4.58-4.70 (m, 1H), 4.25-4.40 (m, 1H), 3.73 (d, 2H), 3.05-3.30 (m, 2H), 2.89 (p, 1H), 1.30-1.54 (m, 8H), 1.20-1.29 (m, 2H), 1.07 (d, 6H).

### Example 90

### Synthesis of 1-{[(4-{4-[((2R)-1-methylpyrrolidin-2-yl)carbonyl]piperazinyl}-1-[5-(difluoro methyl)(1,3,4-thiadiazol-2-yl)]-1H-indazol-6-yl)sulfonyl]amino}cyclopropanecarbonitrile

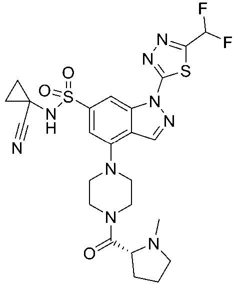

The title compound was prepared as described in Example 89 above by replacing 1-(*cis*-2,6-dimethylpiperazin-1-yl)-2-methylpropan-1-one TFA salt with 1-(methyl-D-prolyl)piperazine TFA salt. LCMS (ES, m/z): [M+H]⁺ 592; ¹H-NMR (400 MHz, DMSO-d₆ , *ppm*) δ 9.21 (brs, 1H), 8.99 (s, 1H), 8.50 (s, 1H), 7.61 (t, 1H), 7.17 (s, 1H), 4.06-3.68 (brt, 4H), 3.59-3.41 (brd, 4H), 3.19 (t, 1H), 2.97 (s, 1H), 2.26 (s, 3H), 2.23-2.18 (m, 1H), 2.05-2.18 (m, 1H), 1.87-1.79 (m, 3H) , 1.42(t, 2H) , 1.30 (t, 2H).

### Example 91

### Synthesis of 1-[({1-[5-(difluoromethyl)(1,3,4-thiadiazol-2-yl)]-4-[6-(2-methylpropanoyl)-3,6-diazabicyclo[3.1.1]hept-3-yl]-1H-indazol-6-yl}sulfonyl)amino]cyclopropanecarbonitrile

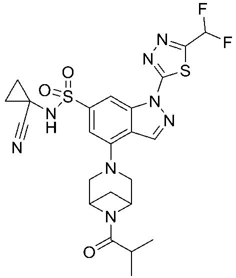

The title compound 1 was prepared as described in Example 89 above by replacing 1-(*cis*-2,6-dimethylpiperazin-1-yl)-2-methylpropan-1-one TFA salt with 1-(3,6-diaza-bicyclo[3.1.1]heptan-6-yl)-2-methylpropan-1-one TFA salt. LCMS (ES, m/z): [M+H]⁺ 563; H-¹H -NMR (400 MHz, DMSO-d₆, *ppm*) δ 9.28 (s, 1H), 8.84 (s, 1H), 8.30 (s, 1H), 7.60 (t, 1H), 6.86 (d, 1H), 4.85 (s, 2H), 4.13 (brs, 1H), 3.78 (obs brs, 1H), 3.74 (d, 1H), 3.49 (d, 1H), 2.88 (q, 1H), 2.60 (p, 1H), 1.74 (d, 1H), 1.32-1.42 (m, 2H), 1.15-1.24 (m, 2H), 0.92 (d, 3H) , 0.63 (d, 3H).

### Example 92

### Synthesis of 1-[({1-[5-(difluoromethyl)(1,3,4-thiadiazol-2-yl)]-4-(2-methyl-2,7-diazaspiro-[3.5]non-7-yl)-1H-indazol-6-yl}sulfonyl)amino]cyclopropanecarbonitrile

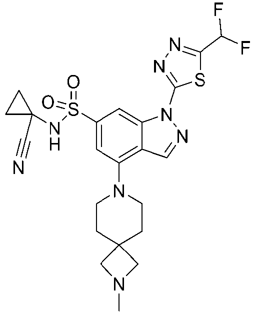

To a stirred solution of 4-chloro-N-(1-cyanocyclopropyl)-1-[5-(difluoromethyl)-1,3,4-thiadiazol-2-yl]indazole-6-sulfonamide (500.00 mg, 1.160 mmol, 1.00 equiv.) and 2-methyl-2,7-diazaspiro[3.5]nonane (542.48 mg, 3.868 mmol, 2.00 equiv.) in dioxane (5.00 mL) in an 8 mL vial, were added RuPhos (361.03 mg, 0.773 mmol, 0.4 equiv.), K₃PO₄ (1847.60 mg, 8.703 mmol, 4.5 equiv.), RuPhos Palladacycle Gen.3 (647.10 mg, 0.773 mmol, 0.4 equiv.) under nitrogen atmosphere. The resulting mixture was stirred for overnight at 90 °C under nitrogen atmosphere for 12h and then quenched with saturated NH₄Cl aqueous. The aqueous layer was extracted with EtOAc and the resulting organic layer was concentrated under vacuum. The residue was purified by Prep-TLC (DCM:MeOH=25:1) to afford 260 mg crude product as a yellow solid. The crude product (260 mg) was purified by Prep-HPLC (Column: XBridge Prep OBD C18 Column, 30×150 mm 5 um; Mobile Phase A: water (0.05% NH₃·H₂O), Mobile Phase B: ACN; Flow rate: 60 mL/min; Gradient: 21% B to 37% B in 7 min; 254;220 nm; RT: 6.73 min) to afford the title compound (51.0 mg). LCMS (ES, m/z): [M+H]⁺: ¹H-NMR (300 MHz, DMSO-*d*₆, *ppm*) δ 8.87 (s, 1H), 8.45 (s, 1H), 7.61 (t, 1H), 7.16 (d, 1H), 3.35-3.38 (m, 4H), 3.04 (s, 4H), 2.28 (s, 3H), 1.92 (t, 4H), 1.43 (dd, 2H), 1.30 (dd, 2H).

### Example 93

### Synthesis of 1-[({1-[5-(difluoromethyl)(1,3,4-thiadiazol-2-yl)]-4-(4-(2-pyridyl)piperazinyl)-1H-indazol-6-yl}sulfonyl)amino]cyclopropanecarbonitrile

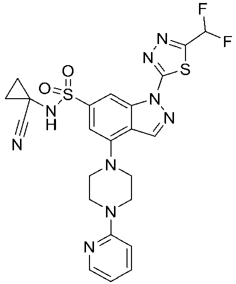

To a stirred solution of 4-chloro-N-(1-cyanocyclopropyl)-1-[5-(difluoromethyl)-1,3,4-thiadiazol-2-yl]indazole-6-sulfonamide (300.00 mg, 0.696 mmol, 1.00 equiv.) and 1-(pyridin-2-yl)piperazine (227.32 mg, 1.393 mmol, 2.00 equiv.)in dioxane (3.00 mL) in an 8 mL vial, RuPhos (129.97 mg, 0.279 mmol, 0.4 equiv.), Pd(OAc)₂ (62.53 mg, 0.279 mmol, 0.4 equiv.) and CS₂CO₃ (567.20 mg, 1.741 mmol, 2.5 equiv.) were added under nitrogen atmosphere. The resulting mixture was stirred for overnight at 90 °C under nitrogen atmosphere and then quenched by the addition of saturated NH₄Cl (aq.). The aqueous layer was extracted with ethyl acetate and the combined extracts were concentrated under vacuum. The residue was purified by Prep-TLC (DCM:MEOH=25:1) to afford crude product (200 mg) as a yellow solid. The crude product was purified by Prep-HPLC (Column: XBridge Prep OBD C₁₈ Column, 30×150 mm 5 um; Mobile Phase A: water (10 mmol/L NH₄HCO₃), Mobile Phase B: ACN; Flow rate: 60 mL/min; Gradient: 48% B to 58% B in 7 min; 254;220 nm; RT1:6.28min) to afford the title compound (30.6 mg). LCMS (ES, m/z): [M+H]⁺ 558.2; ¹H-NMR (300 MHz, DMSO-d₆, *ppm*) δ 9.33 (s, 1H), 8.98 (s, 1H), 8.48 (s, 1H), 8.15 (dd, 1H), 7.42-7.77 (m, 2H), 7.18 (s, 1H), 6.89 (d, 1H), 6.68 (dd, 1H), 3.76-3.78 (m, 4H), 3.58-3.59 (m, 4H), 1.43 (dd, 2H), 1.31 (dd, 2H).

### Example 94

### Synthesis of 1-[({1-[5-(difluoromethyl)(1,3,4-thiadiazol-2-yl)]-4-(4-(3-pyridyl)piperazinyl)-1H-indazol-6-yl}sulfonyl)amino]cyclopropanecarbonitrile

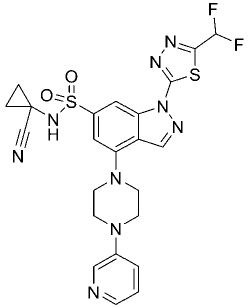

The title compound was prepared as described in Example 93 above by replacing 1-(pyridin-2-yl)piperazine with 1-(pyridin-3-yl)piperazine hydrochloride salt. LCMS (ES, m/z): [M+H]⁺:558; ¹H-NMR (300 MHz, DMSO-*d*₆, *ppm*) δ 9.35 (brs, 1H), 8.98 (s, 1H), 8.50 (s, 1H), 8.37 (d, 1H), 8.03 (d, 1H), 7.39-7.77 (m, 2H), 7.19-7.28 (m, 2H), 3.40-3.65 (m, 8H), 1.39 (dd, 2H), 1.27 (dd, 2H).

### Example 95

### Synthesis of 1-[({1-[5-(difluoromethyl)(1,3,4-thiadiazol-2-yl)]-4-(4-pyridazin-3-ylpiperazinyl)-1H-indazol-6-yl}sulfonyl)amino]cyclopropanecarbonitrile

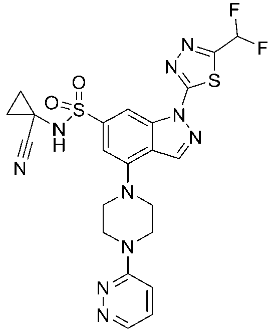

The title compound was prepared as described in Example 93 above by replacing 1-(pyridin-2-yl)piperazine with 3-(piperazin-1-yl)pyridazine hydrochloride salt. LCMS (ES, m/z): [M+H]⁺ 559; ¹H-NMR (300 MHz, DMSO-d₆, *ppm*) δ 9.34 (s, 1H), 9.03 (s, 1H), 8.62 (d, 1H), 8.52 (s, 1H), 7.62 (obs t, 1H), 7.40 (obs dd, 2H), 7.22 (s, 1H), 3.91 (s, 4H), 3.64 (s, 4H), 1.44 (dd, 2H), 1.33 (dd, 2H).

### Example 96

### Synthesis of1-[({1-[5-(difluoromethyl)(1,3,4-thiadiazol-2-yl)]-4-[4-(phenylcarbonyl)piperazinyl] -1H-indazol-6-yl}sulfonyl)amino]cyclopropanecarbonitrile

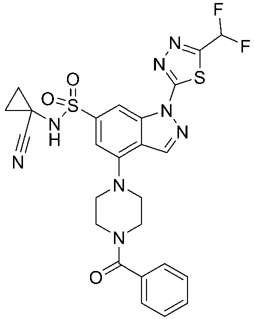

The title compound was prepared as described in Example 93 above by replacing 1-(pyridin-2-yl)piperazine with 1-benzoylpiperazine. LCMS (ES, m/z): [M+H]⁺ 585; ¹H-NMR (400 MHz, DMSO-*d*₆, *ppm*) δ 9.33 (s, 1H), 8.96 (s, 1H), 8.51 (s, 1H), 7.60 (obs t, 1H), 7.49 (s, 5H), 7.18 (s, 1H), 3.51-3.90 (m, 8H), 3.31 (s, 4H), 1.43 (dd, 2H), 1.32 (dd, 2H).

### Example 97

### Synthesis of 1-[({1-(5-methyl(1,3,4-thiadiazol-2-yl))-4-[4-(2-methylpropanoyl)piperazinyl]-1H-indazol-6-yl}sulfonyl)amino]cyclopropanecarbonitrile

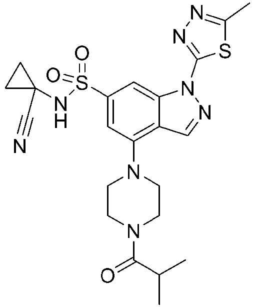

Into a 15-mL sealed tube, were placed 4-chloro-N-(1-cyanocyclopropyl)-1-(5-methyl-1,3,4-thiadiazol-2-yl)-1H-indazole-6-sulfonamide (300.00 mg, 0.696 mmol, 1.00 equiv.), dioxane (4.00 mL), 2-methyl-1-(piperazin-1-yl)propan-1-one TFA salt (265.57 mg, 1.044 mmol, 1.5 equiv.), K₂CO₃ (288.71 mg, 2.089 mmol, 3 equiv.), RuPhos (32.49 mg, 0.070 mmol, 0.1 equiv.), RuPhos Palladacycle Gen.3 (58.24 mg, 0.070 mmol, 0.1 equiv.). The resulting solution was stirred overnight at 90 °C. The reaction was quenched with saturated NH₄Cl (aq.) at room temperature, the aqueous layer was extracted with EtOAc and the combined extracts were concentrated under vacuum. The residue was purified by Prep-TLC (CH₂Cl₂:MeOH=50:1) to afford crude product. The crude product (80 mg) was purified by Prep-HPLC (Column: XBridge Prep OBD C18 Column, 30×150 mm 5 um; Mobile Phase A: water (0.05% NH₃·H₂O), Mobile Phase B:ACN; Flow rate:60 mL/min; Gradient: 17% B to 37% B in 7 min; 254;220 nm; RT: 5.88 min) to afford the title compound (39.2 mg). LCMS (ES, m/z): [M+H] ⁺ 515; ¹H-NMR (300 MHz, DMSO-d₆, *ppm*) δ 9.29 (s, 1H), 8.87 (s, 1H), 8.51 (s, 1H), 7.13 (s, 1H), 3.76-3.79 (m, 4H), 3.45 (s, 4H), 2.96 (p, 1H), 2.76 (s, 3H), 1.44 (dd, 2H), 1.33 (dd, 2H), 1.05 (d, 6H).

### Example 98

### Synthesis of 1-[4-(1-[5-(difluoromethyl)(1,3,4-thiadiazol-2-yl)]-6-{[(methylethyl)amino] sulfonyl}(1H-indazol-4-yl))piperazinyl]-2-methylpropan-1-one

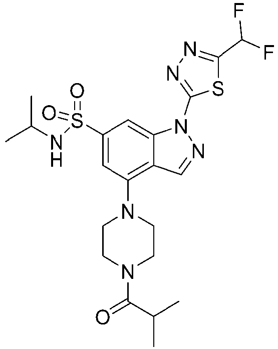

### Step 1: Synthesis of 4-chloro-1-(5-(difluoromethyl)-1,3,4-thiadiazol-2-yl)-N-isopropyl-1H-indazole-6-sulfonamide

Into a 10-mL round-bottom flask, were placed 4-chloro-1-(5-(difluoromethyl)-1,3,4-thiadiazol-2-yl)-1H-indazole-6-sulfonyl chloride (900.00 mg, 2.337 mmol, 1.00 equiv.), propan-2-amine (151.93 mg, 2.570 mmol, 1.1 equiv.), and pyridine (369.65 mg, 4.673 mmol, 2 equiv.). The resulting solution was stirred overnight at room temperature under nitrogen atmosphere and the aqueous layer was extracted with EA. The resulting mixture was concentrated under vacuum and the residue was purified by Prep-TLC (DCM:MeOH=25:1) to afford the title compound (250 mg) as a light yellow solid.

### Step 2: Synthesis of 1-[4-(1-[5-(difluoromethyl)(1,3,4-thiadiazol-2-yl)]-6-{[(methylethyl)amino] sulfonyl}(1H-indazol-4-yl))piperazinyl]-2-methylpropan-1-one

To a stirred solution of 4-chloro-1-(5-(difluoromethyl)-1,3,4-thiadiazol-2-yl)-N-isopropyl-1H-indazole-6-sulfonamide (240.00 mg, 0.588 mmol, 1.00 equiv.) in an 8 mL vial, were added 2-methyl-1-(piperazin-1-yl)propan-1-one TFA salt (299.24 mg, 1.177 mmol, 2 equiv.), RuPhos (219.68 mg, 0.471 mmol, 0.8 equiv.), Cs₂CO₃ (671.07 mg, 2.060 mmol, 3.5 equiv.), RuPhos Palladacycle Gen.3 (196.87 mg, 0.235 mmol, 0.40 equiv.), and dioxane (3.00 mL) under nitrogen atmosphere. The resulting mixture was stirred for overnight at 90 °C under nitrogen atmosphere and then quenched with saturated NH₄Cl (aq.). The aqueous layer was extracted with EA and the combined extracts were concentrated under vacuum. The residue was purified by Prep-TLC (DCM:MeOH=25:1) to afford crude product (200 mg)as a yellow solid. The crude product was purified by Prep-HPLC (Column: XBridge Shield RP18 OBD Column, 30*150 mm, 5 um ; Mobile Phase A: water (10 mmol/L NH₄HCO₃), Mobile Phase B: ACN; Flow rate: 60 mL/min; Gradient: 36 B% to 46% B in 8 min; 254;220 nm; RT: 7.03 min) to afford the title compound (30.7 mg). LCMS (ES, m/z): [M+H]⁺ 528; ¹H-NMR (400 MHz, DMSO-*d*₆, *ppm*) δ 8.93 (s, 1H), 8.45 (s, 1H), 7.83 (s, 1H), 7.60 (t, 1H), 7.17 (s, 1H), 3.77 (d, 4H), 3.45 (d, 4H), 3.30-3.27 (m, 1H), 2.95 (p, 1H), 1.04 (d, 6H), 0.97 (d, 6H).

### Example 99

### Synthesis of 1-[4-(1-[5-(difluoromethyl)(1,3,4-thiadiazol-2-yl)]-6-{[(tert-butyl)amino]sulfonyl} (1H-indazol-4-yl))piperazinyl]-2-methylpropan-1-one

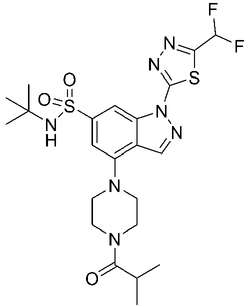

### Step 1: Synthesis of N-(tert-butyl)-4-chloro-1-(5-(difluoromethyl)-1,3,4-thiadiazol-2-yl)-1H-indazole-6-sulfonamide

The title compound N-(tert-butyl)-4-chloro-1-(5-(difluoromethyl)-1,3,4-thiadiazol-2-yl)-1H-indazole-6-sulfonamide was prepared as 4-chloro-1-(5-(difluoromethyl)-1,3,4-thiadiazol-2-yl)-N-isopropyl-1H-indazole-6-sulfonamide by replacing propan-2-amine with tert-butylamine.

### Step 2: Synthesis of 1-[4-(1-[5-(difluoromethyl)(1,3,4-thiadiazol-2-yl)]-6-{[(tert-butyl)amino]-sulfonyl} (1H-indazol-4-yl))piperazinyl]-2-methylpropan-1-one

To a stirred solution of 2-methyl-1-(piperazin-1-yl)propan-1-one TFA salt (492.99 mg, 1.939 mmol, 2.00 equiv.) and N-(tert-butyl)-4-chloro-1-(5-(difluoromethyl)-1,3,4-thiadiazol-2-yl)-1H-indazole-6-sulfonamide (400.00 mg, 0.969 mmol, 1.00 equiv.) in 1,4-dioxane (5 mL) in a 20 mL vial, were added RuPhos (180.96 mg, 0.388 mmol, 0.40 equiv.), RuPhos Palladacycle Gen.3 (324.34 mg, 0.388 mmol, 0.40 equiv.) and K₂CO₃ (468.96 mg, 3.393 mmol, 3.50 equiv.) under nitrogen atmosphere. The resulting mixture was stirred overnight at 90 °C under nitrogen atmosphere and then quenched with saturated NH₄Cl (aq.) , extracted with EtOAc and the combined extracts were concentrated under vacuum. The residue was purified by Prep-TLC (CH₂Cl₂/MeOH=25/1) to provide crude product (230 mg). The crude product was taken up in MeOH to provide a precipitate which was collected by filtration and then dried under vacuum to afford the title compound (189.7 mg). LCMS (ES, m/z): [M+H]⁺ 542; ¹H-NMR (400 MHz, DMSO-d₆, *ppm*) δ 8.92 (s, 1H), 8.48 (s, 1H), 7.78 (s, 1H), 7.60 (m, 1H), 7.22 (d, 1H), 3.77 (dd, 4H), 3.44 (d, 4H), 3.46-3.42 (d, 4H), 2.95 (p, 1H), 1.11 (s, 9H), 1.05 (d, 6H).

### Example 100

### Synthesis of 1-[({1-[5-(difluoromethyl)(1,3,4-thiadiazol-2-yl)]-4-morpholin-4-yl-1H-indazol-6-yl}sulfonyl)amino]cyclopropanecarbonitrile

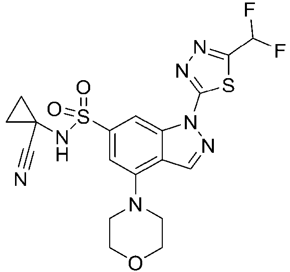

Into a 20-mL sealed tube purged and maintained with an inert atmosphere of nitrogen, were placed 4-chloro-N-(1-cyanocyclopropyl)-1-[5-(difluoromethyl)-1,3,4-thiadiazol-2-yl]-1H-indazole-6-sulfonamide (500 mg, 1.161 mmol, 1 equiv.), t-BuONa (278.83 mg, 2.901 mmol, 2.50 equiv.), t-BuOH (5 mL), morpholine (1011.09 mg, 11.606 mmol, 10.00 equiv.), and t-BuXPhos-Pd G3 (86.73 mg, 0.116 mmol, 0.10 equiv.). The resulting solution was stirred for 1 overnight at 80 °C and the resulting solution was diluted with saturated NH₄Cl (aq.). The resulting solution was extracted with DCM and the DCM extracts were washed with water and then concentrated under vacuum. The resulting residue (50 mg) was purified by Prep-TLC (DCM:MeOH=60:1) to afford the title compound (12.5 mg). LCMS (ES, m/z): [M+H] ⁺ 482; ¹H-NMR (300 MHz, DMSO-d₆, *ppm*) δ 9.35 (s, 1H), 8.99 (d, 1H), 8.52 (s, 1H), 7.61 (t, 1H), 7.18 (d, 1H), 3.87 (t, 4H), 3.41 (t, 4H), 1.44 (dd, 2H), 1.32 (dd, 2H).

### Example 101

### Synthesis of 1-[({1-[5-(difluoromethyl)(1,3,4-thiadiazol-2-yl)]-4-pyrrolidinyl-1H-indazol-6-yl}s ulfonyl)amino]cyclopropanecarbonitrile

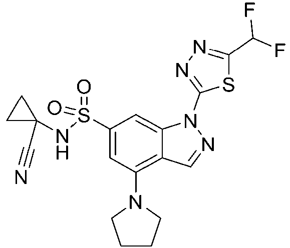

Into a 20-mL sealed tube purged and maintained with an inert atmosphere of nitrogen, were placed 4-chloro-N-(1-cyanocyclopropyl)-1-[5-(difluoromethyl)-1,3,4-thiadiazol-2-yl]-1H-indazole-6-sulfonamide (500 mg, 1.161 mmol, 1 equiv.), t-BuONa (278.83 mg, 2.901 mmol, 2.5 equiv.), t-BuOH (5 mL), pyrrolidine (412.71 mg, 5.803 mmol, 5 equiv.), and tBuXPhos Pd G3 (92.19 mg, 0.116 mmol, 0.1 equiv.). The resulting solution was stirred for 1 overnight at 80 °C. The resulting mixture was concentrated under vacuum and the residue was purified by Prep-TLC (CH₂Cl₂:MeOH=20:1) to afford crude product. The crude product (20 mg) was purified by Prep-HPLC (Column: SunFire C18 OBD Prep Column, 100Å, 5 µm, 19x250 mm; Mobile Phase A: water (1% acetic acid), Mobile Phase B:ACN; Flow rate:20 mL/min; Gradient:60% B to 74% B in 7 min; 254;220 nm; RT: 6.95 min) to afford the title compound (1.4 mg). LCMS (ES, m/z): [M+H] ⁺ 466; ¹H-NMR (300 MHz, DMSO-d₆, *ppm*) δ 8.74 (s, 1H), 8.04 (s, 1H), 7.59 (t, 1H), 6.72 (s, 1H), 3.20-3.80 (H₂O obs peak), 2.05 (s, 4H), 1.71 (s, 5H), 0.78-1.04 (m, 4H).

### Example 102

### Synthesis of 1-[({1-[5-(difluoromethyl)(1,3,4-thiadiazol-2-yl)]-4-(4-methylpiperazinyl)-1H-indazol-6-yl}sulfonyl)amino]cyclopropanecarbonitrile

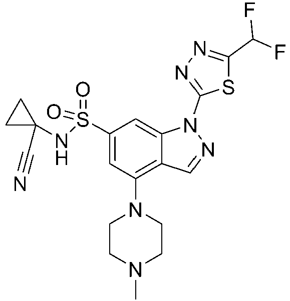

A solution of 4-chloro-N-(1-cyanocyclopropyl)-1-[5-(difluoromethyl)-1,3,4-thiadiazol-2-yl]indazole-6-sulfonamide (500.00 mg, 1.161 mmol, 1.00 equiv.), 1-methylpiperazine (581.23 mg, 5.803 mmol, 5 equiv.), t-BuONa (278.83 mg, 2.901 mmol, 2.5 equiv.) and t-BuXPhos Pd G3 (92.19 mg, 0.116 mmol, 0.1 equiv.) in t-BuOH (5.00 mL) in a 25 mL 3-necked round-bottom flask was stirred for overnight at 80 °C under nitrogen atmosphere. The resulting mixture was concentrated under vacuum and the residue was then purified by Prep-TLC (CH₂Cl₂: MeOH=20:1) to afford crude product (20 mg). The crude product was purified by Prep-HPLC (Column: XBridge Prep OBD C18 Column 30×150 mm 5 um; Mobile Phase A: water(0.05% NH₃·H₂O), Mobile Phase B: ACN; Flow rate: 60 mL/min; Gradient: 20% B to 40% B in 7 min; 254;220 nm; Rt: 6.22 min) to afford the title compound (4.5 mg). LCMS (ES, m/z): [M+H] ⁺ 495; ¹H-NMR (400 MHz, DMSO-d₆, *ppm*) δ 9.44 (brs, 1H), 9.00(s, 1H), 8.55 (s, 1H), 7.62 (t, 1H), 7.23 (s, 1H), 3.62 (brs, 4H), 3.10 (brs, 4H), 2.62 (brs, 3H), 1.45 (dd, 2H), 1.33 (dd, 2H).

### Example 103

### Synthesis of (S)-N-(1-cyanocyclopropyl)-1-(5-(difluoromethyl)-1,3,4-thiadiazol-2-yl)-4-(2-methylmorpholino)-1H-indazole-6-sulfonamide

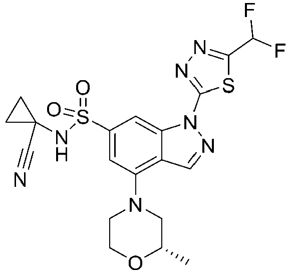

Into a 15-mL sealed tube, were placed 4-chloro-N-(1-cyanocyclopropyl)-1-[5-(difluoromethyl)-1,3,4-thiadiazol-2-yl]-1H-indazole-6-sulfonamide (500.00 mg, 1.161 mmol, 1.00 equiv.), (2S)-2-methylmorpholine (234.78 mg, 2.321 mmol, 2.00 equiv.), t-BuOH (5.00 mL), t-BuONa (278.83 mg, 2.901 mmol, 2.50 equiv.), and t-BuXPhos-Pd-G3 (129.01 mg, 0.162 mmol, 0.14 equiv.) under nitrogen atmosphere. The resulting solution was stirred for 1 overnight at 100 °C under nitrogen atmosphere and then quenched with saturated NH₄Cl (aq.). The resulting mixture was extracted with EtOAc and the combined organic layer was concentrated under reduced pressure. The residue was purified by Prep-TLC (CH₂Cl₂:MeOH=40:1) to afford the crude products. The crude product (20 mg) was purified by Prep-HPLC (Column: Xbridge Prep OBD C18 Column 19*250 mm,5 um; mobile phase A: water(10 mmol/L NH₄HCO₃), mobile Phase B: ACN; flow rate: 20 mL/min; gradient: 51% B to 61% B in 8 min; 254;220 nm; rt: 7.77 min) to afford the title compound (1.6 mg). LCMS (ES, m/z): [M+H]⁺ 496; ¹H-NMR (300MHz, DMSO-d₆, *ppm*) δ 9.35 (s, 1H), 9.00 (s, 1H), 8.52(s, 1H), 7.61 (t, 1H), 7.17 (d, 1H), 4.00 (d, 1H), 3.63-3.84 (m, 4H), 3.05 (t, 1H), 2.74 (t, 1H), 1.45 (dd, 2H), 1.33 (dd, 2H), 1.21 (d, 3H).

### Example 104

### Synthesis of 1-[({1-[5-(difluoromethyl)(1,3,4-thiadiazol-2-yl)]-4-(4-fluoropiperidyl)-1H-indazol-6-yl}sulfonyl)amino]cyclopropanecarbonitrile

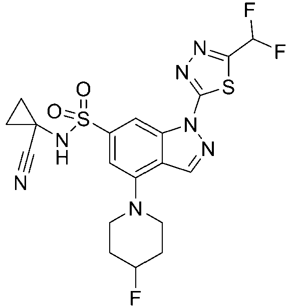

The title compound was prepared as described in Example 103 above by replacing (2S)-2-methylmorpholine with 4-fluoropiperidine hydrochloride salt. LCMS (ES, m/z): [M+H]⁺ 498; ¹H-NMR (300MHz, DMSO-d₆, *ppm*) δ 9.35 (brs, 1H), 8.95(s, 1H), 8.49(s, 1H), 7.62 (t, 1H), 7.20 (s, 1H), 4.86-5.23 (m, 1H), 3.52-3.67 (m, 2H), 3.41-3.51 (m, 3H), 2.08-2.29 (m, 2H), 1.88-2.0 2(m, 2H), 1.44(dd, 2H), 1.34 (dd, 2H).

### Example 105

### Synthesis of 1-[({1-[5-(difluoromethyl)(1,3,4-thiadiazol-2-yl)]-4-(4,4-difluoropiperidyl)-1H-indazol-6-yl}sulfonyl)amino]cyclopropanecarbonitrile

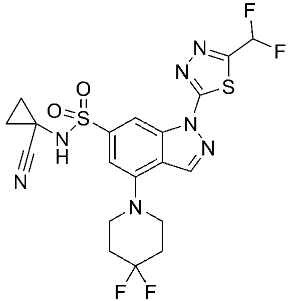

The title compound was prepared as described in Example 103 above by replacing (2S)-2-methylmorpholine with 4,4-difluoropiperidine. LCMS (ES, m/z): [M+H]⁺ 516; ¹H-NMR (300 MHz, DMSO-d₆, *ppm*) δ 9.39 (s, 1H), 8.99 (s, 1H), 8.54 (s, 1H), 7.62 (t, 1H), 7.24 (s, 1H), 3.59 (t, 4H), 2.21-2.30 (m, 4H), 1.44-1.48 (m, 2H), 1.32-1.37 (m, 2H).

### Example 106

### Synthesis of 1-({[4-((3R)-4-{[1-(2,2-difluoroethyl)(4-piperidyl)]carbonyl}-3-methyl piperazinyl)-1-[5-(difluoromethyl)(1,3,4-thiadiazol-2-yl)]-1H-indazol-6-yl]sulfonyl}amino) cyclopropanecarbonitrile

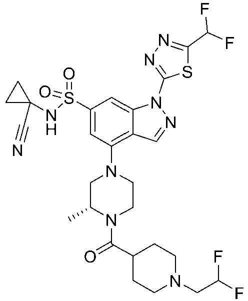

### Step 1: Synthesis of benzyl (R)-4-(1-(tert-butoxycarbonyl)piperidine-4-carbonyl)-3-methylpiperazine-1-carboxylate

Into a 100-mL round-bottom flask purged and maintained with an inert atmosphe -re of nitrogen, was placed benzyl (3R)-3-methylpiperazine-1-carboxylate (1.00 g, 4.268 mmol, 1.00 equiv.), 1-(tert-butoxycarbonyl)piperidine-4-carboxylic acid (0.98 g, 4.268 mmol, 1 equiv.), EDCI (1.23 g, 6.402 mmol, 1.5 equiv.), HOBT (0.87 g, 6.402 mmol, 1.5 equiv.), DIEA (1.65 g, 12.804 mmol, 3 equiv.), DMF (10.00 mL). The resulting solution was stirred for 12 h at room temperature and then quenched with water. The resulting solution was extracted with ethyl acetate, and the organic layer was washed with brine, dried over anhydrous sodium sulfate and concentrated. The crude product was purified by Flash-Prep-HPLC (IntelFlash-1: Column 18, mobile phase, CH₃CN:H₂O=1:1 increasing to CH₃CN:H₂O=2:1 within 30 min.) to afford the title compound (1.7 g).

### Step 2: Synthesis of benzyl (R)-3-methyl-4-(piperidine-4-carbonyl)piperazine-1-carboxylate

Into a 100-mL round-bottom flask purged and maintained with an inert atmosphere of nitrogen, were placed benzyl (R)-4-(1-(tert-butoxycarbonyl)piperidine-4-carbonyl)-3-methylpiperazine-1-carboxylate (1.65 g, 3.815 mmol, 1.00 equiv.), TFA (5.00 mL), and DCM (20.00 mL). The resulting solution was stirred for 12 h at room temperature and then concentrated to provide the title compound (2.4 g).

### Step 3: Synthesis of benzyl (R)-4-(1-(2,2-difluoroethyl)piperidine-4-carbonyl)-3-methylpiperazine-1-carboxylate

Into a 100-mL round-bottom flask purged and maintained with an inert atmosphere of nitrogen, were placed benzyl (R)-3-methyl-4-(piperidine-4-carbonyl)piperazine-1-carboxylate (2.35 g, 6.658 mmol, 1.00 equiv.), 1,1-difluoro-2-iodoethane (2.56 g, 13.316 mmol, 2 equiv.), K₂CO₃ (2.76 g, 19.970 mmol, 3.00 equiv.), and DMF (30.00 mL). The resulting solution was stirred for 12 h at 80 °C in an oil bath and then quenched with water. The resulting solution was extracted with ethyl acetate and the organic layer was washed with brine, dried over anhydrous sodium sulfate and concentrated. The residue was applied onto a silica gel column, eluted with ethyl acetate/ petroleum ether (1/3) to provide the title compound (1 g).

### Step 4: Synthesis of (R)-(1-(2,2-difluoroethyl)piperidin-4-yl)(2-methylpiperazin-1-yl)methanone

Into a 100-mL round-bottom flask purged and maintained with an inert atmosphere of nitrogen, were placed benzyl (R)-4-(1-(2,2-difluoroethyl)piperidine-4-carbonyl)-3-methylpiperazine-1-carboxylate (1 g, 2.442 mmol, 1.00 equiv.), Pd/C (200.00 mg, 1.879 mmol, 0.77 equiv.), and THF (20 mL). The resulting solution was stirred for 12 h at room temperature under hydrogen atmosphere. The solids were filtered out and the resulting mixture was concentrated to provide the title compound (560 mg) (83%) as a colorless oil.

### Step 5: Synthesis of 1-({[4-((3R)-4-{[1-(2,2-difluoroethyl)(4-piperidyl)]carbonyl}-3-methylpiperazinyl)-1-[5-(difluoromethyl)(1,3,4-thiadiazol-2-yl)]-1H-indazol-6-yl]sulfonyl}-amino)cyclopropanecarbonitrile

Into a 25-mL round-bottom flask purged and maintained with an inert atmosphere of nitrogen, were placed 4-chloro-N-(1-cyanocyclopropyl)-1-[5-(difluoromethyl)-1,3,4-thiadiazol-2-yl]indazole-6-sulfonamide (400.00 mg, 0.928 mmol, 1.00 equiv.), PH-IDE-B-0489-4 (383.46 mg, 1.393 mmol, 1.50 equiv.), RuPhos (173.30 mg, 0.371 mmol, 0.40 equiv.), Cs₂CO₃ (907.51 mg, 2.785 mmol, 3.00 equiv.), dioxane (5.00 mL), and RuPhos Palladacycle Gen.3 (310.61 mg, 0.371 mmol, 0.4 equiv.). The resulting solution was stirred for 7 h at 90 °C in an oil bath and then concentrated. The residue was applied onto a silica gel column with dichloromethane/methanol (15/1). The crude product (150 mg) was purified by Prep-HPLC (Column: XBridge Prep OBD C18 Column, 30×150 mm 5 um; Mobile Phase A: water (10 mmol/L NH₄HCO₃+0.1%NH₃·H₂O), Mobile Phase B: ACN; Flow rate: 60 mL/min; Gradient: 38% B to 50% B in 7 min; 254;220 nm; RT: 7.05 min.) to afford the title compound (54.8 mg). LCMS (ES, *m*/*z*): [M+H]⁺ 670; ¹H-NMR (400 MHz, DMSO-d₆, *ppm*) δ 9.30 (s, 1H), 8.85 (s, 1H), 8.47 (s, 1H), 7.60 (t, 1H), 7.13 (s, 1H), 6.12 (tt, 1H), 4.69 (s, 1H), 4.45-4.23 (m, 1H), 3.96-3.65 (m, 3H), 3.34-3.20 (m, 1H), 3.15-2.98 (m, 1H), 2.92 (d, 2H), 2.72 (td, 2H), 2.61-2.50 (m, 1H), 2.26-2.20 (m, 2H), 1.63-1.50 (m, 4H), 1.46-1.37 (m, 3H), 1.33-1.25 (m, 4H).

### Example 107

### Synthesis of 1-({[4-(4-{[1-(2,2-difluoroethyl)(4-piperidyl)]carbonyl}piperazinyl)-1-[5-(difluoromethyl)(1,3,4-thiadiazol-2-yl)]-1H-indazol-6-yl]sulfonyl}amino)cyclopropane carbonitrile

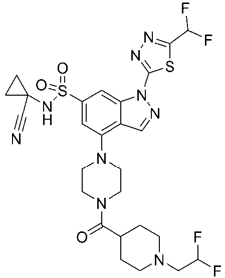

The title compound was prepared as described in Example 106 above by replacing benzyl (3R)-3-methylpiperazine-1-carboxylate in Step 1 with benzyl piperazine-1-carboxylate. LCMS (ES, m/z): [M+H]⁺ 656; ¹H-NMR (400 MHz, DMSO-d₆, *ppm*) δ 9.23 (brs, 1H), 8.96 (s, 1H), 8.50 (s, 1H), 7.60 (t, 1H), 7.17 (s, 1H), 6.12 (tt, 1H), 3.77 (d, 4H), 3.46 (brs, 4H), 2.93 (d, 2H), 2.61-2.76 (m, 3H), 2.24 (td, 2H), 1.59-1.65 (m, 4H), 1.43(dd, 2H), 1.31 (dd, 2H).

### Example 108

### Synthesis of 1-{[(4-{(3R)-3-methyl-4-[(methylcyclopropyl)carbonyl]piperazinyl}-1-[5-(difluoromethyl)(1,3,4-thiadiazol-2-yl)]-1H-indazol-6-yl)sulfonyl]amino}cyclopropane carbonitrile

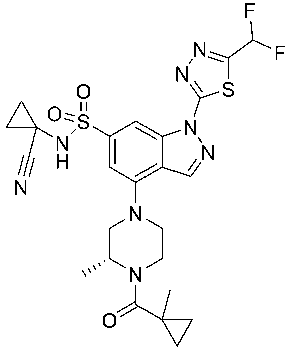

To a stirred mixture of (R)-(1-methylcyclopropyl)(2-methylpiperazin-1-yl)methanone TFA salt (400.00 mg, 1.427 mmol, 1.00 equiv.) and 4-chloro-N-(1-cyanocyclopropyl)-1-[5-(difluoromethyl)-1,3,4-thiadiazol-2-yl]indazole-6-sulfonamide (307.42 mg, 0.714 mmol, 0.50 equiv.) in DMF (6 mL) in a 20 mL vial, were added RuPhos (133.19 mg, 0.285 mmol, 0.20 equiv.), RuPhos Palladacycle Gen.3 (238.71 mg, 0.285 mmol, 0.20 equiv.) and Cs₂CO₃ (813.70 mg, 2.497 mmol, 1.75 equiv.) under nitrogen atmosphere. The resulting mixture was stirred overnight at 100 °C under nitrogen atmosphere. The reaction was quenched with saturated NH₄Cl (aq.), extracted with EtOAc and the combined extracts were concentrated under vacuum. The residue was purified by Prep-TLC (CH₂Cl₂:MeOH=25:1) to afford of crude product (100 mg). The crude product) was purified by Prep-HPLC (Column: XBridge Prep OBD C18 Column, 30×150 mm, 5 um; Mobile Phase A: water (10 mmol/L NH₄HCO₃+0.1%NH₃·H₂O), Mobile Phase B: ACN; Flow rate: 60 mL/min; Gradient: 37% B to 47% B in 9 min; 254;220 nm; RT: 8.47 min) to afford the title compound (11.1 mg). LCMS (ES, m/z): [M+H]⁺ 577; ¹H-NMR (400 MHz, DMSO-d₆, *ppm*) δ 9.23 (brs, 1H), 8.86 (s, 1H), 8.49 (s, 1H), 7.60 (t, 1H), 7.17 (s, 1H), 4.65 (s, 1H), 4.25 (d, 1H), 3.86 (d, 1H), 3.73 (d, 1H), 3.57 (brs, 1H), 3.30-3.24 (m, 1H), 3.10 (s, 1H), 1.45-1.43 (m, 2H), 1.35-1.28 (m, 8H), 0.87 (dd, 2H), 0.58 (s, 2H).

### Example 109

### Synthesis of 1-{[(4-{(3R)-3-methyl-4-[(methylcyclobutyl)carbonyl]piperazinyl}-1-[5-(difluoromethyl)(1,3,4-thiadiazol-2-yl)]-1H-indazol-6-yl)sulfonyl]amino}cyclopropane carbonitrile

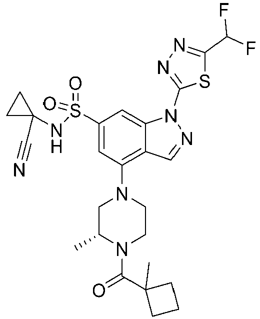

The title compound was prepared as described in Example 108 above by replacing (R)-(1-methylcyclopropyl)(2-methyl piperazin-1-yl)methanone TFA salt with (R)-(1-methylcyclobutyl)(2-methylpiperazin-1-yl)methanone TFA salt. LCMS (ES, m/z): [M+H]⁺ 591; ¹H-NMR (400 MHz, DMSO-d₆, *ppm*) δ 9.25 (brs, 1H), 8.84 (s, 1H), 8.47 (s, 1H), 7.60 (m, 1H), 7.15 (s, 1H), 4.67 (s, 1H), 4.41-4.01 (m, 1H), 3.82 (d, 1H), 3.73 (d, 1H), 3.57 (s, 1H), 3.25-2.85 (m, 2H), 2.50-2.42 (m, 2H), 2.00-1.78 (m, 3H), 1.69-1.60 (m, 1H), 1.50-1.43 (m, 6H), 1.31-1.25 (m, 4H).

### Example 110

### Synthesis of 1-[({4-[(3R)-3-methyl-4-(2-methylbutanoyl)piperazinyl]-1-[5-(difluoromethyl)-(1,3,4-thiadiazol-2-yl)]-1H-indazol-6-yl}sulfonyl)amino]cyclopropane carbonitrile

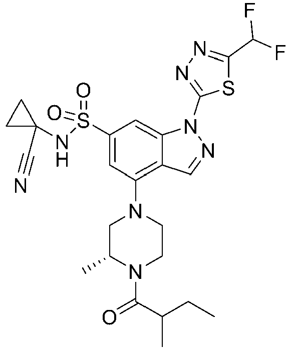

### Step 1: Synthesis of tert-butyl (3R)-3-methyl-4-(2-methyl-butanoyl)piperazine-1-carboxylate

Into a 20-mL round-bottom flask, were placed tert-butyl (3R)-3-methylpiperazine-1-carboxylate (817.08 mg, 4.080 mmol, 1.00 equiv.), 2-methylbutanoic acid (500.00 mg, 4.896 mmol, 1.2 equiv.), EDCI (1173.11 mg, 6.119 mmol, 1.5 equiv.), DIEA (790.90 mg, 6.119 mmol, 1.5 equiv.), HOBT (826.88 mg, 6.119 mmol, 1.5 equiv.), DMF (10.00 mL). The resulting solution was stirred overnight at room temperature under nitrogen atmosphere. The aqueous layer was extracted with EA and the resulting mixture was concentrated under vacuum. The residue was purified by Prep-TLC (DCM:MeOH=25:1) to afford the title compound (1.2 g, 77.46%) as a light yellow solid.

### Step 2: Synthesis of 2-methyl-1-((R)-2-methylpiperazin-1-yl)butan-1-one trifluoroacetate

Into a 20-mL round-bottom flask, was placed tert-butyl (3R)-3-methyl-4-(2-methyl-butanoyl)piperazine-1-carboxylate (1.2 g, 4.219 mmol, 1.00 equiv.), DCM (6.00 mL), TFA (2.00 mL). The resulting solution was stirred for 2 h at room temperature and concentrated under vacuum to give the title compound (1g, 91.58%) as orange oil.

### Step 3: Synthesis of 1-[({4-[(3R)-3-methyl-4-(2-methylbutanoyl)piperazinyl]-1-[5-(difluoromethyl)-(1,3,4-thiadiazol-2-yl)]-1H-indazol-6-yl}sulfonyl)amino]cyclopropane carbonitrile

To a stirred solution of 4-chloro-N-(1-cyanocyclopropyl)-1-[5-(difluoromethyl)-1,3,4-thiadiazol-2-yl]-1H-indazole-6-sulfonamide (381.52 mg, 0.886 mmol, 1.00 equiv.), 2-methyl-1-((R)-2-methylpiperazin-1-yl)butan-1-one trifluoroacetic acid (500 mg, 1.771 mmol, 2.00 equiv.) were added RuPhos (330.58 mg, 0.708 mmol, 0.80 equiv.), Cs₂CO₃ (1009.85 mg, 3.099 mmol, 3.50 equiv.), dioxane (4.00 mL), and RuPhos Palladacycle Gen.3 (296.26 mg, 0.354 mmol, 0.40 equiv.) in an 8 mL vial under nitrogen atmosphere. The resulting mixture was stirred for overnight at 100 °C under nitrogen atmosphere. The reaction mixture was quenched by the addition of saturated NH₄Cl(aq.), extracted with EA and the combined extracts were concentrated under vacuum. The residue was purified by Prep-TLC (DCM:MeOH=25:1) to afford 30 mg crude product as a yellow solid. The crude product was purified by Prep-HPLC (Column: XBridge Shield RP18 OBD Column, 30*150 mm, 5 um; Mobile Phase A: water (10 mmol/L NH₄HCO₃), Mobile Phase B:ACN; Flow rate: 60 mL/min; Gradient: 36% B to 46% B in 8 min; 254;220 nm; RT: 7.03 min.) to afford the title compound (2.4 mg). LCMS (ES, m/z): [M+H]⁺ 579; ¹H-NMR (300 MHz, DMSO-*d*₆, *ppm*) δ 8.87 (s, 1H), 8.47 (s, 1H), 7.61 (t, 1H), 7.15 (s, 1H), 4.73 (s, 1H), 4.20-4.55 (m, 1H), 3.60-4.10 (m, 3H), 3.00-3.25 (m, 1H), 2.68-2.82 (m, 1H), 1.51-1.68 (m, 1H), 1.35-1.49 (m, 4H), 1.22-1.33 (m, 5H), 1.04 (d, 3H), 0.86 (t, 3H).

### Example 111

### Synthesis of1-{[(1-[5-(difluoromethyl)(1,3,4-thiadiazol-2-yl)]-4-{4-[(methylcyclopropyl) carbonyl]piperazinyl}-1H-indazol-6-yl)sulfonyl]amino}cyclopropanecarbonitrile

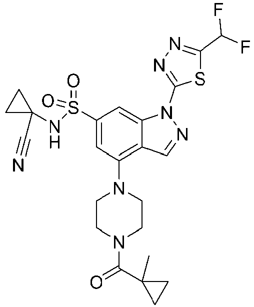

The title compound was prepared as described in Example 108 above by replacing (R)-(1-methylcyclopropyl)(2-methylpiperazin-1-yl)methanone TFA salt with (1-methylcyclopropyl)-(piperazin-1-yl)methanone TFA salt. LCMS (ES, m/z): [M+H]⁺ 563; ¹H-NMR (400 MHz, DMSO-d₆, *ppm*) δ 9.30 (s, 1H), 8.98 (s, 1H), 8.51 (s, 1H), 7.60 (t, 1H), 7.19 (s, 1H), 3.82 (s, 4H), 3.48 (s, 4H), 1.44 (dd, 2H), 1.38-1.29 (m, 5H), 0.87 (t, 2H), 0.59 (t, 2H).

### Example 112

### Synthesis of 1-{[(1-[5-(difluoromethyl)(1,3,4-thiadiazol-2-yl)]-4-{4-[(methylcyclobutyl) carbonyl]piperazinyl}-1H-indazol-6-yl)sulfonyl]amino}cyclopropanecarbonitrile

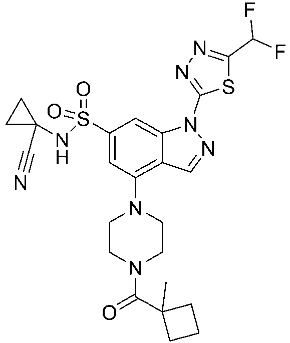

The title compound was prepared as described in Example 108 above by replacing (R)-(1-methylcyclopropyl)(2-methyl piperazin-1-yl)methanone TFA salt with (1-methylcyclobutyl)-(piperazin-1-yl)methanone TFA salt. LCMS (ES, m/z): [M+H]+ 577; ¹H-NMR (300 MHz, DMSO-d₆, ppm) δ 8.88 (s, 1H), 8.46 (s, 1H), 7.56 (t, 1H), 7.16 (s, 1H), 3.62-3.56 (m, 3H), 3.55-3.50 (m, 1H), 3.39 (s, 4H), 2.46-2.41 (m, 2H), 2.06-1.82 (m, 3H), 1.70-1.60 (m, 1H), 1.41 (s, 3H), 1.22-1.18 (m, 2H), 1.15-1.10 (m, 2H).

### Example 113

### Synthesis of 1-[({1-[5-(difluoromethyl)(1,3,4-thiadiazol-2-yl)]-4-[4-(2-methylbutanoyl) piperazinyl]-1H-indazol-6-yl}sulfonyl)amino]cyclopropanecarbonitrile

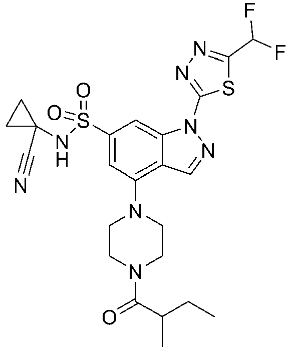

The title compound was prepared as described in Example 108 above by replacing (R)-(1-methyl-cyclopropyl)(2-methyl piperazin-1-yl)methanone TFA salt with 2-methyl-1-(piperazin-1-yl)butan-1-one TFA salt. LCMS (ES, m/z): [M+H]⁺ 565; ¹H-NMR (300 MHz, DMSO-*d*₆, *ppm*) δ 9.31 (s, 1H), 8.98 (s, 1H), 8.51 (s, 1H), 7.61 (t, 1H), 7.17 (d, 1H), 3.78-3.82 (m, 4H), 3.48 (s, 4H), 2.79 (q, 1H), 1.62 (spt, 1H), 1.44 (dd, 2H), 1.25-1.42 (m, 3H), 1.03 (d, 3H), 0.87 (t, 3H).

### Example 114

### Synthesis of 1-[({1-[5-(difluoromethyl)(1,3,4-thiadiazol-2-yl)]-3-chloro-4-(2-oxa-7-aza-spiro[3.5]non-7-yl)-1H-indazol-6-yl}sulfonyl)amino]cyclopropanecarbonitrile

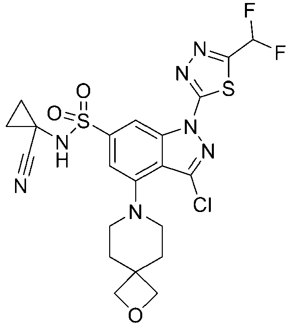

Into a 20-mL vial, were placed 1-[({1-[5-(difluoromethyl)(1,3,4-thiadiazol-2-yl)]-4-(2-oxa-7-azaspiro[3.5]non-7-yl)-1H-indazol-6-yl}sulfonyl)amino]cyclopropanecarbonitrile (200.00 mg, 0.383 mmol, 1.00 equiv.), DMF (4.00 mL), and NCS (76.81 mg, 0.575 mmol, 1.50 equiv.). The resulting solution was stirred overnight at room temperature and then washed with H₂O. The resulting solution was extracted with ethyl acetate, the combined extracts were concentrated under vacuum and the residue was purified by Prep-TLC (CH₂Cl₂:MeOH=50:1) to provide crude product. The crude product was purified by Prep-HPLC (Column: Atlantis HILIC OBD Column 19*150 mm 5 um; Mobile Phase A: water (0.05%TFA ), Mobile Phase B: ACN; Flow rate: 60 mL/min; Gradient: 56% B to 58% B in 7 min; 254/220 nm; Rt: 6.2 min) to afford the title compound (8.6 mg). LCMS (ES, m/z): [M+H]⁺ =556; ¹H-NMR (300 MHz, DMSO-d₆, *ppm*) δ 9.67 (s, 1H), 9.05 (s, 1H), 8.92 (s, 1H), 7.61 (t, 1H), 4.43 (s, 4H), 3.31 (s, 4H), 2.02-2.09 (m, 4H), 1.25-1.46 (m, 4H).

### Example 115

### Synthesis of [4-(1-[5-(difluoromethyl)(1,3,4-thiadiazol-2-yl)]-3-chloro-6-{[(cyanocyclopropyl)-amino]sulfonyl}(1H-indazol-4-yl))piperazinyl]-N,N-dimethylcarboxamide

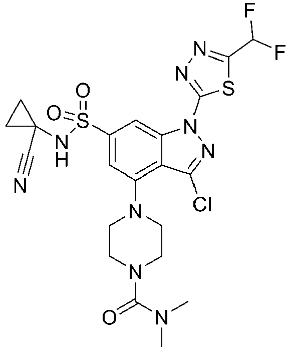

Into a 20-mL vial, were placed [4-(1-[5-(difluoromethyl)(1,3,4-thiadiazol-2-yl)]-6-{[(cyanocyclopropyl)amino]sulfonyl}(1H-indazol-4-yl))piperazinyl]-N,N-dimethyl carboxamide (300.00 mg, 0.544 mmol, 1.00 equiv.), DMF (5.00 mL), and NCS (145.25 mg, 1.088 mmol, 2.00 equiv.). The resulting solution was stirred overnight at room temperature and then washed with H₂O. The resulting solution was extracted with ethyl and the combined extracts were concentrated under vacuum. The crude product was crystallized from DCM/PE (1/5, 3 mL) and then purified by Prep-HPLC (Column: XBridge Prep OBD C18 Column, 30×150 mm 5 um; Mobile Phase A: water (10 mmol/L NH₄HCO₃), Mobile Phase B: ACN; Flow rate: 60 mL/min; Gradient: 30% B to 55% B in 7 min; 254; 220 nm; RT: 6.7 min.) to afford the title compound (79.9 mg). LCMS (ES, m/z): [M+H]⁺ 586; ¹H-NMR (300 MHz, DMSO-d₆, *ppm*) δ 9.61 (s, 1H), 9.05 (s, 1H), 8.96 (s, 1H), 7.61 (t, 1H), 3.44-3.31 (m, 8H), 2.81 (s, 6H), 1.45 (t, 2H), 1.29 (t, 2H).

### Biological Examples

### Example 1

### Inhibition of PARG enzymatic assay

### Enzymatic IC₅₀ Assay

PARG enzyme was incubated with compound or vehicle (DMSO) and the biotinylated-PARylated PARP substrate in a microtiter plate. After adding detection antibody and streptavidin-europium, and then incubating, the plate was read for fluorescence intensity. The low control (DMSO) with low fluorescence intensity represents no inhibition of enzymatic reaction while the high control (no enzyme) with high fluorescence intensity represents full inhibition of enzymatic reaction.

### Materials:

Enzyme:
• PARG

| | | |
|---|---|---|
| ∘ | hPARG: | 250 pM, His-tagged, Peak Proteins, 0.96 mg/mL (8.58 µM) |
| ∘ | Substrate: | 30 nM |
| ∘ | Reaction time: | 70 minutes |

| | |
|---|---|
| Substrate: | hPARP1,His- and TEV-tagged, Peak Proteins, 14 µM |
| Detection Antibody: | anti-His monoclonal antibody-ULight, Perkin Elmer catalog # TRF0105-M |
| Streptavidin-Europium: | Perkin Elmer catalog # AD0062 |
| Assay buffer : | 50 mM Tris-HCl pH 7.4, 50 mM KCl, 3 mM EDTA, 0.4 mM EGTA, 1 mM DTT, 0.01% Tween 20, 0.01% BSA |
| Temperature: | 23°C |
| Total volume: | 20 µL |

Controls:

| | | |
|---|---|---|
| • | 0% inhibition control: | DMSO |
| • | 100% inhibition control: | No enzyme |

### Enzyme Reaction and Detection:

1. Transfer 5 µL of 3 x final concentration of test compounds or DMSO to the appropriate wells of a microtiter plate.
2. Centrifuge the plate at 1000 rpm for 1 minute.
3. Transfer 5 µL of 3 x final concentration of enzyme in assay buffer or assay buffer alone to the appropriate wells.
4. Centrifuge the plate at 1000 rpm for 1 minute.
5. Incubate the plate at room temperature for 1 hour.
6. Transfer 5 µL of 3 x substrate in assay buffer to all the test wells.
7. Centrifuge the plate at 1000 rpm for 1 minute.
8. Incubate the plate at room temperature for 10 minutes.
9. Transfer 5 µL of 3 x mixture of detection antibody and streptavidin-europium in assay buffer to all the test wells.
10. Centrifuge the plate at 1000 rpm for 1 minute.
11. Incubate the plate at room temperature for 1 hour.
12. Read the plate on a plate reader (e.g., Infinite M1000).

The TR-FRET IC₅₀value for compounds of Formula (I) in Examples 1 to 115 are provided in Table 1 below.

### Example 2

### Inhibition of PARG ... cellular assay

The ability of the compounds disclosed herein to inhibit PARG was determined as described below. Briefly, after treating HeLa cells with the compounds of the disclosure for 1 h, followed by treatment with DNA alkylating agent methylmethanesulfonate (MMS) for an additional 1 h, the cells were fixed in 3.7% formaldehyde. The cells were permeabilized in 0.5% Triton-X100, blocked in 5% goat serum, and incubated with mouse monoclonal antibody against poly (ADP) ribose (PAR) polymer overnight at 4 °C. The cells were washed and incubated with an Alexafluor 488-linked secondary antibody together with a nuclear stain (Hoechst 33342) for an hour at room temperature. After washing, images of the cells were captured and analyzed on a high content imaging microscope and software. Two measurement of PAR chain were quantified with the mean intensity over background of the nuclear foci (fluorescent signal at 488 nm range 0.3-2.0 µm foci) quantified as PAR foci (PAR Foci IC₅₀). The cells with a PAR foci signal >1.3 was considered as positive (PAR % positive IC₅₀), and the percentage positive cells are quantified. An increase in PAR foci or percentage positive cells indicates that more PAR chains are present which in turn provides the magnitude of PARG inhibition. The PAR Foci IC₅₀ value for compounds of Formula (I) in Examples 1 to 115 are provided in Table 1 below.

### Example 3

### Cellular viability assay

HCC1806-XRCC1 KD (knock down) cells were plated at 2000 cells/well in 96-well white plates with clear flat bottom. After 24 hours, the compounds of the disclosure were added starting at 30 µM for a 9-point dose response curve at 1:3 dilution. The compounds of the disclosure were added by Tecan digital dispenser excluding the outermost wells of the plate. All treatments were done in duplicates. After 4 days of incubation, 50 ul of Cell Titer-Glo (Promega) was added per well. After incubation with the reagent for 15 minutes, luminescence was read using a plate reader (TECAN). Average values of DMSO treated wells in a plate was calculated. All data points were normalized to that of the average DMSO value. The % of control for each sample compared to DMSO treated control samples. Curves are fit as % of control vs. log [compound concentration] using a 4 parameter inhibition model (Levenberg-Marquardt algorithm): $Fit = \left(A+\left(\left(B-A\right)/\left(1+\left({\left(C/x\right)}^{∧}D\right)\right)\right)\right)$ $Res = \left(y-fit\right)$

The PARGi (HCC1806-shXRCC1) cellular viability for compounds of Formula (I) in Examples 1 to 115 are provided in Table 1 below.

**Table 1**

| TR-FRET and PAR Foci Assay | | | |
|---|---|---|---|
| **** <= 0.1 µM, 0.1 µM < *** <= 0.3 µM, 0.3 µM < ** <= 1 µM, 1 µM < * < 10 µM | | | |
| HCC1806-shXRCC1 PARGi viability | | | |
| **** < 1 µM, 1 µM <= *** <10 µM, 10 µM <= ** <20 µM, 20 µM <= * <= 30 µM | | | |
| Example Number | TR-FRET IC₅₀ (uM) | PAR Foci IC₅₀ (uM) | PARGi cell viability |
| 1 | * | * | * |
| 2 | * | | * |
| 3 | *** | | ** |
| 4 | ** | | * |
| 5 | **** | ** | * |
| 6 | * | * | * |
| 7 | ** | ** | *** |
| 8 | *** | **** | *** |
| 9 | ** | * | |
| 10 | *** | * | |
| 11 | *** | * | |
| 12 | ** | ** | |
| 13 | ** | ** | |
| 14 | ** | * | ** |
| 15 | **** | ** | *** |
| 16 | *** | * | |
| 17 | ** | * | |
| 18 | *** | * | |
| 19 | ** | * | |
| 20 | ** | * | |
| 21 | *** | ** | *** |
| 22 | | ** | |
| 23 | **** | **** | **** |
| 24 | **** | **** | **** |
| 25 | **** | **** | **** |
| 26 | **** | *** | *** |
| 27 | **** | ** | *** |
| 28 | **** | **** | **** |
| 29 | *** | ** | |
| 30 | ** | * | |
| 31 | ** | | ** |
| 32 | **** | **** | **** |
| 33 | **** | **** | *** |
| 34 | ** | | * |
| 35 | **** | **** | **** |
| 36 | **** | | ** |
| 37 | | **** | *** |
| 38 | **** | **** | **** |
| 39 | **** | **** | **** |
| 40 | **** | * | ** |
| 41 | *** | ** | *** |
| 42 | * | | |
| 43 | **** | **** | **** |
| 44 | ** | * | *** |
| 45 | *** | ** | * |
| 46 | *** | * | *** |
| 47 | **** | ** | *** |
| 48 | *** | ** | *** |
| 49 | | ** | *** |
| 50 | **** | ** | *** |
| 51 | *** | ** | ** |
| 52 | **** | ** | *** |
| 53 | *** | | ** |
| 54 | *** | | ** |
| 55 | ** | | |
| 56 | ** | | ** |
| 57 | **** | **** | **** |
| 58 | **** | | *** |
| 59 | **** | ** | *** |
| 60 | **** | *** | *** |
| 61 | **** | | *** |
| 62 | **** | | |
| 63 | **** | | ** |
| 64 | * | | * |
| 65 | ** | | |
| 66 | **** | **** | *** |
| 67 | **** | **** | **** |
| 68 | **** | * | *** |
| 69 | **** | **** | **** |
| 70 | *** | | |
| 71 | **** | *** | *** |
| 72 | **** | *** | *** |
| 73 | *** | * | |
| 74 | **** | | *** |
| 75 | ** | | * |
| 76 | **** | | *** |
| 77 | **** | **** | **** |
| 78 | *** | | |
| 79 | ** | *** | *** |
| 80 | **** | **** | *** |
| 81 | | **** | **** |
| 82 | **** | **** | **** |
| 83 | **** | **** | **** |
| 84 | ** | *** | *** |
| 85 | **** | * | *** |
| 86 | **** | **** | **** |
| 87 | **** | **** | *** |
| 88 | **** | **** | *** |
| 89 | **** | **** | *** |
| 90 | **** | * | *** |
| 91 | * | * | * |
| 92 | ** | | ** |
| 93 | * | ** | *** |
| 94 | ** | ** | *** |
| 95 | **** | *** | *** |
| 96 | **** | **** | *** |
| 97 | **** | *** | *** |
| 98 | *** | * | *** |
| 99 | *** | * | *** |
| 100 | *** | ** | *** |
| 101 | * | * | |
| 102 | *** | ** | |
| 103 | *** | *** | *** |
| 104 | ** | ** | *** |
| 105 | * | ** | |
| 106 | **** | **** | *** |
| 107 | **** | *** | *** |
| 108 | **** | **** | **** |
| 109 | **** | **** | **** |
| 110 | **** | *** | **** |
| 111 | **** | **** | **** |
| 112 | **** | **** | **** |
| 113 | **** | **** | **** |
| 114 | * | ** | ** |
| 115 | ** | ** | ** |

### References

[1] Ame, J. C., E. Fouquerel, L. R. Gauthier, D. Biard, F. D. Boussin, F. Dantzer, G. de Murcia and V. Schreiber (2009). "Radiation-induced mitotic catastrophe in PARG-deficient cells." J Cell Sci 122(Pt 12): 1990-2002.
[2] Barber, L. J., S. Sandhu, L. Chen, J. Campbell, I. Kozarewa, K. Fenwick, I. Assiotis, D. N. Rodrigues, J. S. Reis Filho, V. Moreno, J. Mateo, L. R. Molife, J. De Bono, S. Kaye, C. J. Lord and A. Ashworth (2013). "Secondary mutations in BRCA2 associated with clinical resistance to a PARP inhibitor." J Pathol 229(3): 422-429.
[3] Blenn, C., P. Wyrsch and F. R. Althaus (2011). "The ups and downs of tannins as inhibitors of poly(ADP-ribose)glycohydrolase." Molecules 16(2): 1854-1877.
[4] Caiafa, P., T. Guastafierro and M. Zampieri (2009). "Epigenetics: poly(ADP-ribosyl)ation of PARP-1 regulates genomic methylation patterns." FASEB J 23(3): 672-678.
[5] Curtin, N. J. and C. Szabo (2013). "Therapeutic applications of PARP inhibitors: anticancer therapy and beyond." Mol Aspects Med 34(6): 1217-1256.
[6] Dahl, M., V. Maturi, P. Lonn, P. Papoutsoglou, A. Zieba, M. Vanlandewijck, L. P. van der Heide, Y. Watanabe, O. Soderberg, M. O. Hottiger, C. H. Heldin and A. Moustakas (2014). "Fine-tuning of Smad protein function by poly(ADP-ribose) polymerases and poly(ADP-ribose) glycohydrolase during transforming growth factor beta signaling." PLoS One 9(8): e103651.
[7[ Drost, R. and J. Jonkers (2014). "Opportunities and hurdles in the treatment of BRCA1-related breast cancer." Oncogene 33(29): 3753-3763.
[8] Erdelyi, K., P. Bai, I. Kovacs, E. Szabo, G. Mocsar, A. Kakuk, C. Szabo, P. Gergely and L. Virag (2009). "Dual role of poly(ADP-ribose) glycohydrolase in the regulation of cell death in oxidatively stressed A549 cells." FASEB J 23(10): 3553-3563.
[9] Fathers, C., R. M. Drayton, S. Solovieva and H. E. Bryant (2012). "Inhibition of poly(ADP-ribose) glycohydrolase (PARG) specifically kills BRCA2-deficient tumor cells." Cell Cycle 11(5): 990-997.
[10] Fisher, A. E., H. Hochegger, S. Takeda and K. W. Caldecott (2007). "Poly(ADP-ribose) polymerase 1 accelerates single-strand break repair in concert with poly(ADP-ribose) glycohydrolase." Mol Cell Biol 27(15): 5597-5605.
[11] Frizzell, K. M., M. J. Gamble, J. G. Berrocal, T. Zhang, R. Krishnakumar, Y. Cen, A. A. Sauve and W. L. Kraus (2009). "Global analysis of transcriptional regulation by poly(ADP-ribose) polymerase-1 and poly(ADP-ribose) glycohydrolase in MCF-7 human breast cancer cells." J Biol Chem 284(49): 33926-33938.
[12] Fujihara, H., H. Ogino, D. Maeda, H. Shirai, T. Nozaki, N. Kamada, K. Jishage, S. Tanuma, T. Takato, T. Ochiya, T. Sugimura and M. Masutani (2009). "Poly(ADP-ribose) Glycohydrolase deficiency sensitizes mouse ES cells to DNA damaging agents." Curr Cancer Drug Targets 9(8): 953-962.
[13] Guastafierro, T., A. Catizone, R. Calabrese, M. Zampieri, O. Martella, M. G. Bacalini, A. Reale, M. Di Girolamo, M. Miccheli, D. Farrar, E. Klenova, F. Ciccarone and P. Caiafa (2013). "ADP-ribose polymer depletion leads to nuclear Ctcf re-localization and chromatin rearrangement(1)." Biochem J 449(3): 623-630.
[14] Ji, Y. and A. V. Tulin (2009). "Poly(ADP-ribosyl)ation of heterogeneous nuclear ribonucleoproteins modulates splicing." Nucleic Acids Res 37(11): 3501-3513.
[15] Le May, N., I. Iltis, J. C. Ame, A. Zhovmer, D. Biard, J. M. Egly, V. Schreiber and F. Coin (2012). "Poly (ADP-ribose) glycohydrolase regulates retinoic acid receptor-mediated gene expression." Mol Cell 48(5): 785-798.
[16] Mashimo, M., J. Kato and J. Moss (2014). "Structure and function of the ARH family of ADP-ribosyl-acceptor hydrolases." DNA Repair (Amst).
[17] Mortusewicz, O., E. Fouquerel, J. C. Ame, H. Leonhardt and V. Schreiber (2011). "PARG is recruited to DNA damage sites through poly(ADP-ribose)- and PCNA-dependent mechanisms." Nucleic Acids Res 39(12): 5045-5056.
[18] Nakadate, Y., Y. Kodera, Y. Kitamura, T. Tachibana, T. Tamura and F. Koizumi (2013). "Silencing of poly(ADP-ribose) glycohydrolase sensitizes lung cancer cells to radiation through the abrogation of DNA damage checkpoint." Biochem Biophys Res Commun 441(4): 793-798.
[19] Shirai, H., H. Fujimori, A. Gunji, D. Maeda, T. Hirai, A. R. Poetsch, H. Harada, T. Yoshida, K. Sasai, R. Okayasu and M. Masutani (2013). "Parg deficiency confers radio-sensitization through enhanced cell death in mouse ES cells exposed to various forms of ionizing radiation." Biochem Biophys Res Commun 435(1): 100-106.
[20] Shirai, H., A. R. Poetsch, A. Gunji, D. Maeda, H. Fujimori, H. Fujihara, T. Yoshida, H. Ogino and M. Masutani (2013). "PARG dysfunction enhances DNA double strand break formation in S-phase after alkylation DNA damage and augments different cell death pathways." Cell Death Dis 4: e656.
[21] Sun, Y., T. Zhang, B. Wang, H. Li and P. Li (2012). "Tannic acid, an inhibitor of poly(ADP-ribose) glycohydrolase, sensitizes ovarian carcinoma cells to cisplatin." Anticancer Drugs 23(9): 979-990.
[22] Zhou, Y., X. Feng and D. W. Koh (2010). "Enhanced DNA accessibility and increased DNA damage induced by the absence of poly(ADP-ribose) hydrolysis." Biochemistry 49(34): 7360-7366.
[23] Zhou, Y., X. Feng and D. W. Koh (2011). "Synergistic cytotoxicity of N-methyl-N'-nitro-N-nitrosoguanidine and absence of poly(ADP-ribose) glycohydrolase involves chromatin decondensation." Int J Oncol 39(1): 121-127.

### ASPECTS OF THE INVENTION:

**1**. A compound of Formula (I): 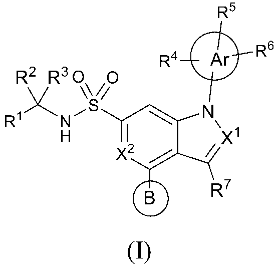 wherein:
   X¹ is selected from the group consisting of N or CR⁸ where R⁸ is hydrogen, halo, C₁₋₂ alkyl, and C₁₋₂ haloalkyl;
   X² is selected from the group consisting of N, CH, and CF;
   R¹ is selected from the group consisting of hydrogen, cyano, formyl, -CONH₂, -CH₂OH, -CH₂OC₁₋₂ alkyl, C₁₋₂ alkyl, and C₁₋₂ haloalkyl;
   R² and R³ are C₁₋₂ alkyl; or
   R² and R³ together with the carbon atom to which they are attached form cyclopropyl;
   Ar is a 5-membered heteroaryl;
   R⁴ is selected from the group consisting of C₁₋₃ alkyl, C₁₋₃ haloalkyl, hydroxyC₁₋₃alkyl, -C(O)H and cyano;
   R⁵ and R⁶ are each independently absent or selected from the group consisting of hydrogen, C₁₋₆ alkyl, hydroxy, C₁₋₆ alkoxy, halo, C₁₋₆ haloalkyl, and C₁₋₆ haloalkoxy;
   R⁷ is selected from the group consisting of hydrogen, deuterium, halo, C₁₋₆ alkyl, and C₁₋₆ haloalkyl; and
   ring B is C₃₋₆ cycloalkyl, phenyl, heteroaryl, heterocyclyl, fused heterocyclyl, spiro heterocyclyl, or bridged heterocyclyl, wherein phenyl, heteroaryl, heterocyclyl, fused heterocyclyl, spiro heterocyclyl, and bridged heterocyclyl of ring B are substituted with R^{a}, R^{b}, and/or R^{c} wherein R^{a} is hydrogen, C₁₋₆ alkyl, hydroxy, C₁₋₆ alkoxy, halo, C₁₋₆ haloalkyl, C₁₋₆ haloalkoxy, hydroxyC₁₋₆ alkyl, heteroaryl, heterocyclyl, -C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is hydrogen or C₁₋₆ alkyl), -C(O)NR^{f}R^{g} (where R^{f} and R^{g} are independently selected from hydrogen, C₁₋₆ alkyl, C₁₋₆ haloalkyl, aminoC₁₋₆ alkyl, and hydroxyC₁₋₆ alkyl; or R^{f} and R^{g} together with the nitrogen atom to which they are attached form heterocyclyl), or -S(O)₂NR^{h}Rⁱ (where R^{h} and Rⁱ are independently selected from hydrogen, C₁₋₆ alkyl, C₁₋₆ haloalkyl, aminoC₁₋₆ alkyl, or hydroxyC₁₋₆ alkyl; or R^{h} and Rⁱ together with the nitrogen atom to which they are attached form heterocyclyl), and R^{b} and R^{c} are independently selected from hydrogen, C₁₋₆ alkyl, hydroxy, C₁₋₆ alkoxy, halo, C₁₋₆ haloalkyl, and C₁₋₆ haloalkoxy; or
   ring B is a five to six membered heterocyclyl substituted with R^{a}, R^{b}, and/or R^{c}, wherein R^{b} and R^{c} are on adjacent ring vertices and are combined to form a four to six membered heterocyclyl having 0 to 2 additional heteroatom ring vertices selected from N, O, and S, and R^{a} is selected from hydrogen, C₁₋₆ alkyl, hydroxy, C₁₋₆ alkoxy, halo, C₁₋₆ haloalkyl, C₁₋₆ haloalkoxy, hydroxyC₁₋₆ alkyl, heteroaryl, heterocyclyl, -C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is hydrogen or C₁₋₆ alkyl), -C(O)NR^{f}R^{g} (where R^{f} and R^{g} are independently selected from hydrogen, C₁₋₆ alkyl, C₁₋₆ haloalkyl, aminoC₁₋₆ alkyl, and hydroxyC₁₋₆ alkyl; or R^{f} and R^{g} together with the nitrogen atom to which they are attached form heterocyclyl), and -S(O)₂NR^{h}Rⁱ (where R^{h} and Rⁱ are independently selected from hydrogen, C₁₋₆ alkyl, C₁₋₆ haloalkyl, aminoC₁₋₆ alkyl, or hydroxyC₁₋₆ alkyl; or R^{h} and Rⁱ together with the nitrogen atom to which they are attached form heterocyclyl); and
   further wherein heteroaryl and heterocyclyl of R^{a}; phenyl, heteroaryl, and heterocyclyl of R^{d}, heterocyclyl formed by R^{f} and R^{g} combining with the nitrogen to which they are attached, and heterocyclyl formed by R^{h} and Rⁱ combining with the nitrogen to which they are attached are each independenly unsubstituted or substituted with one, two, or three substituents selected from C₁₋₆ alkyl, hydroxy, C₁₋₆ alkoxy, halo, C₁₋₆ haloalkyl, and C₁₋₆ haloalkoxy; or
   a pharmaceutically acceptable salt thereof.
**2**. A compound of Formula (I): 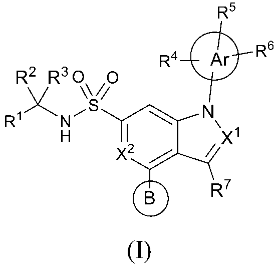 wherein:
   X¹ is selected from the group consisting of N or CR⁸ where R⁸ is hydrogen, halo, C₁₋₂ alkyl, and C₁₋₂ haloalkyl;
   X² is selected from the group consisting of N, CH, and CF;
   R¹ is selected from the group consisting of hydrogen, cyano, formyl, -CONH₂, -CH₂OH, -CH₂OC₁₋₂ alkyl, C₁₋₂ alkyl, and C₁₋₂ haloalkyl;
   R² and R³ are C₁₋₂ alkyl; or
   R² and R³ together with the carbon atom to which they are attached form cyclopropyl;
   Ar is a 5-membered heteroaryl;
   R⁴ is selected from the group consisting of C₁₋₃ alkyl, C₁₋₃ haloalkyl, hydroxyC₁₋₃alkyl, -C(O)H and cyano;
   R⁵ and R⁶ are each independently absent or selected from the group consisting of hydrogen, C₁₋₆ alkyl, hydroxy, C₁₋₆ alkoxy, halo, C₁₋₆ haloalkyl, and C₁₋₆ haloalkoxy;
   R⁷ is selected from the group consisting of hydrogen, deuterium, halo, C₁₋₆ alkyl, and C₁₋₆ haloalkyl; and
   ring B is C₃₋₆ cycloalkyl, phenyl, heteroaryl, heterocyclyl, fused heterocyclyl, spiro heterocyclyl, or bridged heterocyclyl, wherein phenyl, heteroaryl, heterocyclyl, fused heterocyclyl, spiro heterocyclyl, and bridged heterocyclyl of ring B are substituted with R^{a}, R^{b}, and/or R^{c} wherein R^{a} is hydrogen, C₁₋₆ alkyl, hydroxy, C₁₋₆ alkoxy, halo, C₁₋₆ haloalkyl, C₁₋₆ haloalkoxy, hydroxyC₁₋₆ alkyl, heteroaryl, heterocyclyl, -C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is hydrogen or C₁₋₆ alkyl), -C(O)NR^{f}R^{g} (where R^{f} and R^{g} are independently selected from hydrogen, C₁₋₆ alkyl, C₁₋₆ haloalkyl, aminoC₁₋₆ alkyl, and hydroxyC₁₋₆ alkyl; or R^{f} and R^{g} together with the nitrogen atom to which they are attached form heterocyclyl), or -S(O)₂NR^{h}Rⁱ (where R^{h} and Rⁱ are independently selected from hydrogen, C₁₋₆ alkyl, C₁₋₆ haloalkyl, aminoC₁₋₆ alkyl, or hydroxyC₁₋₆ alkyl; or R^{h} and Rⁱ together with the nitrogen atom to which they are attached form heterocyclyl), and R^{b} and R^{c} are independently selected from hydrogen, C₁₋₆ alkyl, hydroxy, C₁₋₆ alkoxy, halo, C₁₋₆ haloalkyl, and C₁₋₆ haloalkoxy; and
   further wherein heteroaryl and heterocyclyl of R^{a}; phenyl, heteroaryl, and heterocyclyl of R^{d}, heterocyclyl formed by R^{f} and R^{g} combining with the nitrogen to which they are attached, and heterocyclyl formed by R^{h} and Rⁱ combining with the nitrogen to which they are attached are each independenly unsubstituted or substituted with one, two, or three substituents selected from C₁₋₆ alkyl, hydroxy, C₁₋₆ alkoxy, halo, C₁₋₆ haloalkyl, and C₁₋₆ haloalkoxy; or
   a pharmaceutically acceptable salt thereof.
**3**. The compound of aspect 1 or aspect 2, or a pharmaceutically acceptable salt thereof wherein X¹ is N.
**4**. The compound of aspect 1 or aspect 2, or a pharmaceutically acceptable salt thereof wherein X¹ is CR⁸.
**5**. The compound of aspect **4**, or a pharmaceutically acceptable salt thereof wherein R⁸ is hydrogen, fluoro, methyl, ethyl, difluoromethyl, or trifluoromethyl.
**6**. The compound of aspect **4**, or a pharmaceutically acceptable salt thereof wherein R⁸ is hydrogen.
**7**. The compound of any one of aspects 1 to **6**, or a pharmaceutically acceptable salt thereof wherein X² is CH or CF.
**8**. The compound of any one of aspects 1 to **6**, or a pharmaceutically acceptable salt thereof wherein X² is N.
**9**. The compound of any one of aspects 1 to **8**, or a pharmaceutically acceptable salt thereof wherein R¹ is hydrogen, cyano, methyl, or ethyl.
**10**. The compound aspect **9**, or a pharmaceutically acceptable salt thereof wherein R¹ is hydrogen.
**11**. The compound aspect **9**, or a pharmaceutically acceptable salt thereof wherein R¹ is cyano.
**12**. The compound aspect **9**, or a pharmaceutically acceptable salt thereof wherein R¹ is methyl.
**13**. The compound of any one of aspects 1 to **12**, or a pharmaceutically acceptable salt thereof wherein R⁷ is hydrogen, deuterium, fluoro, chloro, methyl, difluoromethyl, or trifluorommethyl.
**14**. The compound of aspect **13**, or a pharmaceutically acceptable salt thereof wherein R⁷ is hydrogen, chloro, or fluoro.
**15**. The compound of any one of aspects 1 to **14**, or a pharmaceutically acceptable salt thereof wherein R² and R³ are C₁₋₂ alkyl.
**16**. The compound of any one of aspects 1 to **14**, or a pharmaceutically acceptable salt thereof wherein R² and R³ together with the carbon atom to which they are attached form cyclopropyl.
**17**. The compound of any one of aspects 1 to **16**, or a pharmaceutically acceptable salt thereof wherein Ar is imidazolyl, isoxazolyl, pyrazolyl, 1,2,3-triazolyl, 1,2,4-triazolyl, thiazolyl, 1,2,4-oxadiazolyl, 1,3,4-oxadiazolyl, 1,2,4-thiadiazolyl, or 1,3,4-thiadiazolyl.
**18**. The compound of any one of aspects 1 to **16**, or a pharmaceutically acceptable salt thereof wherein Ar is 1,3,4-thiadiazol-2-yl.
**19**. The compound of any one of aspects 1 to **18**, or a pharmaceutically acceptable salt thereof wherein R⁴ is attached to the carbon atom of Ar that is meta to the atom of Ar that is attached to the nitrogen atom of the remainder of the molecule.
**20**. The compound of aspect **19**, or a pharmaceutically acceptable salt thereof wherein R⁴ is methyl, ethyl, difluoromethyl, trifluoromethyl, cyano, or C(O)H_{.}
**21**. The compound of aspect **19**, or a pharmaceutically acceptable salt thereof wherein R⁴ is difluoromethyl, cyano, or C(O)H_{.}
**22**. The compound of aspect **19**, or a pharmaceutically acceptable salt thereof wherein R⁴ is difluoromethyl.
**23**. The compound of any one of aspects 1 to **22**, or a pharmaceutically acceptable salt thereof wherein R⁵ and R⁶ are hydrogen or absent.
**24**. The compound of any one of aspects 1 to **23**, or a pharmaceutically acceptable salt thereof wherein ring B is phenyl substituted with R^{a}, R^{b}, and/or R^{c}.
**25**. The compound of any one of aspects 1 to **23**, or a pharmaceutically acceptable salt thereof wherein ring B is heteroaryl substituted with R^{a}, R^{b}, and/or R^{c}.
**26**. The compound of aspect **25**, or a pharmaceutically acceptable salt thereof wherein ring B is imidazolyl, pyridazinyl, thiazolyl, pyridinyl, pyrimidinyl, pyrazinyl, or indazolyl, each ring substituted with R^{a}, R^{b}, and/or R^{c} where R^{a} is hydrogen, C₁₋₆ alkyl, hydroxy, C₁₋₆ alkoxy, halo, C₁₋₆ haloalkyl, hydroxyC₁₋₆ alkyl, heteroaryl, -C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is hydrogen or C₁₋₆ alkyl), -C(O)NR^{f}R^{g} (where R^{f} and R^{g} are independently selected from hydrogen, C₁₋₆ alkyl, C₁₋₆ haloalkyl, aminoC₁₋₆ alkyl, and hydroxyC₁₋₆ alkyl), and R^{b} and R^{c} are independently selected from hydrogen, C₁₋₆ alkyl, hydroxy, C₁₋₆ alkoxy, halo, C₁₋₆ haloalkyl, and C₁₋₆ haloalkoxy; and further wherein heteroaryl of R^{a}; phenyl, heteroaryl, and heterocyclyl of R^{d} are unsubstituted or substituted with one, two, or three substituents independently selected from C₁₋₆ alkyl, and C₁₋₆ haloalkyl.
**27**. The compound of any one of aspects 1 to **23**, or a pharmaceutically acceptable salt thereof wherein ring B is heterocyclyl substituted with R^{a}, R^{b}, and/or R^{c}.
**28**. The compound of aspect **27**, or a pharmaceutically acceptable salt thereof wherein ring B is morpholinyl, 1,1-dioxothiomorpholinyl, azetinyl, pyrrolidinyl, piperidinyl, 6-oxo-1,6-dihydropyridinyl, or piperazinyl, each ring substituted with R^{a}, R^{b}, and/or R^{c} where R^{a} is hydrogen, C₁₋₆ alkyl, hydroxy, C₁₋₆ alkoxy, halo, C₁₋₆ haloalkyl, hydroxyC₁₋₆ alkyl, heteroaryl, - C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is hydrogen or C₁₋₆ alkyl), -C(O)NR^{f}R^{g} (where R^{f} and R^{g} are independently selected from hydrogen, C₁₋₆ alkyl, C₁₋₆ haloalkyl, aminoC₁₋₆ alkyl, and hydroxyC₁₋₆ alkyl), and R^{b} and R^{c} are independently selected from hydrogen, C₁₋₆ alkyl, hydroxy, C₁₋₆ alkoxy, halo, C₁₋₆ haloalkyl, and C₁₋₆ haloalkoxy; and further wherein heteroaryl of R^{a}; phenyl, heteroaryl, and heterocyclyl of R^{d} are unsubstituted or substituted with one, two, or three substituents independently selected from C₁₋₆ alkyl, and C₁₋₆ haloalkyl.
**29**. The compound of aspect **27** or **28**, or a pharmaceutically acceptable salt thereof wherein R^{a} is attached to the atom of ring B that is para to the ring atom attaching ring B to the remainder of the molecule.
**30**. The compound of aspect **29**, or a pharmaceutically acceptable salt thereof wherein R^{a} is hydrogen, C₁₋₆ alkyl, hydroxy, halo, C₁₋₆ haloalkyl, hydroxyC₁₋₆ alkyl, heteroaryl, -C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is C₁₋₆ alkyl), -C(O)NR^{f}R^{g} (where R^{f} and R^{g} are independently selected from hydrogen, C₁₋₆ alkyl, C₁₋₆ haloalkyl, and aminoC₁₋₆ alkyll), and R^{b} and R^{c} are independently selected from hydrogen, C₁₋₆ alkyl, or halo; and further wherein heteroaryl of R^{a}; heterocyclyl of R^{d} are unsubstituted or substituted with one, two, or three substituents independently selected from C₁₋₆ alkyl, and C₁₋₆ haloalkyl.
**31**. The compound of any one of aspects 1 to **23**, or a pharmaceutically acceptable salt thereof wherein ring B is bicyclic heterocylyl, fused heterocyclyl, spiro heterocyclyl, or bridged heterocyclyl, each ring substituted with R^{a}, R^{b}, and/or R^{c}; or ring B is a five to six membered heterocyclyl substituted with R^{a}, R^{b}, and/or R^{c}, wherein R^{b} and R^{c} are on adjacent ring vertices and are combined to form a four to six membered heterocyclyl having 0 to 2 additional heteroatom ring vertices selected from N, O, and S.
**32**. The compound of aspect **31**, or a pharmaceutically acceptable salt thereof wherein ring B is 2-oxaspiro[3.5]non-6-en-7-yl, 2-oxaspiro[3.5]non-7-yl, 2-oxa-8-azaspiro[4.5]dec-8-yl, 9-oxa-3-azaspiro[5.5]undec-3-yl, 2-oxa-6-azaspiro[3.4]oct-6-yl, 1-oxa-7-azaspiro[3.5]non-7-yl, 1-oxa-8-azaspiro[4.5]dec-8-yl, 6-oxa-2-azaspiro[3.3]hept-2-yl, 2,8-diazaspiro[4.5]dec-8-yl, 7-oxa-3-azabicyclo[3.3.0]oct-3-yl, 8-oxa-3-azabicyclo[4.3.0]non-3-yl, 2-oxa-6-azaspiro[3.5]non-6-yl, 7-oxo-3,6,8-triazabicyclo[4.3.0]non-3-yl, 3-pyrrolino[3,4-c]pyrazol-2-yl, 3,6-diazabicyclo[3.1.1]hept-3-yl, 2,7-diazaspiro[3.5]non-7-yl, each ring optionally substituted with R^{a} where R^{a} is hydrogen or alkyl.
**33**. A compound of aspect **1**, or a phamcetucially acceptable salt thereof, wherein said compound is selected from the group in Table 1 or from an Example 1 to 115.
**34**. A pharmaceutical composition comprising a compound of any one of aspects 1 to **33**, or a pharmaceutically acceptable salt thereof and a pharmaceutically acceptable excipient.
**35**. A method of treating cancer in a subject in need thereof, said method comprising administering to said subject an effective amount of a compound of any one of aspects 1 to **33**, or a pharmaceutically acceptable salt thereof.

## Claims

1. A compound of Formula (I): 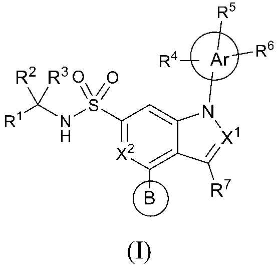 wherein:
X¹ is selected from the group consisting of N and CR⁸ where R⁸ is hydrogen, halo, C₁₋₂ alkyl, and C₁₋₂ haloalkyl;
X² is selected from the group consisting of N, CH, and CF;
R¹ is selected from the group consisting of hydrogen, cyano, formyl, -CONH₂, -CH₂OH, -CH₂OC₁₋₂ alkyl, C₁₋₂ alkyl, and C₁₋₂ haloalkyl;
R² and R³ are C₁₋₂ alkyl; or
R² and R³ together with the carbon atom to which they are attached form cyclopropyl;
Ar is a 5-membered heteroaryl;
R⁴ is selected from the group consisting of C₁₋₃ alkyl, C₁₋₃ haloalkyl, hydroxyC₁₋₃alkyl, -C(O)H, and cyano;
R⁵ and R⁶ are each independently absent or selected from the group consisting of hydrogen, C₁₋₆ alkyl, hydroxy, C₁₋₆ alkoxy, halo, C₁₋₆ haloalkyl, and C₁₋₆ haloalkoxy;
R⁷ is selected from the group consisting of hydrogen, deuterium, halo, C₁₋₆ alkyl, and C₁₋₆ haloalkyl; and
ring B is bicyclic heterocyclyl, fused heterocyclyl, spiro heterocyclyl, or bridged heterocyclyl, each ring substituted with R^{a}, R^{b}, and/or R^{c}; or ring B is a five to six membered heterocyclyl substituted with R^{a}, R^{b}, and/or R^{c}, wherein R^{b} and R^{c} are on adjacent ring vertices and are combined to form a four to six membered heterocyclyl having 0 to 2 additional heteroatom ring vertices selected from N, O, and S, wherein
R^{a} is hydrogen, C₁₋₆ alkyl, hydroxy, C₁₋₆ alkoxy, halo, C₁₋₆ haloalkyl, C₁₋₆ haloalkoxy, hydroxyC₁₋₆ alkyl, heteroaryl, heterocyclyl, -C(O)R^{d} (where R^{d} is hydrogen, C₁₋₆ alkyl, C₁₋₆ haloalkyl, C₃₋₆ cycloalkyl, phenyl, heteroaryl, or heterocyclyl), -C(O)OR^{e} (where R^{e} is hydrogen or C₁₋₆ alkyl), -C(O)NR^{f}R^{g} (where R^{f} and R^{g} are independently selected from hydrogen, C₁₋₆ alkyl, C₁₋₆ haloalkyl, aminoC₁₋₆ alkyl, and hydroxyC₁₋₆ alkyl; or R^{f} and R^{g} together with the nitrogen atom to which they are attached form heterocyclyl), or -S(O)₂NR^{h}Rⁱ (where R^{h} and Rⁱ are independently selected from hydrogen, C₁₋₆ alkyl, C₁₋₆ haloalkyl, aminoC₁₋₆ alkyl, and hydroxyC₁₋₆ alkyl; or R^{h} and Rⁱ together with the nitrogen atom to which they are attached form heterocyclyl), and R^{b} and R^{c} are independently selected from hydrogen, C₁₋₆ alkyl, hydroxy, C₁₋₆ alkoxy, halo, C₁₋₆ haloalkyl, and C₁₋₆ haloalkoxy; and
further wherein heteroaryl and heterocyclyl of R^{a}; phenyl, heteroaryl, and heterocyclyl of R^{d}, heterocyclyl formed by R^{f} and R^{g} combining with the nitrogen to which they are attached, and heterocyclyl formed by R^{h} and Rⁱ combining with the nitrogen to which they are attached are each independently unsubstituted or substituted with one, two, or three substituents selected from C₁₋₆ alkyl, hydroxy, C₁₋₆ alkoxy, halo, C₁₋₆ haloalkyl, and C₁₋₆ haloalkoxy; or
a pharmaceutically acceptable salt thereof.

2. The compound of claim 1, or a pharmaceutically acceptable salt thereof, wherein X¹ is N.

3. The compound of claim 1, or a pharmaceutically acceptable salt thereof, wherein X¹ is CR⁸.

4. The compound of claim **3**, or a pharmaceutically acceptable salt thereof, wherein R⁸ is hydrogen, fluoro, methyl, ethyl, difluoromethyl, or trifluoromethyl.

5. The compound of claim **3**, or a pharmaceutically acceptable salt thereof, wherein R⁸ is hydrogen.

6. The compound of any one of claims 1 to **5**, or a pharmaceutically acceptable salt thereof, wherein X² is CH or CF.

7. The compound of any one of claims 1 to **5**, or a pharmaceutically acceptable salt thereof, wherein X² is N.

8. The compound of any one of claims 1 to **7**, or a pharmaceutically acceptable salt thereof, wherein R¹ is hydrogen, cyano, methyl, or ethyl.

9. The compound of claim **8**, or a pharmaceutically acceptable salt thereof, wherein R¹ is hydrogen, or R¹ is cyano, or R¹ is methyl.

10. The compound of any one of claims 1 to **9**, or a pharmaceutically acceptable salt thereof, wherein R⁷ is hydrogen, deuterium, fluoro, chloro, methyl, difluoromethyl, or trifluoromethyl, and preferably wherein R⁷ is hydrogen, chloro, or fluoro.

11. The compound of any one of claims 1 to **10**, or a pharmaceutically acceptable salt thereof, wherein R² and R³ are C₁₋₂ alkyl or wherein R² and R³ together with the carbon atom to which they are attached form cyclopropyl.

12. The compound of any one of claims 1 to **11**, or a pharmaceutically acceptable salt thereof, wherein R⁵ and R⁶ are hydrogen or absent.

13. The compound of any one of claims **1** to **12**, or a pharmaceutically acceptable salt thereof, wherein ring B is 2-oxaspiro[3.5]non-6-en-7-yl, 2-oxaspiro[3.5]non-7-yl, 2-oxa-8-azaspiro[4.5]dec-8-yl, 9-oxa-3-azaspiro[5.5]undec-3-yl, 2-oxa-6-azaspiro[3.4]oct-6-yl, 1-oxa-7-azaspiro[3.5]non-7-yl, 1-oxa-8-azaspiro[4.5]dec-8-yl, 6-oxa-2-azaspiro[3.3]hept-2-yl, 2,8-diazaspiro[4.5]dec-8-yl, 7-oxa-3-azabicyclo[3.3.0]oct-3-yl, 8-oxa-3-azabicyclo[4.3.0]non-3-yl, 2-oxa-6-azaspiro[3.5]non-6-yl, 7-oxo-3,6,8-triazabicyclo[4.3.0]non-3-yl, 3-pyrrolino[3,4-c]pyrazol-2-yl, 3,6-diazabicyclo[3.1.1]hept-3-yl, 2,7-diazaspiro[3.5]non-7-yl, each ring optionally substituted with R^{a} where R^{a} is hydrogen or C₁₋₆ alkyl.

14. A pharmaceutical composition comprising a compound of any one of claims **1** to **13**, or a pharmaceutically acceptable salt thereof, and a pharmaceutically acceptable excipient.

15. A compound of any one of claims **1** to **13**, or a pharmaceutically acceptable salt thereof, or a pharmaceutical composition of claim **14**, for use in a method of treating cancer in a subject in need thereof, said method comprising administering to said subject an effective amount of the compound of any one of claims **1** to **13**, or the pharmaceutically acceptable salt thereof, or the pharmaceutical composition of claim **14**.
